# EUROPEAN PATENT APPLICATION

(11) **EP 2 977 060 A1**
(43) Date of publication of application: **27.01.2016**
(21) Application number: 15181215.3
(22) Date of filing: 23.12.2010
(51) Int. Cl.: A61K 39/00, G01N 33/50, G01N 33/68

(54) **RECEPTORS OF RSPO2 AND RSPO3**

(30) Priority: 23.12.2009 US 289734 P
(62) Divisional of application: 10807590.4
(71) Applicant: Deutsches Krebsforschungszentrum, 69120 Heidelberg (DE)
(72) Inventor: NIEHRS, Christof, 69120 Heidelberg (DE); GLINKA, Andrey, 68535 Edingen-Neckarhausen (DE); OKAWARA, Bisei, Suehiro-Cho 514-0012 (JP)
(74) Representative: Heubeck, Christian

(57) **Abstract**

The present invention relates to the finding that Syndecans (Sdc) are receptors of Rspondin-2 (Rspo2) and Rspondin-3 (Rspo3). Thus, the present invention relates to the identification of Rspo2, Rspo3 and/or Sdc activity modulators by determining if a test compound has the ability to modulate the binding of an Rspo2 and/or Rspo3 polypeptide to an Sdc polypeptide. Further, the invention refers to novel uses for antagonists of Rspo2 and/or Rspo3 in the treatment of Sdc-associated disorders and for Sdc antagonists in the treatment of Rspo2- and/or Rspo3-associated disorders.

## Description

The present invention relates to the finding that Syndecans (Sdc) are receptors of Rspondin-2 (Rspo2) and Rspondin-3 (Rspo3) and Glypicans (Glp) are receptors of Rspo3. Thus, the present invention relates to the identification of Rspo2, Rspo3, Sdc and/or Glp activity modulators by determining if a test compound has the ability to modulate the binding of an Rspo2 and/or Rspo3 polypeptide to an Sdc polypeptide or the binding of an Rspo3 polypeptide to a Glp polypeptide. Further, the invention refers to novel uses for antagonists of Rspo2 and/or Rspo3 in the treatment of Sdc-or Glp-associated disorders and for Sdc or Glp antagonists in the treatment of Rspo2- and/or Rspo3-associated disorders.

Wnt growth factors play a pivotal role in development and disease and understanding their complex signaling mechanisms and biological roles is of wide interest (Nusse 2005; Grigoryan et al. 2008). Wnts transmit their signals via different receptors and downstream pathways (Angers and Moon 2009; MacDonald et al., 2009; Tada and Kai 2009). Besides Wnts, *R-spondins (Rspo1-4;* roof plate-specific spondin) encode a family of secreted proteins in vertebrates, which can potently activate β-catenin signaling (Kazanskaya et al. 2004; Kim et al. 2005; Kazanskaya et al. 2007). *R-spondins* show specific embryonic expression patterns and are often co-expressed with and induced by Wnts (Kamata et al. 2004; Kazanskaya et al. 2004; Nam et al. 2006b), suggesting that they serve as positive feedback modulators of local Wnt signals. They are involved in embryonic patterning and differentiation in frogs and mice (Kazanskaya et al. 2004; Kim et al. 2005; Aoki et al. 2006; Blaydon et al. 2006; Kishigami et al. 2006; Parma et al. 2006). R-spondins are also implicated in human disease and hold therapeutic promise as potent stem cell growth factors (Kim et al. 2005; Blaydon et al. 2006; Parma et al. 2006; Zhao et al. 2009). Of importance for this study is Rspo3, which is involved in vasculogenesis and angiogenesis in *Xenopus* and mouse development (Aoki et al. 2006; Kazanskaya et al. 2007).

Rspondins synergize with Wnts and Fzd and require the presence of Wnts to activate β-catenin signaling (Nam et al. 2006a; Kazanskaya et al. 2007; Kim et al. 2008b). Their involvement in other Wnt pathways, notably the Wnt/PCP pathway has not been reported. R-spondin family members encode approx. 30 kDa proteins which show high structural similarity and about 60% overall sequence homology. They all contain a C-terminal thrombospondin I domain and two N-terminal Furin-like cystein rich domains, which are present in certain proteases and growth factors, such as IGF.

Another group of Wnt coreceptors are the Syndecans, a family of four transmembrane proteoglycans, which control cell proliferation, differentiation, adhesion, and migration (Bellin et al. 2002; Bass et al. 2009). Syndecans not only act as co-receptors for various growth factors, but they can also transduce signals via their intracellular domain, by interacting with numerous effectors. Syndecans cluster in response to ligand binding, become endocytosed, and may control vesicular trafficking (Oh et al. 1997; Tkachenko and Simons 2002). Unlike in mouse, in *Xenopus,* Syndecans are essential for embryonic development (Kramer and Yost 2002; Munoz et al. 2006; Matthews et al. 2008; Kuriyama and Mayor 2009; Olivares et al. 2009). Particularly important for this study is Syndecan-4 (Sdc4), which modulates signaling by FGF and chemokines (Tkachenko and Simons 2002; Charnaux et al. 2005; Iwabuschi and Goetinck 2006). Sdc4 promotes cell migration in a variety of cells and in *Xenopus* it is essential for gastrulation movements, neural crest migration and neural induction by promoting Wnt/PCP and FGF signaling (Munoz et al. 2006; Matthews et al.2008; Kuriyama and Mayor 2009).

Here it is shown that Syndecans (Sdc), i.e. Syndecan-1 (Sdc1), Syndecan-2 (Sdc2), Syndecan-3 (Sdc3) and Syndecan-4 (Sdc4) act as receptors for Rspondin-2 (Rspo2) and Rspondin3 (Rspo3) to promote Wnt signaling. In contrast thereto, no binding of syndecans to Rspondin-1 (Rspo1) and Rspondin-4 (Rspo4) was detected. No interactions between Rspo2 or Rspo3 and syndecans had previously been reported. Thus, the present invention discloses an additional pathway for Rspo2 and Rspo3 signalling which provides novel uses in research diagnosis and therapy. Further, by gain and loss of function experiments we demonstrate that Sdc4 and Rspo3 functionally interact to induce Wnt/PCP signaling during *Xenopus* gastrulation and head cartilage morphogenesis. The study reveals a novel mechanism of action of Rspondin-2 and Rspondin-3, showing that their signaling is mediated by syndecans, e.g. Syndecan-4 and is required for Wnt/PCP activation.

Further, it is shown that Rspondins, particularly Rspondin-3, binds to glypicans, particularly Glypican-1, Glypican-2, Glypican-3, Glypican-4, Glypican-5 and Glypican-6.

A first aspect of the present invention relates to a method of identifying a modulator of Rspondin-2 (Rspo2) and/or Rspondin-3 (Rspo3) and/or Syndecan (Sdc) activity, particularly Sdc1, Sdc2, Sdc3 and/or Sdc4 activity comprising evaluating and/or screening, if a test compound has the ability to modulate binding of an Rspo2 or Rspo3 polypeptide to an Sdc polypeptide compared to a control.

In a further aspect, the present invention relates to an antagonist of a Rspondin-2 (Rspo2) and/or Rspondin-3 (Rspo3) polypeptide or a Rspondin-2 (Rspo2) and/or Rspondin-3 (Rspo3) nucleic acid for use in the treatment of disorders caused by, associated with and/or accompanied by Syndecan (Sdc) hyperactivity, particularly Sdc1, Sdc2, Sdc3 and/or Sdc4 hyperactivity.

In a still further aspect the present invention relates to an antagonist of a Syndecan (Sdc), particularly Sdc1, Sdc2, Sdc3 and/or Sdc4 polypeptide or a Syndecan (Sdc), particularly Sdc1, Sdc2, Sdc3 and/or Sdc4 nucleic acid for use in the treatment of disorders caused by, associated with and/or accompanied by Rspondin-2 (Rspo2) and/or Rspondin-3 (Rspo3) hyperactivity.

In a still further aspect, the present invention relates to a method of identifying a modulator of Rspondin, particularly Rspondin-3 (Rspo3) and Glypican (Glp) activity, particularly Glp1, Glp2, Glp3, Glp4, Glp5 and/or Glp6 activity comprising evaluating and/or screening, if a test compound has the ability to modulate binding of an Rspo, particularly Rspo3 polypeptide, to a Glp polypeptide compared to a control.

In a still further aspect, the present invention relates to an antagonist of a Rspondin, particularly Rspondin-3 (Rspo3) polypeptide and/or nucleic acid for use in the treatment of disorders caused by, associated with and/or accompanied by Glypican (Glp) hyperactivity, particularly Glp1, Glp2, Glp3, Glp4, Glp5 and/or Glp6 hyperactivity.

In a still further aspect, the present invention relates to an antagonist of a Glypican (Glp), particularly Glp1, Glp2, Glp3, Glp4, Glp5 and/or Glp6 polypeptide or a Glp, particularly Glp1, Glp2, Glp3, Glp4, Glp5 and/or Glp6 nucleic acid for use in the treatment of disorders caused by, associated with and/or accompanied by Rspondin, particularly Rspondin-3 (Rspo3) hyperactivity.

As used herein the terms **'Rspondin-2 polypeptide' or 'Rspo2 polypeptide'** according to the present invention refer to polypeptides that encode Rspondin-2 or which may be derived from mammalian or other vertebrate organisms.

Preferably, the Rspondin-2 or is human Rspondin-2. The amino acid sequence of human Rspondin-2 polypeptide is shown in Gene Bank Acc. No. NM_178565, the content of which is herein incorporated by reference. A particular sequence for human Rspondin-2 is shown in SEQ ID NO:3.

As used herein the terms **"Rspondin-3 polypeptide' or 'Rspo3 polypeptide'** according to the present invention refers to polypeptides that encode Rspondin-3 which may be derived from mammalian or other vertebrate organisms.

Preferably, the Rspondin-3 polypeptide is human Rspondin-3. The amino acid sequence of human Rspondin-3 polypeptide is shown in Gene Bank Acc. No. BC022367, the content of which is herein incorporated by reference. A particular sequence for human Rspondin-3 is shown in SEQ ID NO: 1.

Further examples of Rspo2 or Rspo3 sequences are Rspo2 or Rspo3 polypeptide sequences from Xenopus, e.g. *Xenopus tropicalis* and *Xenopus laevis* or from *Mus musculus.*

Rspo2 or Rspo3 polypeptides are further defined herein as polypeptides that show at least 40%, preferably at least 60%, more preferably at least 80%, at least 90%, at least 95%, at least 98% or at least 99% sequence identity at the amino acid level to the respective human Rspo2 or Rspo3 polypeptide over its entire length (Kazanskaya et al., 2004, Dev. Cell 7, 525-534).

As used herein, the term **"Syndecan polypeptide"** or **"Sdc polypeptide"** according to the present invention refers to polypeptides that encode Syndecan-1, Syndecan-2, Syndecan-3 or Syndecan-4 which may be derived from mammalian or other vertebrate organisms.

Preferably, the Syndecan polypeptide is human Syndecan-1, Syndecan-2, Syndecan-3 or Syndecan-4. More preferably the Syndecan polypeptide is Syndecan-4. The amino acid sequences of human Sdc1, Sdc2, Sdc3 and Sdc4 polypeptides are shown in Gene Bank Acc. Nos. NM_002997 (Sdc1), NM_002998 (Sdc2), NM_014654 (Sdc3), and NM_002999 (Sdc4), the contents of which are herein incorporated by reference. Particular sequences for human Sdc1, Sdc2, Sdc3 and Sdc4 are as follows: Human Syndecan-1 amino acid sequence (SEQ ID NO:5); Human Syndecan-2 amino acid sequence (SEQ ID NO:7); Human Syndecan-3 amino acid sequence (SEQ ID NO:9); Human Syndecan-4 amino acid sequence (SEQ ID NO:11).

Further examples of Syndecan sequences are Syndecan polypeptide sequences from *Xenopus,* e.g. *Xenopus tropicalis* and *Xenopus laevis* or from *Mus musculus.*

Sdc polypeptides are further defined herein as polypeptides that show at least 40%, preferably at least 60%, more preferably at least 80%, at least 90%, at least 95%, at least 98% or at least 99% sequence identity at the amino acid level to the respective human Sdc polypeptide over its entire length.

As used herein, the term **"Glypican polypeptide"** or "**Glp polypeptide"** according to the present invention refers to polypeptides that encode Glypican-1, Glypican-2, Glypican-3, Glypican-4, Glypican-5 or Glypican-6 which may be derived from a mammalian or other vertebrate organisms.

Preferably, the Glypican polypeptide is human Glp1, Glp2, Glp3, Glp4, Glp5 or Glp6.

The amino acid sequences of human Glp1, Glp2, Glp3, Glp4, Glp5 and Glp6 polypeptides are shown in GeneBank Acc. Nos. NP_002072.2 (Glp1), NP_689955.1 (Glp2), NP_001158089.1 or NP_001158090.1 (Glp3), NP_001439.2 (Glp4), NP_004457.1 (Glp5) and NP_005699.1 (Glp6), the contents of which are herein incorporated by reference.

Further examples of Glypican sequences are Glypican polypeptide sequences from Xenopus, e.g. *Xenopus tropicalis* and *Xenopus laevis* or from *Mus musculus.*

The term **'polypeptide'** includes full-length proteins, proteinaceous molecules, fragments of proteins, fusion proteins, peptides, oligopeptides, variants, derivatives, analogs or functional equivalents thereof.

The Rspo2, Rspo3 or Sdc (i.e. Sdc1, Sdc2, Sdc3 or Sdc4) or Glp (i.e. Glp1, Glp2, Glp3, Glp4, Glp5 or Glp6) gene product itself may contain deletions, additions or substitutions of amino acid residues within the Rspo2, Rspo3, Sdc or Glp sequence, which result in a silent change thus retaining significant signal transducing capacity thus producing a functionally equivalent Rspo2, Rspo3, Sdc or Glp. Such amino acid substitutions may be made on the basis of similarity in polarity, charge, solubility, hydrophobicity, hydrophilicity, and/or the amphipatic nature of the residues involved. For example, negatively charged amino acids include aspartic acid and glutamic acid; positively charged amino acids include lysine and arginine; amino acids with uncharged polar head groups having similar hydrophilicity values include the following: leucine, isoleucine, valine; glycine, analine; asparagine, glutamine; serine, threonine; phenylalanine, tyrosine.

As used herein the terms **'Rspondin-2 nucleic acid' or Rspo2 nucleic acid'** refer to nucleic acid sequences that encode Rspondin-2 which may be derived from mammalian or other vertebrate organisms. Preferably, the Rspo2 nucleic acid encodes human Rspo2. The nucleic acid sequence of human Rspondin-2 is shown in Gene Bank Acc. No. NM 178565, the contents of which is herein incorporated by reference. A particular human Rspondin-2 nucleic acid is shown in SEQ ID NO: 4.

As used herein the terms **'Rspondin-3 nucleic acid' or 'Rspo3 nucleic acid'** refer to nucleic acid sequences that encode Rspondin-3 which may be derived from mammalian or other vertebrate organisms. Preferably, the Rspo3 nucleic acid encodes human Rspo3. The nucleic acid sequence of human Rspondin-3 is shown in Gene Bank Acc. No. BC 022367, the content of which is herein incorporated by reference. A particular human Rspondin-3 nucleic acid is shown in SEQ ID NO: 2.

Further examples of Rspo2 and Rspo3 nucleic acids are those which encode Rspo2 or Rspo3 from Xenopus, e.g. *Xenopus tropicalis* and *Xenopus laevis* or from *Mus musculus.*

As used herein the terms **"Syndecan nucleic acid"** or **"Sdc nucleic acid"** refer to nucleic acid sequences that encode Syndecan-1, Syndecan-2, Syndecan-3 or Syndecan-4 which may be derived from mammalian or other vertebrate organisms. Preferably, the Syndecan nucleic acid encodes a human Syndecan. More preferably the Syndecan is Syndecan-4. The nucleic acid sequences of human Syndecans are shown in Gene Bank Acc. No. NM_002997 (Sdc1), Gene Bank Acc. No. NM_002998 (Sdc2), Gene Bank Acc. No. NM_014654 (Sdc3) and Gene Bank Acc. No. NM_002999 (Sdc4), the contents of which are herein incorporated by reference. Particular sequences for human Syndecan nucleic acid sequences are as follows: Human Syndecan-1 nucleic acid sequence (SEG ID NO: 6), Human Syndecan-2 nucleic acid sequence (SEQ ID NO:8), Human Syndecan-3 nucleic acid sequence (SEQ ID NO:10), Human Syndecan-4 nucleic acid sequence (SEQ ID NO:12).

Further examples of Syndecan nucleic acids are those which encode the Syndecans 1, 2, 3 or 4 from *Xenopus,* e.g. *Xenopus tropicalis* and *Xenopus laevis* or from *Mus musculus.*

As used herein, the terms **"Glypican nucleic acid"** or **"Glp nucleic acid"** refer to nucleic acid sequences that encode Glp1, Glp2, Glp3, Glp4, Glp5 or Glp6, which may be derived from mammalian or other vertebrate organisms. Preferably, the Glp nucleic acid encodes a human Glp. The nucleic acid sequences of human Glypicans are shown in GeneBank Acc. Nos. NM_002081.2 (Glp1), NM_152742.1 (Glp2), NM_01164617.1 or NM_001164618.1(Glp3), NM_001448.2 (Glp4), NM_004466.4 (Glp5) and NM_005708.3 (Glp6), the contents of which are herein incorporated by reference.

Further examples of Glp nucleic acids are those which encode the Glypicans-1, 2, 3, 4, 5 or 6 from *Xenopus* or from *Mus musculus.*

Rspo2, Rspo3, Sdc or Glp nucleic acids are further defined herein as molecules selected from
(a) nucleic acid molecules encoding Rspo2, Rspo3, Sdc or Glp polypeptides, e.g. encoding human Rspo2, Rspo3, Sdc1, Sdc2, Sdc3, Sdc4, Glp1, Glp2, Glp3, Glp4, Glp5 or Glp6 polypeptides,
(b) nucleic acid molecules which hybridize under stringent conditions to a nucleic acid molecule of (a) and/or a nucleic acid molecule which is complementary thereto,
(c) nucleic acid molecules which encode the same polypeptide as a nucleic acid molecule of (a) and/or (b), and
(d) nucleic acid molecules which encode a polypeptide which is at least 40%, preferably at least 60%, more preferably at least 80%, and most preferably at least 90% identical to a polypeptide encoded by a nucleic acid molecule of (a) over its entire length.

The nucleic acid molecules may be e.g. DNA molecules or RNA molecules. Nucleic acid molecules which may be used in accordance with the invention may include deletions, additions or substitutions of different nucleotide residues resulting in a sequence that encodes the same or a functionally equivalent gene product.

As used herein, the terms **'regulators'** or **'effectors'** or **'modulators'** of Rspo2, Rspo3, Sdc and/or Glp polypeptides or Rspo2, Rspo3, Sdc and/or Glp nucleic acids are used interchangeably herein and any of the above may be used to refer to antibodies, peptides, low molecular weight organic or inorganic molecules and other sources of potentially biologically active materials capable of modulating Rspo2, Rspo3, Sdc and/or Glp polypeptides, i. e. binding of Rspo2 or Rspo3 to an Sdc, i.e. Sdc1, Sdc2, Sdc3 and/or Sdc4 polypeptide or to a Glp, i.e. Glp1, Glp2, Glp3, Glp4, Glp5 or Glp6. Said regulators, effectors or modulators can be naturally occurring or synthetically produced.

As used herein the term **'agonist'** refers to regulators or effectors or modulators of Rspo2, Rspo3, Sdc or Glp polypeptides that stimulate the binding of Rspo2 or Rspo3 to an Sdc, e.g. Sdc1, Sdc2, Sdc3 or Sdc4, or to a Glp, e.g. Glp1, Glp2, Glp3, Glp4, Glp5 or Glp6.

As used herein, the term **'antagonist'** refers to regulators or effectors or modulators of Rspo2, Rspo3, Sdc or Glp polypeptides or Rspo2, Rspo3, Sdc or Glp nucleic acids that inhibit the binding of Rspo2 or Rspo3 to an Sdc, i.e. Sdc1, Sdc2, Sdc3 or Sdc4, or to a Glp, e.g. Glp1, Glp2, Glp3, Glp4, Glp5 or Glp6, and thus inhibit, decrease or prevent processes associated with Rspo2, Rspo3, Sdc and/or Glp hyperactivity.

Examples of suitable antagonists are mutated or truncated forms of Rspo2, Rspo3 or an Sdc, i.e. Sdc1, Sdc2, Sdc3 or Sdc4 or an Glp, e.g. Glp1, Glp2, Glp3, Glp4, Glp5 or Glp6, having a dominant negative effect, Rspo2-, Rspo3-, Sdc- or Glp-binding polypeptides, e.g. anti-Rspo2, anti-Rspo3 or anti-Sdc, i.e. anti-Sdc1, anti-Sdc2, anti-Sdc3 or anti-Sdc4 or anti-Glp, i.e. anti-Glp1, anti-Glp2, anti-Glp3, anti-Glp4, anti-Glp5 or anti-Glp6 antibodies including recombinant antibodies or antibody fragments containing at least one antigen binding site. Further examples of antagonists are nucleic acids capable of inhibiting Rspo2, Rspo3 or Sdc, i.e. Sdc1, Sdc2, Sdc3 or Sdc4, or Glp, i.e. Glp1, Glp2, Glp3, Glp4, Glp5 or Glp6 translation, transcription, expression and/or activity, e.g. aptamers, antisense molecules, ribozymes or nucleic acid molecules capable of RNA interference such as siRNA molecules including nucleic acid analogs such as peptidic nucleic acids or morpholino nucleic acids. Such nucleic acids may bind to or otherwise interfere with Rspondin nucleic acids.

As used herein, the term **'antibody'** or **'antibodies'** includes but is not limited to recombinant polyclonal, monoclonal, chimeric, humanized, or single chain antibodies or fragments thereof including Fab fragments, single chain fragments, and fragments produced by an Fab expression library. Neutralizing antibodies i.e., those which compete for the receptor binding site of an Rspo2 or Rspo3 polypeptide are especially preferred for diagnostics and therapeutics.

As used herein the term **'modified'** when used with respect to the expression of an Rspo2, Rspo3, Sdc or Glp polypeptide or an Rspo2, Rspo3, Sdc or Glp nucleic acid refers to an Rspo2, Rspo3, Sdc or Glp polypeptide or Rspo2, Rspo3, Sdc or Glp nucleic acid that is expressed at a different level (e.g. with a higher expression level) that is expressed in a different location (e.g. in a different cell type than where it is usually expressed) or that is expressed at a different time (e.g. in a situation where it is constitutively expressed rather that expressed in response to a particular signal). In particular a cell or non-human transgenic organism that demonstrates modified expression of an Rspo2, Rspo3, Sdc or Glp nucleic acid or an Rspo2, Rspo3, Sdc or Glp polypeptide may exhibit permanently modified expression (e.g. due to changes in the genome of the cell or the organism) or it may exhibit transiently modified expression (e.g. due to temporary transfection of an mRNA sequence).

As used herein, the term **'treating'** or **'treatment'** refers to an intervention performed with the intention of preventing the development or altering the pathology of, and thereby alleviating a disorder, disease or condition, including one or more symptoms of such disorder or condition. Accordingly, 'treating' refers to both therapeutic treatment and prophylactic or preventative measures. Those in need of treating include those already with the disorder as well as those in which the disorder is to be prevented. The related term 'treatment', as used herein, refers to the act of treating a disorder, symptom, disease or condition, as the term 'treating' is defined above.

As used herein the term **"binding",** when used to describe the interaction between an Rspondin polypeptide (specifically Rspo2 or Rspo3) and a Syndecan (specifically Sdc1, Sdc2, Sdc3 or Sdc4) or the interaction between an Rspondin polypeptide (specifically Rspo3) and a Glypican (specifically Glp1, Glp2, Glp3, Glp4, Glp5 or Glp6) refers to reversible interaction between said Rspondin and said Syndecan. The term includes interactions between the core of one protein and post-translational modifications that may be present on a second protein. In particular, with reference to the interactions disclosed herein it includes the interaction between an Rspondin and a Syndecan comprising glycosaminoglycan chains (GAGs) attached thereto. This interaction may involve binding to the GAGs of Syndecan. In a specific embodiment, it refers to the interaction between Rspo3 and Sdc4 comprising GAGs attached thereto. This interaction may involve binding to the GAGs of Sdc4. Regardless of the mechanism by which Rspondins 2 or 3 interact with Syndecans 1, 2, 3 or 4, a person of skill in the art will appreciate the need to use in any assays a protein that is correctly folded and contains all the essential post-translational modifications required for activity. This is the case regardless of whether the protein is purified from its native environment or expressed in a heterologous manner. Means to ensure that the protein(s) contain all the required post-translational modifications are well within the abilities of a person of skill in the art and are further taught in the examples herein. Additionally, when using a functional assay a person of skill in the art will appreciate the need to use a protein active in said functional assay which will implicitly contain all the post-translational modifications required.

Preferably the binding between an Rspondin polypeptide (specifically Rspo2 or Rspo3) and a Syndecan (Sdc1, Sdc2, Sdc3 or Sdc4) involves a binding constant of ≤ 10 nM, of ≤ 5 nM, of ≤ 2 nM or of ≤ 1 nM. Determination of the binding constant may involve , but is not limited to the methods described in the Examples herein.

The term **"glycosaminoglycan"** (abbreviated as **"GAG"**) is used herein to refer to a class of linear unbranched polysaccharides comprising a repeating disaccharide unit, which typically comprise hexosamine and a hexose or a hexuronic acid. In some embodiments, the repeating disaccharide unit comprises a glucosamine or galactosamine followed by an iduronic or glucuronic acid. Glycosaminoglycans are tvpically highly negatively charged and have special structural features that contribute to their various functions. For example, in an extended conformation, glycosaminoglycans contribute to the viscosity of the fluid of which they are a part. Their rigidity provides structural integrity that is central to their role in cell migration. Glycosaminoglycans are the most abundant heteropolysaccharides in the body, forming a major component of the extracellular matrix as well as a major part of glycoproteins commonly found on the cell surface. Glycosaminoglycans are often covalently attached to proteins, forming together with the protein a proteoglycan. Examples of glycosaminoglycans include, but are not limited to, chondroitin sulfate, dermatan sulfate, keratan sulfate, heparin, heparan sulfate, and hyaluronan. Those of ordinary skill in the art will appreciate that there is structural variability in the chemical structures within one type of glycosaminoglycan (such as heparin). Though glycosaminoglycans are recognizable by a general chemical structural theme, a glycosaminoglycan of a given type exists in multiple forms and varies in the composition of disaccharide units that comprise it. They also vary in the extent and pattern of sulfation along the molecule. Nevertheless, the term "glycosaminoglycan" will be understood by those of ordinary skill in the art to mean a particular class of polysaccharides as described above.

The term **"morpholino"** or **"Mo"** as used herein relates to a molecule sometimes referred to as phosphorodiamidate morpholino oligonucleotide used to modify gene expression. Said morpholinos are synthetic molecules which are the product of a redesign of natural nucleic acid structure. Usually 15-50, e.g. 25 bases in length, they bind to complementary sequences of RNA by standard nucleic acid base-pairing. Structurally, the difference between morpholinos and DNA is that while morpholinos have standard nucleic acid bases, those bases are bound to morpholine rings instead of deoxyribose rings and linked through phosphorodiamidate groups instead of phosphates. Morpholinos are most commonly used as single-stranded oligos, though heteroduplexes of a morpholino strand and a complementary DNA strand may be used in combination with cationic cytosolic delivery reagents. Morpholino oligomers (oligos) are an antisense technology used to block access of other molecules to specific sequences within nucleic acid. Morpholinos block small (-25 base) regions of the base-pairing surfaces of ribonucleic acid (RNA). Morpholinos are usually used to knock down gene function. This is achieved by preventing cells from making a targeted protein or by modifying the splicing of pre-mRNA.

The present inventors have found that Syndecan-1 (Sdc1), Syndecan-2 (Sdc2), Syndecan-3 (Sdc3) and Syndecan-4 (Sdc4) are receptors for Rspondin-2 (Rspo2), and Rspondin-3 (Rspo3) and that together they activate Wnt/PCP signaling. In *Xenopus* embryos, Sdc4 and Rspo3 are essential for Wnt/PCP driven processes, e.g. gastrulation movements. In gastrulae, the cooperation of Rspo3 and Wnt-5a is required for Sdc4 mediated PCP signaling. Thus, the present inventors conclude that Rspo2 or Rspo3 signaling is mediated by Scd1, Sdc2, Sdc3 or Sdc4 and is required for Wnt/PCP activation.

Further, the present inventors have found that Glypicans Glp1, Glp2, Glp3, Glp4, Glp5 and Glp6 are receptors for Rspondin-3.

Thus, the present inventors conclude that Rspo3 signalling may be at least partially mediated by Glp1, Glp2, Glp3, Glp4, Glp5 or Glp6.

An embodiment of the present invention refers to novel evaluation and/or screening methods and systems based on a determination of the binding of an Rspo2 or Rspo3 polypeptide to an Sdc, i.e. Sdc1, Sdc2, Sdc3 or Sdc4 polypeptide. Thus, the present invention provides a method of identifying a modulator of Rspondin-2 (Rspo2) or Rspondin-3 (Rspo3) and/or Syndecan-1 (Sdc1), Syndecan-2 (Sdc2), Syndecan-3 (Sdc3) or Syndecan-4 (Sdc4) activity, comprising evaluating and/or screening, if a test compound has the ability to modulate binding of an Rspo2 or Rspo3 polypeptide to an Sdc1, Sdc2, Sdc3 or Sdc4 polypeptide compared to a control. In a specific embodiment the present invention provides said method which comprises evaluating and/or screening if a test compound has the ability to modulate binding of an Rspo3 polypeptide to Sdc4. In a further specific embodiment, the present invention provides a method which comprises evaluating and/or screening if a test compound has the ability to modulate binding of Rspo2 or Rspo3 to the GAG chains of Sdc1, Sdc2, Sdc3 or Sdc4 comprising GAG chains attached thereto. Specifically the binding of Rspo3 to Sdc4 comprising GAG chains attached thereto is preferred.

An embodiment of the present invention refers to novel evaluation and/or screening methods and systems based on a determination of the binding of an Rspo2 or Rspo3 polypeptide to an Glp, i.e. Glp1, Glp2, Glp3, Glp4, Glp5 or Glp6 polypeptide. Thus, the present invention provides a method of identifying a modulator of Rspondin-3 (Rspo3) and/or Glypican-1 (Glp1), Glypican-2 (Glp2), Glypican-3 (Glp3), Glypican-4 (Glp4), Glypican-5 (Glp5) or Glypican-6 (Glp6) activity, comprising evaluating and/or screening, if a test compound has the ability to modulate binding of an Rspo3 polypeptide to an Glp1, Glp2, Glp3, Glp4, Glp5 or Glp6 polypeptide compared to a control.

In one embodiment of this aspect, the method comprises evaluating and/or screening, if a test compound has the ability to stimulate binding. In this embodiment, the test compound is an agonist having the ability to increase binding of an Rspo2 or Rspo3 polypeptide to an Sdc or of an Rspo3 polypeptide to a Glp polypeptide compared to a control e.g. a reference measurement in the absence of the test compound.

Therefore, in one embodiment the present invention provides a method of screening for an Rspo2, Rspo3 and/or an Sdc agonist said method comprising:
(a) contacting Rspo2 or Rspo3 with an Sdc, selected from Sdc1, Sdc2, Sdc3, Sdc4 and combinations thereof, in the presence of the test compound;
(b) contacting Rspo2 or Rspo3 with the Sdc in the absence of the test compound; and
(c) comparing the binding between Rspo2 or Rspo3 and the Sdc between (a) and (b) where an increase in binding in the presence of the compound indicates the compound is an Rspo2, Rspo3 and/or Sdc agonist.

In a further embodiment, the method comprises evaluating and/or screening if a test compound has the ability to inhibit binding. In this embodiment, the test compound is an antagonist having the ability to inhibit binding of an Rspo2 or Rspo3 polypeptide to an Sdc polypeptide compared to a control e.g. a reference measurement in the absence of the test compound.

Therefore, in one embodiment the present invention provides a method of screening for an Rspo2, Rspo3 and/or an Sdc antagonist said method comprising:
(a) contacting Rspo2 or Rspo3 with an Sdc selected from Sdc1, Sdc2, Sdc3, Sdc4 and combinations thereof, in the presence of the test compound;
(b) contacting Rspo2 or Rspo3 with the Sdc in the absence of the test compound; and
(c) comparing the binding between Rspo2 or Rspo3 and the Sdc between (a) and (b) where a decrease in binding in the presence of the compound indicates the compound is an Rspo3 and/or Sdc antagonist.

In a further embodiment, the method comprises evaluating and/or screening if a test compound has the ability to enhance or inhibit binding of a known interacting partner of an Sdc, selected from Sdc1, Sdc2, Sdc3, Sdc4 and combinations thereof in conditions when it is already prebound with Rspo2 or Rspo3. In this embodiment, the test compound having the ability to inhibit or enhance binding of this known interacting partner to an Sdc polypeptide compared to a control e.g. a reference measurement in the absence of the test compound. An example of such known interacting partner of Sdc4 is FGF (Tkachenko and Simons 2002).

The method may involve determination of the binding in a cell-free system, e.g. determination of binding between purified or partially purified Rspo2 or Rspo3 and Sdc polypeptides. In this embodiment the binding may be determined by immobilizing one of the components on a solid phase, e.g. in a microtiter well or on a microchip, and contacting the solid phase with the other component in non-immobilized form, optionally comprising a suitable reporter group, in the presence of a test compound.

Where the binding assay is being performed in a cell-free assay it is necessary to ensure that the proteins are correctly folded and have the correct post-translational modifications, including but not limited to the presence of glycosaminoglycans (GAGs). A person of skill in the art will be aware of celllular systems, e.g. eukaryotic, particularly mammalian celllular systems and purification methods which ensure this, including but not limited to the cellular systems and purification methods described in the Examples herein.

In a further embodiment, the binding is determined in a cellular system, e.g. in a recombinant cell or non-human transgenic organism, e.g. in a cell or organism which overexpresses Rspo2 or Rspo3 and/or at least one Sdc, selected from Sdc1, Sdc2, Sdc3 and Sdc4. In this embodiment, the binding may be determined by direct measurement of Rspo2/Sdc or Rspo3/Sdc complexes or by indirect measurement, e.g. activation or inhibition of the Rspo2/Sdc or Rspo3/Sdc signaling, e.g. the Wnt/PCP cascade.

When the binding is determined in a cellular system a person of skill in the art will be able to ensure that the host cellular machinery will be able to provide the correct post-translational modifications, including but not limited to the presence of glycosaminoglycans (GAGs). A person of skill in the art will be aware of cellular systems which ensure this e.g. eukaryotic, particularly mammalian cellular systems, including but not limited to the cellular systems described in the Examples herein.

Therefore, in one embodiment the present invention provides a method of screening for an Rspo2 or Rspo3 and/or an Sdc agonist said method comprising:
(a) contacting Rspo2 or Rspo3 with an Sdc, selected from Sdc1, Sdc2, Sdc3, Sdc4 and combinations thereof, in the presence of the test compound;
(b) contacting Rspo2 or Rspo3 with the Sdc in the absence of the test compound; and
(c) comparing the activity of Rspo2 or Rspo3 and/or the Sdc between (a) and (b) where an increase in activity in the presence of the compound indicates the compound is an Rspo2, Rspo3 and/or Sdc agonist.

Therefore, in one embodiment the present invention provides a method of screening for an Rspo2 or Rspo3 and/or an Sdc antagonist said method comprising:
(a) contacting Rspo2 or Rspo3 with an Sdc, selected from Sdc1, Sdc2, Sdc3, Sdc4 and combinations thereof, in the presence of the test compound;
(b) contacting Rspo2 or Rspo3 with the Sdc in the absence of the test compound; and
(c) comparing the activity of Rspo2 or Rspo3 and/or the Sdc between (a) and (b) where an decrease in activity in the presence of the compound indicates the compound is an Rspo2 or Rspo3 and/or Sdc antagonist.

In a specific embodiment, the screening method may comprise the steps:
(a) determining the binding of an Rspo2- or Rspo3- alkaline phosphatase fusion (AP) polypeptide in the presence of the test compound to a cell recombinantly expressing an Sdc polypeptide selected from Sdc1, Sdc2, Sdc3, Sdc4 and combinations thereof,
(b) determining the binding of the fusion polypeptide in the absence of the test compound to the cell; and
(c) comparing the AP activity bound to the cell between (a) and (b) wherein a decrease in binding in the presence of the test compound indicates the compound is an Rspo2, Rspo3 and/or Sdc antagonist.

In a specific embodiment related to any of the screening methods described above, the Rspondin is Rspondin-3.

In a specific embodiment related to any of the screening methods described above, the interaction between Rspo3 and Sdc4 is measured.

The above indicated methods are also suitable for screening Glp agonists or antagonists, wherein the effect of a test compound on the interaction between Rspo3 and Glp is determined. An increase in binding in the presence of the test compound indicates that the compound is an Rspo3 and/or Glp agonist. A decrease in binding in the presence of the compound indicates that the compound is an Rspo3 and/or Glp antagonist.

Thus, the present invention also provides recombinant cells or non-human transgenic organisms overexpessing Rspo2 or Rspo3 and/or an Sdc, i.e. Sdc1, Sdc2, Sdc3 and/or Sdc4, and/or a Glp, i.e. Glp1, Glp2, Glp3, Glp4, Glp5 and/or Glp6. These cells and organisms are particularly suitable for identifying modulators, e.g. agonists or antagonists of Rspo2, Rspo3, an Sdc and/or a Glp.

For example, screening may be carried out to identify antibodies capable of inhibiting the binding of Rspo2 or Rspo3 to an Sdc, i.e. Sdc1, Sdc2, Sdc3 and/or Sdc4, or the binding of Rspo3 to a Glp, i.e. Glp1, Glp2, Glp3, Glp4, Glp5 and/or Glp6. The antibodies may be chimeric antibodies, fully human antibodies, or antibody variable domains, which may be used to inhibit binding. Alternatively, screening of peptide libraries or organic compounds with a recombinantly expressed soluble Rspo2 or Rspo3 polypeptide, an Sdc polypeptide, and a Glp polypeptide, cell lines expressing an Rspo2 or Rspo3 polypeptide, an Sdc polypeptide and a Glp polypeptide or transgenic non-human animals expressing an Rspo2 or an Rspo3 polypeptide may be useful for identification of therapeutic molecules that function by modulating, e.g. inhibiting, the binding of Rspo2 or Rspo3 to an Sdc or the binding of Rspo3 to a Glp and thus are suitable as regulators or effectors or modulators of Rspo2 or Rspo3 and an Sdc or a Glp, e.g. antagonists of Rspo2 or Rspo3 and an Sdc or a Glp. Alternatively, screening of oligonucleotide libraries, e.g. shRNA libraries or siRNA libraries with cell lines that express an Rspo2 or an Rspo3 nucleic acid and an Sdc or a Glp nucleic acid or an Rspo2 or Rspo3 polypeptide and an Sdc or a Glp polypeptide may be useful for identification of therapeutic molecules that function by modulating, e.g. inhibiting, the binding of Rspo2 or Rspo3 to an Sdc or a Glp and thus are suitable as bone formation regulators or effectors or modulators of Rspo2 or Rspo3 and an Sdc or a Glp, e.g. antagonists of Rspo3 and Sdc4.

The method of the present invention is particularly suitable for identifying and/or evaluating candidate agents for the treatment of an Rspo2-, Rspo3- and/or Sdc-, i.e. Sdc1-, Sdc2-, Sdc3- or Sdc4- and/or Glp-associated disorder, e.g. a disorder which is caused by, associated with and/or accompanied by Sdc, Glp and/or Rspo2 or Rspo3 hyperactivity, particularly increased binding of Rspo2 or Rspo3 to an Sdc or of Rspo3 to a Glp. Examples of Rspo2-, Rspo3-, Sdc- and/or Glp-associated disorders are e.g. proliferation-associated disorders such as cancer, such as hepatocellular carcinoma, inflammatory disorders, bone-associated disorders, such as osteoarthritis and wound healing.

A further aspect of the present invention refers to an antagonist of an Rspo2 or Rspo3 polypeptide or an Rspo2 or Rspo3 nucleic acid for use in the treatment of disorders caused by, associated with and/or accompanied by an Sdc, i.e. Sdc1, Sdc2, Sdc3 and/or Sdc4 hyperactivity. The antagonist may e.g. be an anti-Rspo2 antibody, anti-Rspo3 antibody or an Sdc fragment, e.g. an Sdc4 fragment, particularly from amino acids 1 to145 or a part thereof, preferably having a length of at least 10, 20 or 30 amino acids, with or without point mutations in positions of glycosaminoglycans (GAG) attachment. More preferably, the Sdc fragment, e.g. the Sdc4 fragment comprises GAGs attached thereto. It can be also a Rspo2 or Rspo3 fragment particularly an Rspo3 fragment from amino acid 25 to amino acid 209 or a part thereof, preferably having a length of at least 10, 20 or 30 amino acids, with or without point mutations or a nucleic acid molecule capable of inhibiting Rspo2 or Rspo3 expression.

According to this embodiment, the treatment preferably comprises determination of Sdc, i.e. Sdc1, Sdc2, Sdc3 and/or Sdc4 amount and/or activity in a subject to be treated, e.g. on the mRNA level or the protein level. Preferably, this aspect refers to the treatment of a proliferative disorder, e.g. selected from cancer, such as hepatocellular carcinoma, an inflammatory disorder, a bone-associated disorder, such as osteoarthritis, and wound healing, wherein the disorder is characterized by an increased amount and/or activity of an Sdc, e.g. compared to a healthy control.

Still a further aspect of the present invention refers to an antagonist of an Sdc, i.e. Sdc1, Sdc2, Sdc3 and/or Sdc4 polypeptide or an Sdc, i.e. Sdc1, Sdc2, Sdc3 and/or Sdc4 nucleic acid for use in the treatment of disorders caused by, associated with and/or accompanied by Rspo2 and/or Rspo3 hyperactivity. Preferably, the antagonist may e.g. be an anti-Rspo2 antibody, an anti-Rspo3 antibody, an Sdc fragment, e.g. an Sdc4 fragment, particularly from amino acid 1 to 145 or a part thereof, preferably having a length of at least 10, 20 or 30 amino acids, with or without point mutations in positions of glycosaminoglycans (GAG) attachment. More preferably, the Sdc fragment, e.g. the Sdc4 fragment comprises GAGs attached thereto. It can be also an Rspo2 or Rspo3 fragment, e.g. an Rspo3 fragment, particularly from amino acid 25 to amino acid 209 or a part thereof, preferably having a length of at least 10, 20 or 30 amino acids, with or without point mutations or a nucleic acid molecule capable of inhibiting Rspo2 or Rspo3 expression.

In a further aspect the present invention provides an antagonist of a Rspondin-2 (Rspo2) or Rspondin-3 (Rspo3) polypeptide for use as a medicament, wherein said antagonist inhibits or blocks the interaction of a Rspondin-2 (Rspo2) or Rspondin-3 (Rspo3) polypeptide with a Syndecan (Sdc) polypeptide. In a specific embodiment the Sdc is Sdc4. In a further particular embodiment the antagonist is an antibody, preferably a monoclonal antibody. In a further particular embodiment the antibody is against Rspondin-2 (Rspo2). In a further particular embodiment the antibody is against Rspondin-3 (Rspo3).

In another aspect the present invention provides an antagonist of an Rspondin-2 (Rspo2) or Rspondin-3 (Rspo3) polypeptide, wherein said antagonist is used in the treatment of cancer, an inflammatory disorder, a bone associated disorder, or wound healing.

In another aspect, the present invention provides an antagonist of a Syndecan (Sdc) polypeptide for use as a medicament, wherein said antagonist inhibits or blocks the interaction of a Syndecan (Sdc) polypeptide with a Rspondin-2 (Rspo2) or a Rspondin-3 (Rspo3) polypeptide. In a particular embodiment said antagonist blocks the interaction of a Syndecan (Sdc) polypeptide with a Rspondin-3 (Rspo3) polypeptide. In a specific embodiment said antagonist is an antibody, preferably a monoclonal antibody. In a further specific embodiment, said antagonist is used in the treatment of cancer, an inflammatory disorder, a bone associated disorder, or wound healing.

In a preferred embodiment of this aspect, the treatment comprises determination of Rspo2 and/or Rspo3 amount and/or activity in a subject to be treated, e.g. on the mRNA level or the protein level. Preferably, the disorder is a proliferative disorder, e.g. selected from cancer, such as hepatocellular carcinoma, an inflammatory disorder, a bone-associated disorder, such as osteoarthritis and wound healing, wherein the disorder is characterized by an increased amount and/or activity of Rspo2 and/or Rspo3.

In a further aspect, the present invention provides an antagonist of an Rspo2 polypeptide or an Rspo3 nucleic acid for the use in the treatment of disorders caused by, associated with and/or accompanied by a Glp hyperactivity. The antagonist may e.g. be an anti-Rspo3 antibody or a biologically inactive Glp fragment, preferably having a length of at least 10, 20 or 30 amino acids. It can also be an Rspo3 fragment as described above.

According to this embodiment, the treatment preferably comprises determination of Glp amount and/or activity in a subject to be treated, e.g. on the mRNA level or the protein level. Preferably, this aspect refers to the treatment of a proliferative disorder, an inflammatory disorder, a bone-associated disorder and wound healing, wherein the disorder is characterized by an increased amount and/or activity of a Glp, e.g. compared to a healthy control.

Still a further aspect of the present invention refers to an antagonist of a Glp polypeptide or a Glp nucleic acid for use in the treatment of disorders caused by, associated with and/or accompanied by Rspo, particularly Rspo3 hyperactivity. Preferably, the antagonist may e.g. be an anti-Rspo3 antibody or a Glp fragment as described above.

Preferably, the antagonist inhibits or blocks the interaction of a Rspondin3 (Rspo) polypeptide with a Glypican (Glp) polypeptide.

In all aspects of the present invention, the antagonist may be administered in human or veterinary medicine.

Determination of Rspo2, Rspo3 and/or Sdc, i.e. Sdc1, Sdc2, Sdc3 and/or Sdc4, and/or Glp amount and/or activity may involve measurement of Rspo2, Rspo3, Sdc and/or Glp expression on the mRNA level or protein level, direct measurement of Rspo2/Sdc and/or Rspo3/Sdc and/or Rspo/Glp binding, and/or measurement of the canonical and/or non-canonical Wnt pathway.

In one embodiment the activity of the non-canonical Wnt pathway is measured by Jnk phosphorylation and/or assaying convergent extension movement in *Xenopus* embryos (Yamanaka et al., 2002). The activation of the Wnt/PCP pathway can also be measured by ATF luciferase reporter assay in Xenopus embryos.

In one embodiment the activity of the canonical Wnt pathway is measured by TOPFLASH luciferase reporter assays or b-catenin stabilisation arrays (Kazanskaya et al., 2004; Kim et al., 2008b).

In a preferred embodiment, the antagonist is an antibody. Various procedures known in the art may be used for the production of antibodies to epitopes of an Rspo2, Rspo3 or Sdc, i.e. Sdc1, Sdc2, Sdc3 and/or Sdc4, or Glp, i.e. Glp1, Glp2, Glp3, Glp4, Glp5 and/or Glp6 polypeptide.

Monoclonal antibodies that bind to an Rspo2, Rspo3, Sdc or Glp polypeptide may be labeled allowing one to follow their location and distribution in the body after injection. Tagged antibodies may be used as a non-invasive diagnostic tool for imaging bone formation and/or resorption associated with conditions where treatment involves inhibiting loss of bone mass and/or promoting bone formation.

Immunotoxins may also be designed which target cytotoxic agents to specific sites in the body. For example, high affinity Rspo2-, Rspo3-, Sdc- or Glp-specific monoclonal antibodies may be covalently complexed to bacterial or plant toxins, such as diptheria toxin, abrin or ricin. A general method of preparation of antibody/hybrid molecules may involve use of thiol-crosslinking reagents such as SPDP, which attack the primary amino groups on the antibody and by disulfide exchange, attach the toxin to the antibody.

For the production of antibodies, various host animals may be immunized by injection with the Rspo2, Rspo3, Sdc or Glp polypeptide including but not limited to rabbits, mice, rats, etc. Various adjuvants may be used to increase the immunological response, depending on the host species, including but not limited to Freund's (complete and incomplete), mineral gels such as aluminium hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and Corynebacterium parvum.

Monoclonal antibodies to Rspo2, Rspo3, Sdc or Glp polypeptides may be prepared by using any technique which provides for the production of antibody molecules by continuous cell lines in culture. These include but are not limited to the hybridoma technique originally described by Köhler and Milstein, (Nature, 1975, 256: 495-497), the human B-cell hybridoma technique (Kosbor et al., 1983, Immunology Today, 4: 72; Cote et a/., 1983, Proc. Natl. Acad. Sci., 80: 2026-2030) and the EBV-hybridoma technique (Cole et al., 1985, Monoclonal Antibodies and Cancer Therapy, Alan R. Liss, Inc., pp. 77-96). In addition, techniques developed for the production of "chimeric antibodies" (Morrison et al., 1984, Proc. Natl. Acad. Sci., 81: 6851-6855; Neuberger et al., 1984, Nature, 312: 604-608; Takeda et al., 1985, Nature, 314: 452-454) by splicing the genes from a mouse antibody molecule of appropriate antigen specificity together with genes from a human antibody molecule of appropriate biological activity can be used. Alternatively, techniques described for the production of single chain antibodies (U.S. Pat. No. 4,946,778) can be adapted to produce Rspo2-, Rspo3-, Sdc- or Glp-specific single chain antibodies.

Antibody fragments which contain specific binding sites for Rspo2, Rspo3, an Sdc or a Glp may be generated by known techniques. For example, such fragments include but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse et al., 1989, Science, 246: 1275-1281) to allow rapid and easy identification of monoclonal Fab fragments with the desired specificity to Rspondin.

Antibodies to Rspo2 polypeptides may antagonise the activity of Rspondin-2 by preventing it from binding to an Sdc, i.e. Sdc1, Sdc2, Sdc3 and/or Sdc4. Therefore, antibodies which bind specifically to Rspo2, may be antagonists of Rspo2.

Antibodies to Rspo3 polypeptides may antagonise the activity of Rspondin-3 by preventing it from binding to an Sdc, i.e. Sdc1, Sdc2, Sdc3 and/or Sdc4 or to a Glp. Therefore, antibodies which bind specifically to Rspo3, may be antagonists of Rspo3.

Antibodies to Sdc polypeptides, i.e. Sdc1, Sdc2, Sdc3 or Sdc4 may antagonise the activity of the respective Sdc by preventing it from binding to Rspo2 and/or Rspo3. Therefore, antibodies which bind specifically to an Sdc may be antagonists of the respective Sdc.

Antibodies to Glp polypeptides, i.e. Glp1, Glp2, Glp3, Glp4, Glp5 or Glp6, may antagonize the activity of the respective Glp by preventing it from binding to Rspo3. Therefore, antibodies which specifically bind to a Glp may be antagonists of the respective Glp.

In addition, mutated or truncated forms of Rspo2, Rspo3, of an Sdc or of a Glp, having a dominant negative effect, may be used as antagonists.

Included in the scope of the invention are nucleic acid antagonists of Rspo2, Rspo3 or an Sdc. Anti-sense molecules, e.g. anti-sense RNA and DNA molecules or morpholinos act to directly block the translation of mRNA by binding to targeted mRNA and preventing protein translation. In regard to antisense molecules, oligodeoxyribonucleotides or morpholinos derived from the translation initiation site, e.g., between -10 and +10 regions of the Rspo3 or Sdc4 nucleotide sequence, are preferred.

Ribozymes are enzymatic RNA molecules capable of catalyzing the specific cleavage of RNA. The mechanism of ribozyme action involves sequence-specific hybridization of the ribozyme molecule to complementary target RNA, followed by a endonucleolytic cleavage. Within the scope of the invention are engineered hammerhead motif ribozyme molecules that specifically and efficiently catalyze endonucleolytic cleavage of target RNA sequences.

Specific ribozyme cleavage sites within any potential RNA target are initially identified by scanning the target molecule for ribozyme cleavage sites which include the following sequences, GUA, GUU and GUC. Once identified, short RNA sequences of between 15 and 20 ribonucleotides corresponding to the region of the target gene containing the cleavage site may be evaluated for predicted structural features such as secondary structure that may render the oligonucleotide sequence unsuitable. The suitability of candidate targets may also be evaluated by testing their accessibility to hybridization with complementary oligonucleotides, using ribonuclease protection assays.

RNAi molecules are double-stranded RNA molecules or analogues thereof including analogues with morpholino building blocks, which capable of mediating RNA interference of a target mRNA molecule, e.g. siRNA molecules which are short double-stranded RNA molecules with a length of preferably 19-25 nucleotides and optionally at least one 3'-overhang or precursors thereof or DNA molecules coding therefor.

Anti-sense molecules, e.g. anti-sense RNA and DNA molecules or analogues thereof including morpholinos, ribozymes and RNAi molecules of the invention may be prepared by any method known in the art for the synthesis of RNA molecules. These include techniques for chemically synthesizing oligodeoxyribonucleotides or morpholinos well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Various modifications to the DNA molecules may be introduced as a means of increasing intracellular stability and half-life. Possible modifications include but are not limited to the addition of flanking sequences of Morpholino derivatives as well as ribo- or deoxy-nucleotides to the 5' and/or 3' ends of the molecule or the use of phosphorothioate or 2' O-methyl rather than phosphodiesterase linkages within the oligodeoxyribonucleotide backbone.

In a particular embodiment of the invention, antagonists of Rspo2 or Rspo3 polypeptides, Sdc polypeptides or Glp polypeptides or Rspo2 or Rspo3 nucleic acids, Sdc nucleic acids or Glp polypeptides may be used in the treatment of conditions where treatment involves reducing Rspo2 or Rspo3 and/or Sdc activity by inhibiting binding of Rspo2 and/or Rspo3 to an Sdc, i.e. Sdc1, Sdc2, Sdc3 and/or Sdc4, or reducing Rspo3 and/or Glp activity by inhibiting binding of Rspo3 to a Glp.

In a particular embodiment of the invention the antagonist is an antibody. In a most particular embodiment of the invention an Rspo2, Rspo3, Sdc or Glp antibody may be used to treat conditions wherein treatment involves reducing Rspo2, Rspo3 and/or Sdc activity by inhibiting binding of Rspo2 or Rspo3 to an Sdc, i.e. Sdc1, Sdc2, Sdc3 and/or Sdc4, or reducing Rspo3 and/or Glp activity by inhibiting binding of Rspo3 to a Glp.

In a particular embodiment of the invention the nucleic acid antagonist is a nucleic acid capable of inhibiting Rspo2, Rspo3, Sdc or Glp translation, transcription, expression and/or activity. In a most particular embodiment of the invention a nucleic acid capable of inhibiting Rspo2, Rspo3, Sdc or Glp translation, transcription, expression and/or activity may be used to treat conditions wherein treatment involves reducing Rspo2, Rspo3, an Sdc and/or aGlp activity by inhibiting binding of Rspo2 or Rspo3 to an Sdc, or of Rspo3 to a Glp. In a most particular embodiment of the invention an siRNA, shRNA or other antisense nucleic acid against Rspo2, Rspo3, an Sdc or a Glp may be used to treat conditions where treatment involves reducing Rspo2, Rspo3 and/or Sdc activity by inhibiting binding of Rspo2 or Rspo3 to an Sdc, i.e. Sdc1, Sdc2, Sdc3 and/or Sdc4, or reducing Rspo3 and/or Glp activity by inhibiting binding of Rspo3 to a Glp.

Pharmaceutically active antagonists of Rspo2, Rspo3, Sdc or Glp polypeptides or Rspo2, Rspo3, Sdc or Glp nucleic acids can be administered to a patient either by itself, or in pharmaceutical compositions where it is mixed with suitable carriers or excipient(s).

Depending on the specific conditions being treated, these agents may be formulated and administered systemically or locally. Techniques for formulation and administration may be found in "Remington's Pharmaceutical Sciences," Mack Publishing Co., Easton, Pa., latest edition. Suitable routes may, for example, include oral, rectal, transmucosal, or intestinal administration; parenteral delivery, including intramuscular, subcutaneous, intramedullary injections, as well as intrathecal, direct intraventricular, intravenous, intraperitoneal, intranasal, or intraocular injections, or, in the case of solid tumors, directly injected into a solid tumor. For injection, the agents of the invention may be formulated in aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

The antagonists can be formulated readily using pharmaceutically acceptable carriers well known in the art into dosages suitable for oral administration. Such carriers enable the active agents of the invention to be formulated as tablets, pills, capsules, liquids, gels, syrups, slurries, suspensions and the like, for oral ingestion by a patient to be treated.

Pharmaceutical compositions suitable for use in the present invention include compositions wherein the antagonists are contained in an effective amount to achieve its intended purpose. Determination of the effective amounts is well within the capability of those skilled in the art, especially in light of the detailed disclosure provided herein.

In addition to the antagonists these pharmaceutical compositions may contain suitable pharmaceutically acceptable carriers comprising excipients and auxiliaries which facilitate processing of the antagonists into preparations which can be used pharmaceutically. The preparations formulated for oral administration may be in the form of tablets, dragees, capsules, or solutions.

The pharmaceutical compositions of the present invention may be manufactured in a manner that is itself known, e.g., by means of conventional mixing, dissolving, granulating, dragee-making, levigating, emulsifying, encapsulating, entrapping or lyophilizing processes.

Pharmaceutical formulations for parenteral administration include aqueous solutions of the antagonists in water-soluble form. Additionally, suspensions of the agents may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the agents to allow for the preparation of highly concentrated solutions.

Pharmaceutical preparations for oral use can be obtained by combining the antagonists with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of doses.

Pharmaceutical preparations which can be used orally include push-fit capsules made of gelatin, as well as soft, sealed capsules made of gelatin and a plasticizer, such as glycerol or sorbitol. The push-fit capsules can contain the active agents in admixture with filler such as lactose, binders such as starches, and/or lubricants such as talc or magnesium stearate and, optionally, stabilizers. In soft capsules, the active agants may be dissolved or suspended in suitable liquids, such as fatty oils, liquid paraffin, or liquid polyethylene glycols. In addition, stabilizers may be added.

Compositions comprising an antagonist formulated in a compatible pharmaceutical carrier may be prepared, placed in an appropriate container, and labelled for treatment of osteoporosis and other conditions where treatment involves promoting bone formation and/or inhibiting bone resorption.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Many of the active agents may be provided as salts with pharmaceutically compatible counterions. Pharmaceutically compatible salts may be formed with many acids, including but not limited to hydrochloric, sulfuric, acetic, lactic, tartaric, malic, succinic, etc. Salts tend to be more soluble in aqueous or other protonic solvents that are the corresponding free base forms.

For any antagonist used in the method of the invention, the therapeutically effective dose can be estimated initially from cell culture assays. For example, a dose can be formulated in animal models to achieve a circulating concentration range that includes the IC₅₀ as determined in cell culture (i.e., the concentration of the test compound which achieves a half-maximal inhibition of the PTP activity). Such information can be used to more accurately determine useful doses in humans.

A therapeutically effective dose refers to that amount of the antagonist nucleic acids that results in amelioration of symptoms or a prolongation of survival in a patient. Toxicity and therapeutic efficacy can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Antagonists which exhibit large therapeutic indices are preferred. The data obtained from these cell culture assays and animal studies can be used in formulating a range of dosage for use in human. The dosage of such antagonists lies preferably within a range of circulating concentrations that include the ED50 with little or no toxicity. The dosage may vary within this range depending upon the dosage form employed and the route of administration utilized. The exact formulation, route of administration and dosage can be chosen by the individual physician in view of the patient's condition. (See e.g. Fingl et al., 1975, in "The Pharmacological Basis of Therapeutics", Ch. 1 p1).

Dosage amount and interval may be adjusted individually to provide plasma levels of the active agents which are sufficient to maintain the desired inhibitory effects. Usual patient dosages for systemic administration range from 1-2000 mg/day, commonly from 1-250 mg/day, and typically from 10-150 mg/day. Stated in terms of patient body weight, usual dosages range from 0.02-25 mg/kg/day, commonly from 0.02-3 mg/kg/day, typically from 0.2-1.5 mg/kg/day.

Dosage amount and interval may be adjusted individually to provide plasma levels of the active agents which are sufficient to maintain the Rspondin inhibitory or promoting effects. Usual average plasma levels should be maintained within 50-5000 µg/ml, commonly 50-1000 µg/ml, and typically 100-500 µg/ml.

Alternately, one may administer the active agents in a local rather than systemic manner, for example, via injection directly into a target site often in a depot or sustained release formulation.

Furthermore, one may administer the pharmaceutical composition in a targeted drug delivery system, for example, in a liposome coated with target-specific antibody. The liposomes will be targeted to and taken up selectively by the target site.

In cases of local administration or selective uptake, the effective local concentration of the pharmaceutical composition may not be related to plasma concentration.

The Rspo2, Rspo3, Sdc or Glp nucleic acids or compounds capable of binding to Rspo2, Rspo3, Sdc or Glp polypeptides or Rspo2, Rspo3, Sdc or Glp nucleic acids, such as antibodies or nucleotide probes, may be used for diagnostic purposes for detection of Rspo2, Rspo3, Sdc or Glp expression e.g. in proliferative disorders as indicated above.

Reagents suitable for detecting Rspo2, Rspo3, Sdc or Glp polypeptidese or Rspo2, Rspo3, Sdc or Glp nucleic acids or compounds capable of binding to Rspo2, Rspo3, Sdc or Glp polypeptides or Rspo2, Rspo3, Sdc or Glp nucleic acids may have a number of uses for the diagnosis of processes, conditions or diseases resulting from, associated with and/or accompanied by, aberrant expression of Rspo2, Rspo3 or Sdc1, Sdc2, Sdc3, Sdc4, Glp1, Glp2, Glp3, Glp4, Glp5 and/or Glp6. The diagnostic procedures are preferably carried out on samples obtained from a subject, e.g. a human patient, e.g. samples from body fluids such as whole blood, plasma, serum or urine, or tissue samples such as biopsy or autopsy samples. For example, the Rspo2, Rspo3, Sdc1, Sdc2, Sdc3, Sdc4 or Glp sequence may be used in amplification, e.g. hybridization assays to diagnose abnormalities of Rspondin expression; e.g., Southern or Northern analysis, including *in situ* hybridization assays.

Further, the present invention is explained in more detail by the following Figures and Examples.

### Brief description of the Figures

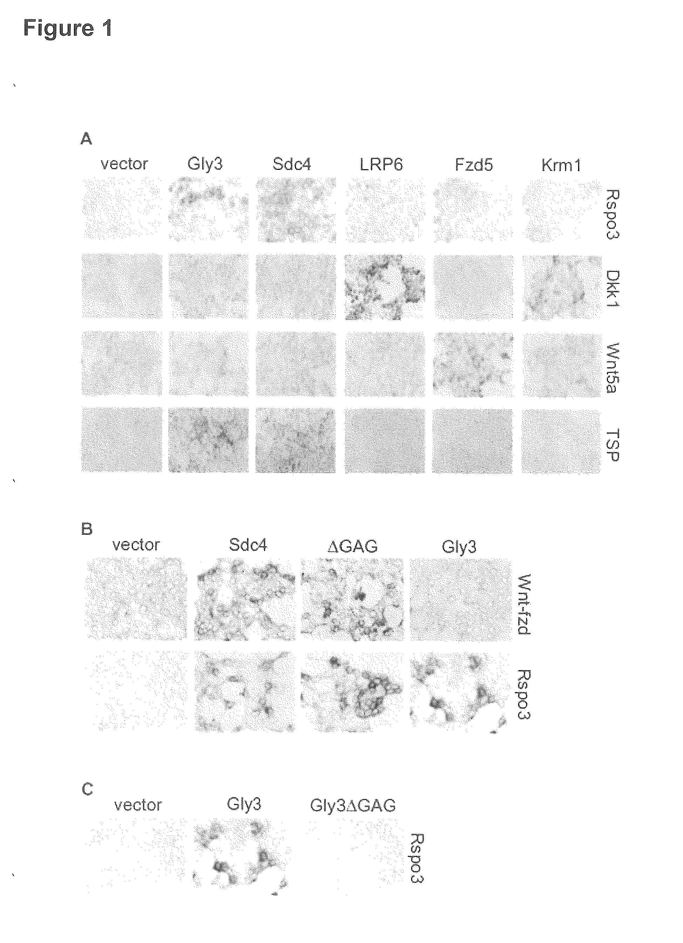

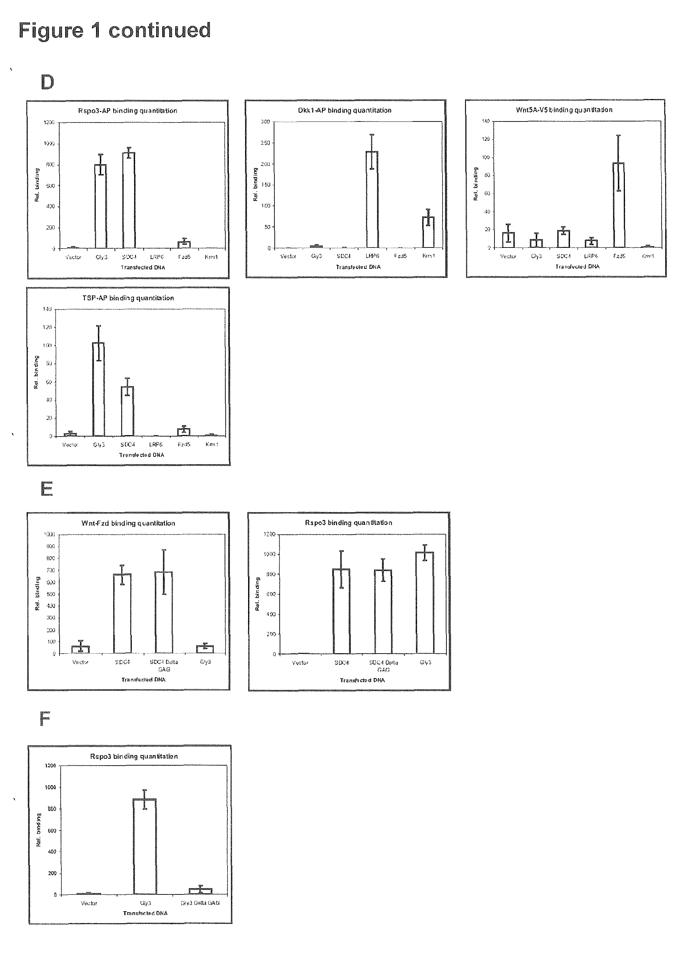

**Figure 1****.** Rspondin-3 interacts with syndecan **4.**
   **(A-C)** Cell surface binding assays. **(A,** D) Rspo3 binds to syndecan 4 and glypican 3. HEK293T cells were transfected with indicated plasmids and V5-tagged Wnt5a or alkaline phosphatase tagged Rspo3, Dkk1, or TSP proteins were applied for cell surface binding as indicated. **(B, C, E, F)** Cells were transfected with indicated plasmids and conditioned media.

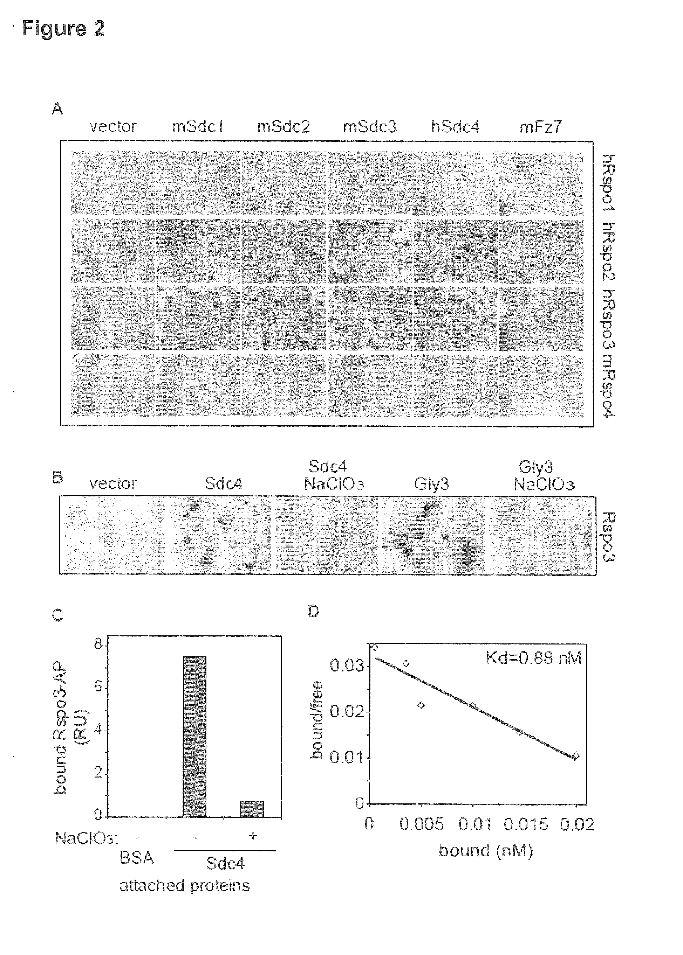

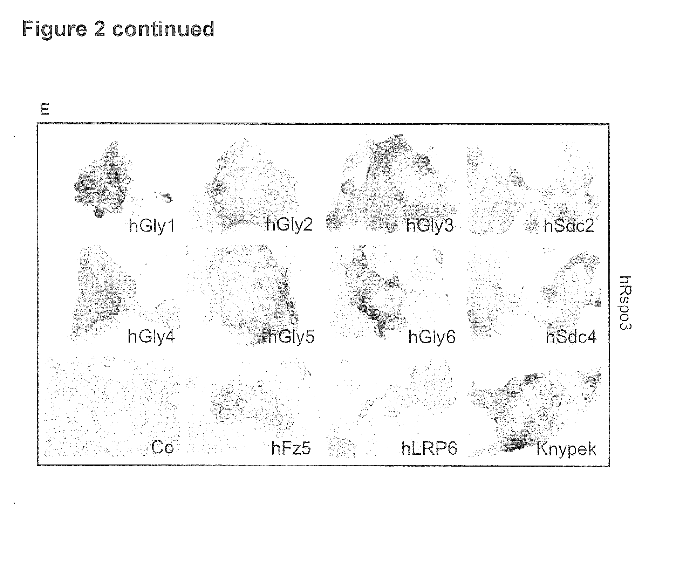

**Figure 2****.** R-spondin 3 binds syndecan 4 and glypicans.
   (A) Rspo2 and Rspo3 bind to Syndecan-1, Syndecan-2, Syndecan-3 and Syndecan-4. Rspo1 and Rspo4 do not bind. HEK293 T-cells were transfected with indicated plasmids and alkaline phosphatase tagged Rspo1, Rspo2, Rspo3 and Rspo4 proteins were applied for cell surface binding as indicated. Cells were transfected with indicated plasmids and conditioned media containing alkaline phosphatise (AP)-fusion proteins of R-spondins were applied for cell surface binding and AP staining as indicated. (B) Cells were transfected with indicated plasmids and incubated with or without NaClO₃. After 24 h Rspo3-AP was applied for binding and proceeded for staining. **(C)** In vitro binding assay with N-streptag Sdc4ΔTMC purified from conditioned media of control or NaClO₃ treated cells with purified Rspo3-AP (RU, relative units). **(D)** Scatchard plot analysis for in vitro binding assays with purified N-streptag Sdc4ΔTMC and Rspo3-AP. **(E)** Rspo3 binds to Glypican-1, Glypican-2, Glypican-3, Glypican-4, Glypican-5 and Glypican-6.
**Figure 3****.** Integrity of Rspo-AP fusion proteins used in Fig. 1A was controlled using Western blot analysis with anti-AP antibody detection.
**Figure 4****:** In vivo [³⁵S] Sulfate metabolic labeling of N-streptag Sdc4ΔTMC in presence or absence of sodium chlorate. Samples were analyzed after streptavidin precipitation by Western blot (left) and autoradiography (right). Note that the majority of Sdc4 at 45 kD is unsulfated.
**Figure 5****.** Rspondin-3 is required for Xenopus gastrulation and non-canonical Wnt signaling.
   **(A)** Loss-of Rspo3 function causes gastrulation defects in *Xenopus* embryos. Top, 4-cell stage embryos were microinjected equatorially into two dorsal blastomeres with 40 ng per embryo of Rspo3 Mo. Note the spina bifida phenotype with two tail ends (arrowheads) in embryos injected with Rspo3 Mo (61%, n=66) and but not control (0%, n=45) embryos. Middle, in situ hybridisation for *Xbra* at gastrula stage (st. 11). Control and Rspo3 Mo embryos showed 100% (n=20) and 95% (n=20) normal *Xbra* staining, respectively. However, note the enlarged blastopore in the Rspo3 Morphant. Bottom, Rspo3 Mo inhibits elongation of Activin injected animal caps.
   (**B**-**D**) Rspo3 signaling requires Sdc4 and Wnt5a to induce gastrulation defects. Embryos were injected into two dorsal blastomeres at 4-cell stage with Morpholinos and/or mRNA as indicated (40ng Rspo3 Mo; 100pg wild type or dominant negative hSdc4 mRNA; 10 or 20 ng of Sdc4 Mo; 2.5 or 10 ng of Wnt5a Mo ("+", and "++"); 250 pg xRspo3 mRNA per embryo were used). **(E)** Confocal microscopy cell protrusion assay. Dorsal or ventral (VMZ) marginal zone at stage 10.5 from embryos injected with membrane-RFP mRNA and 40 ng Rspo3 Mo or 10 ng Wnt5a Mo and/or 50 pg hRspo3 mRNA per embryo were dissociated. White arrowheads indicate protrusions. Right, Quantification of protrusions from three independent experiments (between 300-600 cells counted for each bar). Standard deviation of the mean is indicated. **(F)** Top, nuclear phospho-JNK immunostaining in stage 10.5 dorsal mesoderm from embryos dorsally injected with indicated Morpholinos or mRNAs. Bottom, nuclear Hoechst stain.

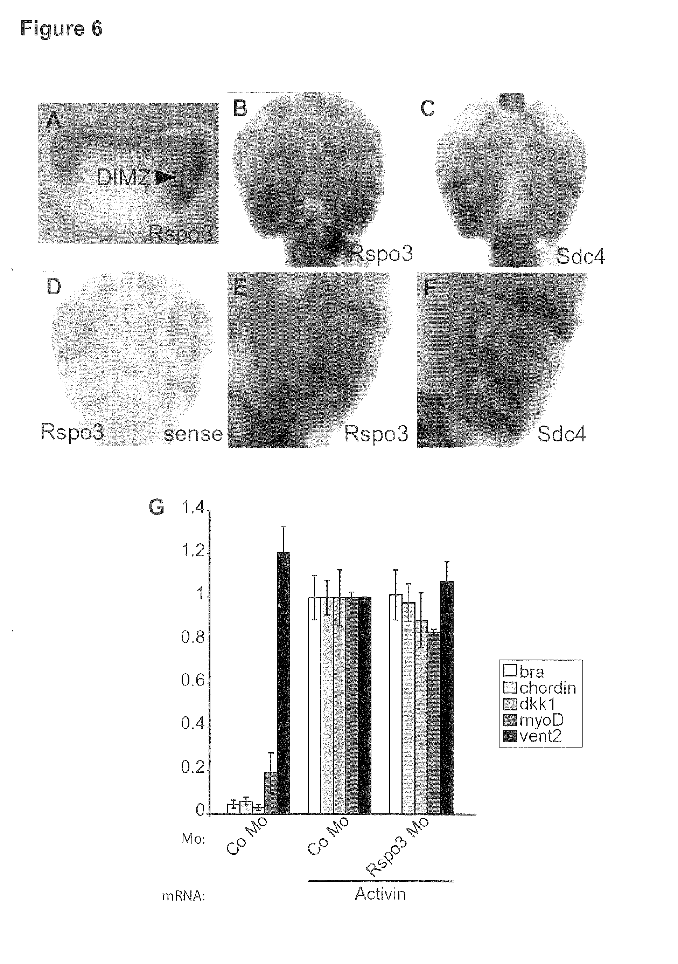

**Figure 6****.** Expression of Rspo3 and Sdc4 in Xenopus embryos **(A-F). (A)** Rspo3 expression in dorsal involuting marginal zone of stage 10 sagitally bisected gastrula and **(B, E)** stage 46 tadpole head cartilage. **(C, F)** Sdc4 expression in stage 46 tadpole head cartilage. **(D)** Sense probe for Rspo3 transcript gives no signal with stage 46 tadpole. **(G)** Expression profiles of the indicated mesodermal marker genes in animal caps injected with activin and Rspo3 Mo.

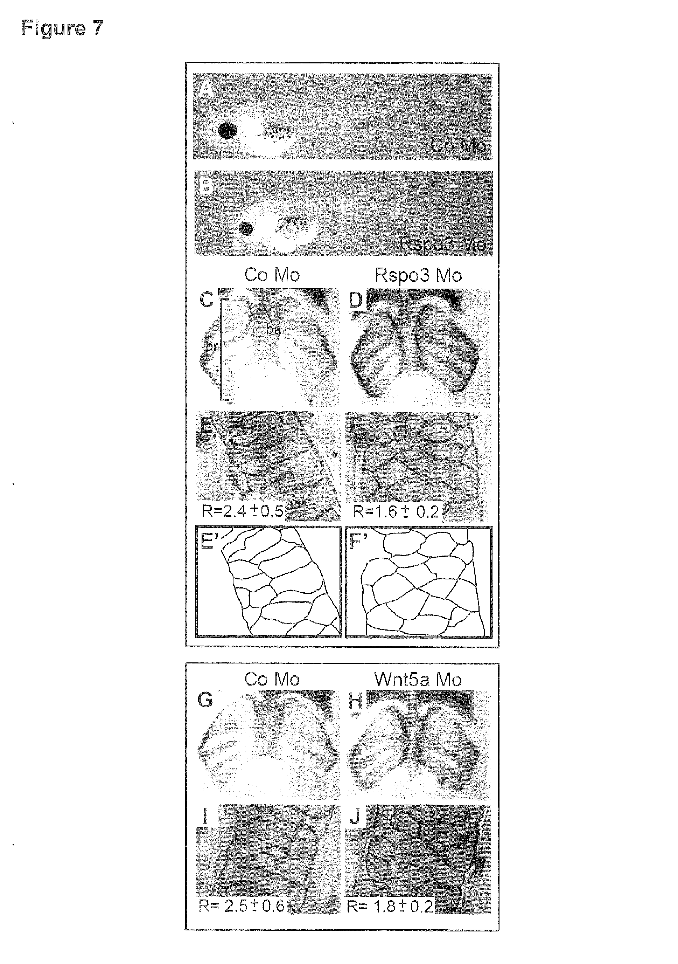

**Figure 7****.** R-spondin 3 is required for head cartilage morphogenesis.
   **(A-B)** Tadpole stage *Xenopus* embryos injected at 8-cell stage with 10 ng per embryo of Rspo3 Mo into the animal four blastomeres, note reduced head in Rspo3 Morphants (68 %, n=98) compared to Co Mo (0.1%, n=85). **(C-D and G-H)** Ceratobranchial cartilage was stained with Alcian blue and dissected from stage 46 embryos injected at 8-cell stage animally with either 10 ng of Rspo3 Mo or 2.5 ng of Wnt5a Mo per embryo. Note compact cartilage elements. **(E-F and I-J)** Ceratobranchial cartilage was dissected from Morpholino injected embryos and flattened. The length-to-width ratio (R) of chondrocytes was determined and are indicated. **(E' and F')** Schematic drawing of cell outlines of E and F. Abbreviations: br, ceratobranchial cartilage; ba, basihyal cartilage.

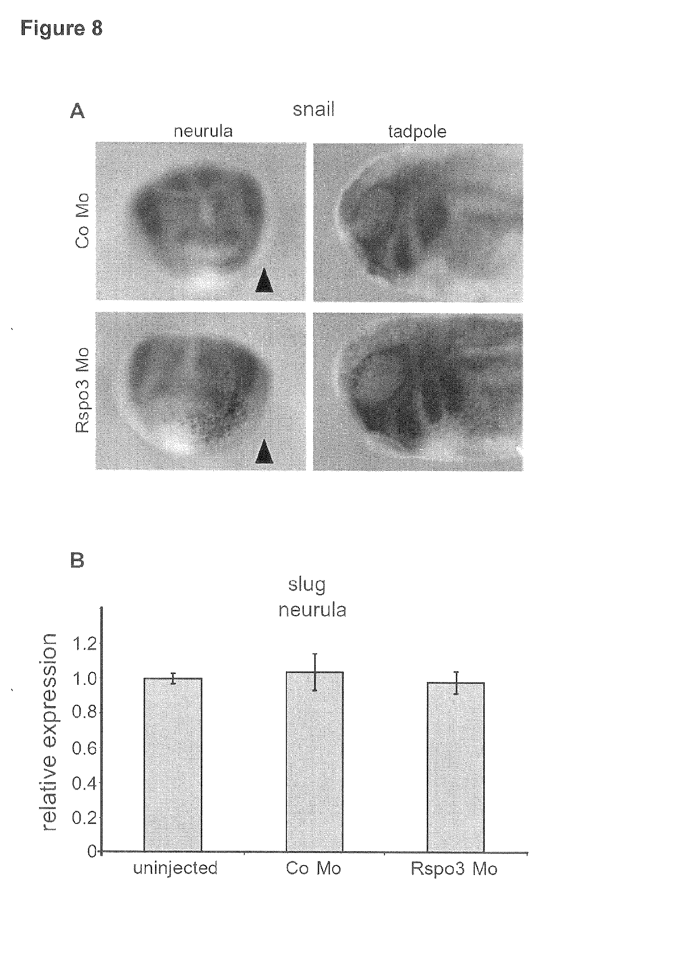

**Figure 8****:** Neural crest markers are unaffected in Rspo3 Morphants.
   **(A)** 8-cell embryos were injected unilaterally with 2.5 ng per blastomere Co Mo or Rspo3 Mo and lacZ lineage tracer into two animal blastomeres. **(B)** Embryos were injected as in (A) but into all animal blastomeres and without lineage tracer. Expression of the indicated marker genes was analyzed at neurula or at tailbud stage as indicated by whole-mount in situ hybridization (A) or by qPCR (B). Rspo3 Mo-injected embryos show unaffected *snail* expression patterns (95%, n=20 for neurula and 95.5%, n=22 for tailbud) as Co Mo-injected embryos (100%, n=21 for neurula and 100%, n=20 for tailbud).

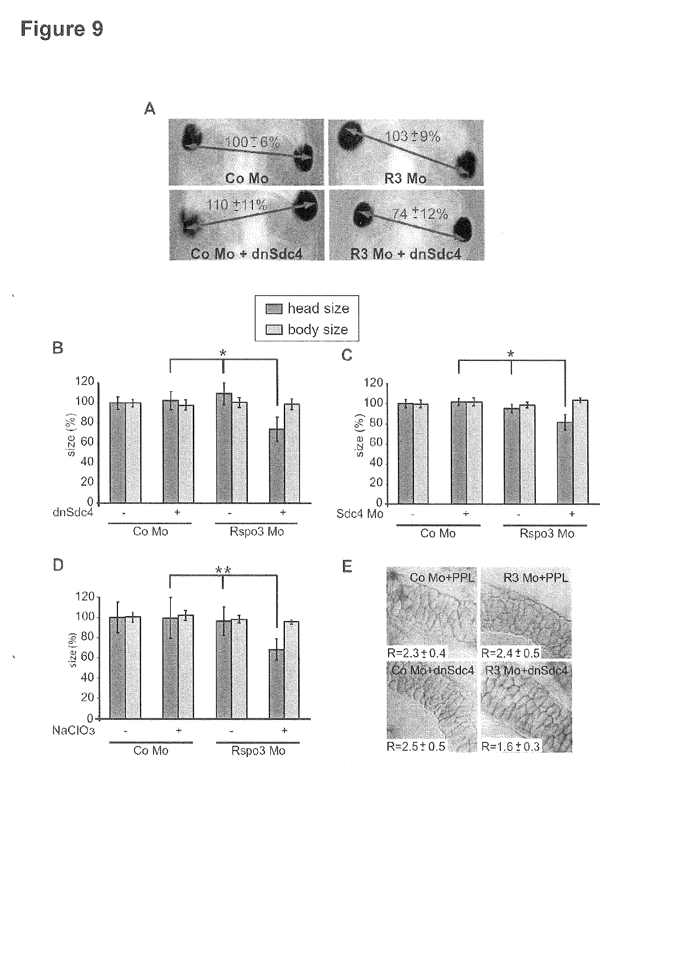

**Figure 9****.** Rspo3 and HSPGs cooperate in head cartilage morphogenesis.
   **(A)** Dorsal view of embryos injected with sub-threshold doses of Rspo3 Mo (5 ng per embryo) and/or dominant negative Sdc4 (dnSdc4, 25 pg per embryo) mRNA. Arrow shows the distance between the eyes; the distance in Co Mo-injected embryo was set as 100% and relative distances are indicated (n= 21-25). Note that head volume approximately reduces with the 3^{rd} power of head diameter. **(B-C)** Combined sub-threshold inhibition of Rspo3 and Sdc4 impairs head cartilage morphogenesis. Statistical analysis of embryos injected with low doses of Rspo3 Mo and/or dnSdc4 mRNA or 1 ng per embryo Sdc4 Mo. Note that head size was specifically reduced while the length of the body axis was unaffected. **(D)** Combined sub-threshold inhibition of Rspo3 and HSPG sulfation impairs head cartilage morphogenesis. Statistical analysis of embryos injected with Rspo3 Mo and/or treated with 60 mM NaClO₃ between stage 20-46. Head diameter and head-tail body length are shown. Head size is significantly reduced by the inhibition of both Rspo3 and Sdc4 (p-values; *<0.005, **<0.01). **(E)** Ceratobranchial cartilage was dissected from Rspo3 Mo and/or dnSdc4 mRNA injected embryos in A and flattened. The length-to-width ratio (R) of chondrocytes is indicated.

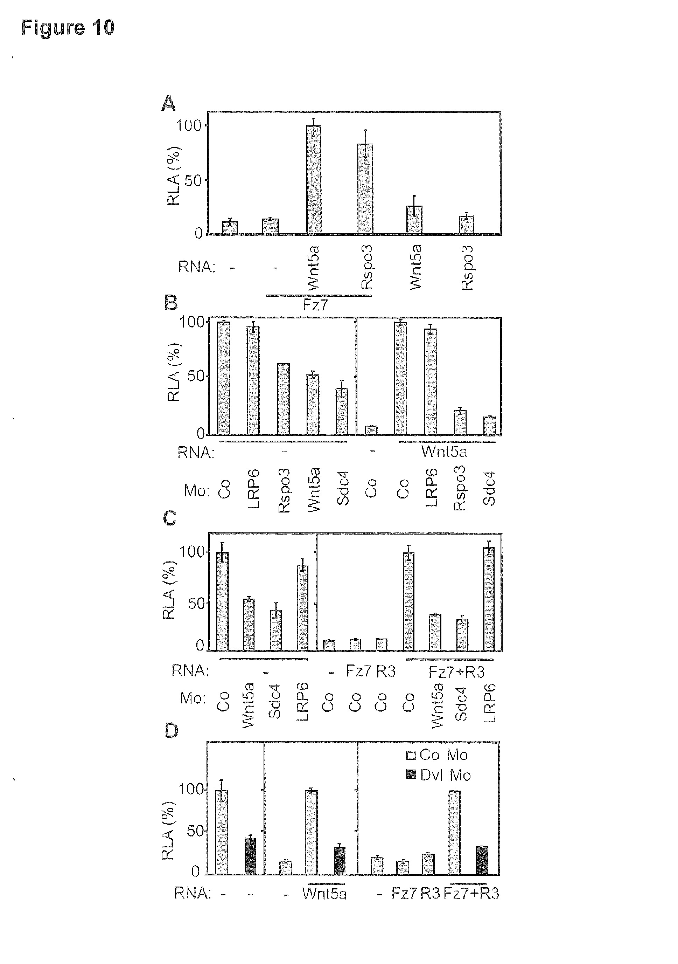

**Figure 10****.** Rspo3/PCP signaling requires Wnt5a, Sdc4 and Dvl.
   **(A-D)** ATF2-Luciferase reporter assay in *Xenopus* embryos. 4-cell stage embryos were injected equatorially with ATF2-Luc reporter plasmid and the indicated antisense Morpholinos and mRNAs. Luciferase reporter assays were carried out from whole embryos lysed at gastrula stage (st. 12). Luciferase activity in embryos injected with either CoMo only or CoMo plus mRNA of the indicated activators within each conditions were set to 100%. RLA, relative luciferase activity.

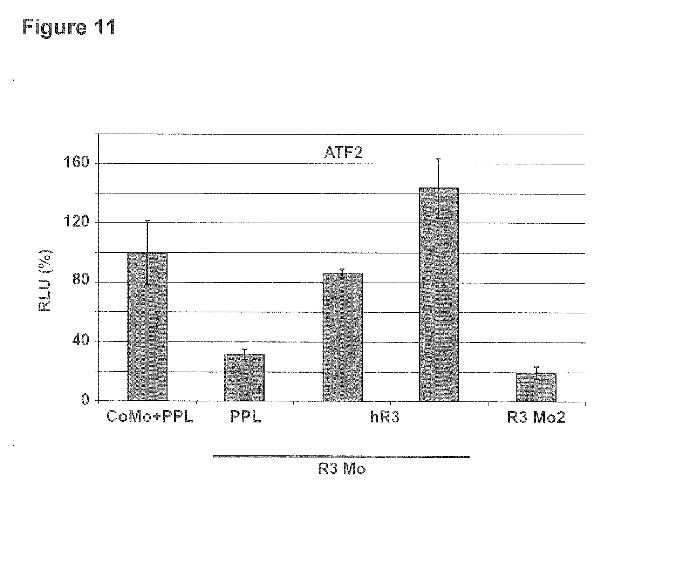

**Figure 11****. Rspo3 Mo specificity**
   ATF2-Luciferase reporter assay in *Xenopus* embryos. 4-cell stage embryos were injected equatorially with ATF2-Luc and Renilla reporter plasmids and two different antisense Morpholinos (R3 Mo; R3 Mo2) against *xRspo3* (40 ng per embryo) and/or hRspo3 mRNA (50 and 100 pg per embryo). Luciferase reporter assays were carried out from whole embryos harvested at gastrula stage (st. 12). Relative luciferase activity in embryos injected with either Co Mo plus *PPL* mRNA was set to 100%. R3 Mo2 is a morpholino having the sequence GCAGTCGCAATTGCATAGTAACCTT and was injected at 40ng per embryos. RLA, relative luciferase activity.

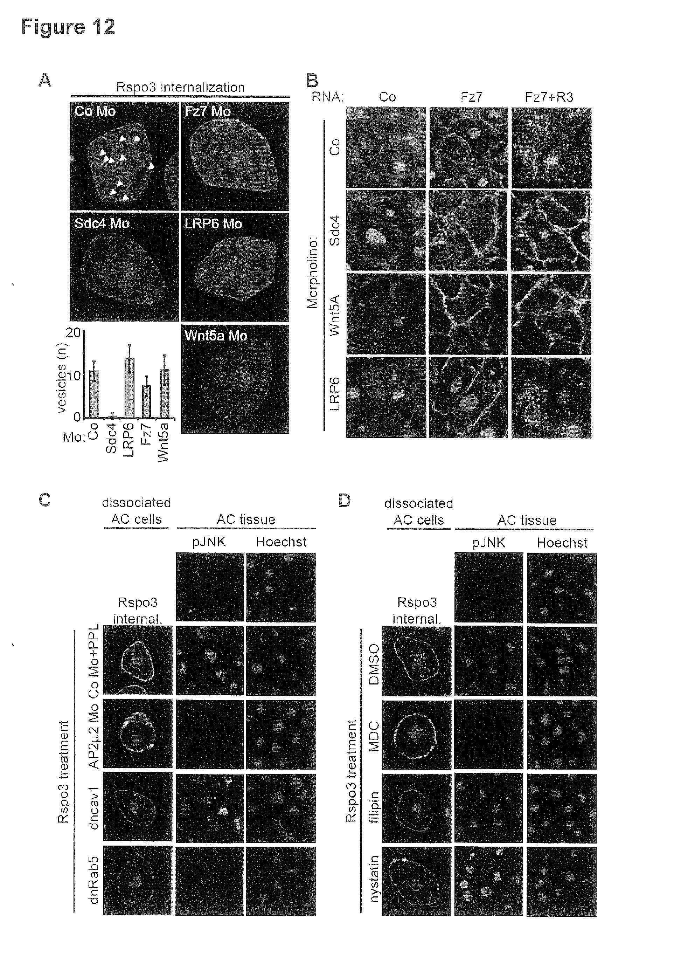

**Figure 12****.** Rspo3 requires clathrin mediated endocytosis to induce phospho-JNK
   **(A)** Confocal microscopy of dissociated animal cap cells treated with hRspo3ΔC-SNAP549 protein for 1 h (orange, arrowheads in CoMo). 4-cell stage embryos were injected animally with membrane-bound Venus mRNA (green) and indicated Morpholinos (Mo) (20 ng Sdc4; 20 ng Fz7; 5 ng of Wnt5a). The average number of vesicles per cell is indicated in the graph. **(B)** Confocal microscopy of Dvl-GFP (green) in stage 8 *Xenopus* animal caps. 4-cell stage embryos were injected animally with indicated Mo and/or mRNAs together with Dvl-GFP mRNA. **(C, D)** Confocal microscopy of animal cap (AC) cells from embryos injected at 4-cell stage with indicated Mo or mRNA **(C)** or treated with endocytosis inhibitors **(D)** as described in Materials and Methods. "Dissociated AC cells", embryos were injected animally with membrane-bound Venus mRNA (green), ACs were explanted and dissociated at stage 8 and incubated for 1 h with hRspo3ΔC-SNAP549 (red), fixed and analyzed. "AC tissue", whole stage 8 ACs were treated either with Protein A (top two panels) or recombinant hRspo3ΔC-streptag-PrA₂ ("Rspo3 treatment") and immunostained with anti-pJNK antibody as described in Materials and Methods. Cells and tissues were counter-stained with Hoechst (blue). Note that clathrin inhibitor treated cells (AP2Δ2 Mo, MDC), bind and cluster Rspo3 at the surface but fail to internalize the protein and to induce phospho-JNK.

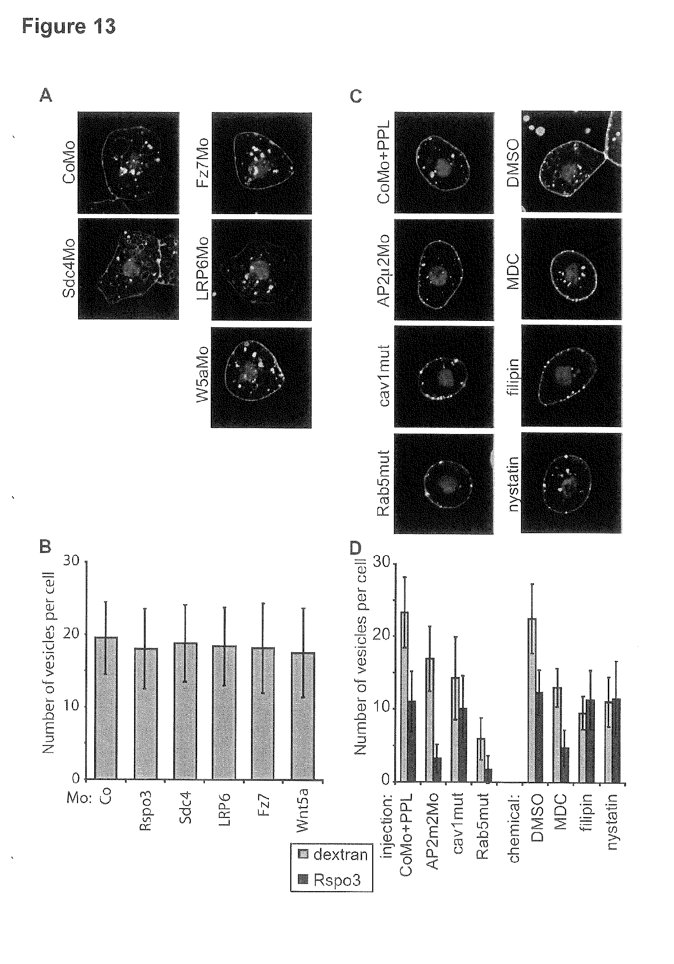

**Figure 13****.** Control of fluorescent dextran internalization. **(A-D)** Fluorescein-Dextran (green) internalization in dissociated animal cap cells injected with membrane bound RFP and indicated Morpholinos or mRNA (A, B) or treated with indicated inhibitor (C, D). Animal cap cells were pre-treated with either DMSO, monodansylcadaverine (MDC), filipin III or nystatin for 45 minutes and incubated with Fluorescein-Dextran and the indicated inhibitors. Bars show the average number of fluorescent vesicles (excluding surface signals) for hRspo3ΔC-SNAP549 protein or Fluorescein-Dextran per cell. Error bars indicate standard deviation of the mean from 3 independent experiments. The scored number of cells are over n>40 (B) and n> 48 cells (D). Nuclear Hoechst staining, blue.

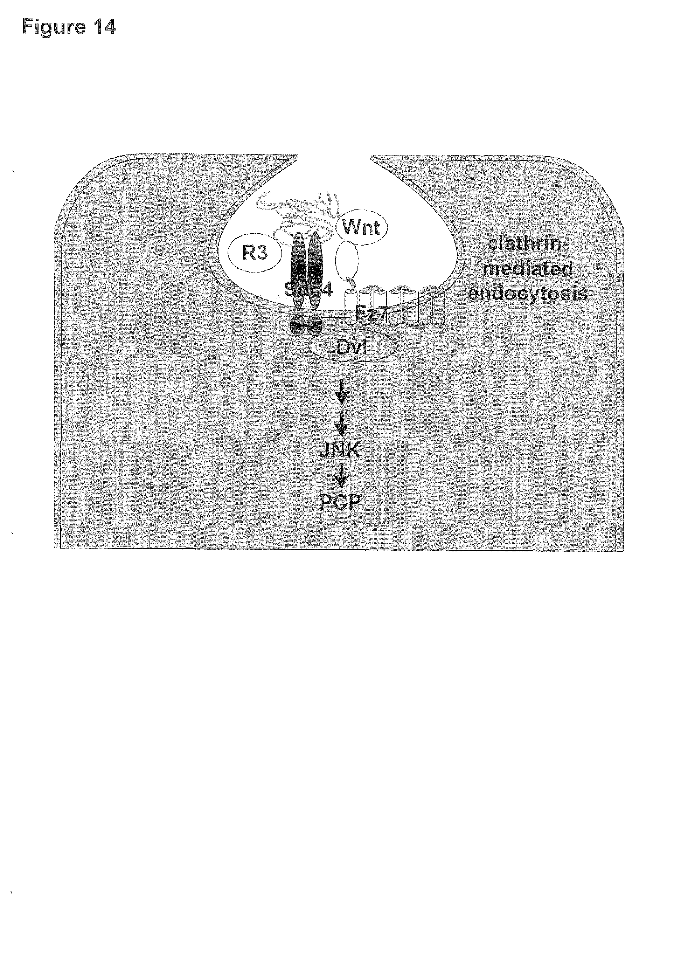

**Figure 14****.** Model for Rspo3 association with a Wnt receptor complex mediating PCP signaling. Endocytosis of Wnt receptor complexes is essential for Wnt/PCP signal transduction. Rspo3 binding to Sdc4 promotes clathrin mediated endocytosis of the Wnt receptor complex and thereby signaling via the PCP pathway.

### Examples

### MATERIALS AND METHODS

### Constructs

All human Rspondin-1, human Rspondin-2, human Rspondin-3, mouse Rspondin-4, mouse Syndecan-1, mouse Syndecan-2, mouse Syndecan-3, human Syndecan-4, and glypican3 constructs were created by PCR and cloned into pCS2+. Constructs were made by adding tags (alkaline phosphatase, HRP or Flag) for example hRspo2 was fused C-terminally with AP to generate hRspo1-AP. Constructs for human Glypican-1, human Glypican-2, human Glypican-3, human Glypican-4, human Glypican-5 and human Glypican-6 were made accordingly. C-terminal deletions of hRspo2 (aa 1-206), hRspo3 (aa1-209) and mRspo4 (aa 1-198) were fused to AP to generate hRspo2ΔC-AP, hRspo3ΔC-AP and mRspo4ΔC-AP respectively. In hRspo3TSP-AP (TSP) the signal peptide was fused to the TSP domain (aa146-209) followed by AP tag. hRspo3ΔC-SNAP was generated by fusing hRspo3 (aa1-209) to the SNAP tag fragment of pSNAP-tag(m) vector (New England Biolabs). phSdc4-EYFP was made by placing EYFP behind full-length syndecan 4. N-streptag *hSdc4* was subcloned by replacing its signal peptide by that of mKremen2 in front of Streptag-HA-CBP fragment of pGLUE expression vector (Angers et al. 2006) pN-streptag-hSdc4ΔC was made by deleting the last 28 amino acids. In pN-streptag-hSdc4ΔGAG serine 63, 97, 95, 138 were replaced by alanine. pN-streptag-hSdc4ΔTMC was cloned by deleting the last 52 amino acids. phGly3 was subcloned in pCS2+. Full length cDNA clones of mSdc1 and mSdc2 in pCMV-Sport6 vector were purchased from imaGenes (clones IRAVp968B1218D and IRAVp968B0992D). mSdc3 was subcloned by cloning imaGenes clone (IRAVp968F09131D) into pCS2+. pN-Flag-hGly3 was cloned in pCS2+ vector with mKremen2 signal peptide. In pN-Flag-hGly3ΔGAG serine 445 and 509 were replaced by alanine. For pPGKmWnt5aV5construct, the CMV promoter in pcDNA3 was replaced by PGK promoter, and then *mWnt3a* was subcloned in the resulting vector followed by V5 tag. pFzd5-GFP is described in Kikuchi (Yamamoto et al. 2006), pCaveolin-GFP is described in Helenius (Pelkmans et al. 2004). Wnt8-Fzd5 fusion is described in Williams (Holmen et al. 2002). Full length cDNA clones hGlp1, hGlp2, hGlp3, hGlp4, hGlp5 and hGlp6 were purchased from ImaGenes (clones IRAUp969F11104D, IRATp970D1144D, IRAUp969D0389D, IRATp970D0937D, IRATp970B0558D, and IRAMp995J1814Q). Clones for hGlps2-5 were used as they were. The clone of hGlp1 was cloned in pcs2+ vector under control of the CMV promoter.

### Cell culture, conditioned media, transfections and endocytosis inhibitors.

HEK293T cells were maintained in DMEM, 10% fetal calf serum (FCS) and 10% CO₂. Wherever recombinant proteins are mentioned, conditioned media were used. Wnt3a and Rspo3 conditioned media were prepared as described, respectively, in (Mao et al. 2001) and Kazanskaya et al. 2004). All plasmid transfections were performed with FuGene6 (Roche), and cells were harvested 24 hours post transfection. For recombinant protein production to avoid any contamination of preparations with polycathions 293T cells were transiently electroporated with corresponding DNA using Neon™ Transfection System (Invitrogen). If indicated, 24 h after transfection or electroporation cells culture medium was supplemented with 25 mM NaClO3 and incubated another 24 h.

### Luciferase reporter assays and siRNA transfections

For luciferase reporter assays in *Xenopus* embryos, embryos were injected with ATF2-luciferase (van Dam et al. 1995) and pRenilla-TK plus Mo and/or synthetic mRNA (typically 50pg per embryo). Three pools of 7 embryos each were lysed with passive lysis buffer (Promega) and assayed for luciferase activity using the Dual luciferase system (Promega).

### Cell surface binding

Protein conditioned media were produced by transient transfection of HEK293T cells with plasmids encoding fusion proteins: hRspo1-AP, hRspo2ΔC-AP, hRspo3ΔC-AP, hRspo3TSP-AP, and dkk1-AP in complete DMEM. XWnt8-hFzd5 fusion was produced in serum-free medium (OPTIMEM I, Gibco) and conditioned medium was concentrated about 100-fold using Centricon Plus-20 filters (Millipore). Wnt5aV5 protein conditioned media was produced using L cells stable transfected with pWnt5aV5. For cell-surface binding experiments, 293T cells were plated on poly-lysine coated plates, transfected with genes to be tested using FuGENE 6 (Roche) for 48 h, incubated with conditioned media containing fusion proteins for 2 h on ice, washed with Hank's buffer, fixed for 30 min with 0,5mM DSP (Pierce) in Hank's-100mM HEPES, pH 7,2, washed with 0,1M Tris pH 8,0 and stained with Fast Red (Roche). Red staining that shows some cells is indicative for binding of tested fusion protein to transfected gene. It should be compared with background staining obtained from cells transfected with control plasmid or other genes that are not able bind applied fusion protein.

### In vitro binding assay

For *in vitro* binding experiments hRspo3ΔC-AP was partially purified from conditioned medium in two steps. hRspo3ΔC-AP from conditioned medium was absorbed to Heparin agarose beads (Sigma, Type I), washed with TBS, and eluted with 0.5 M NaCl, 20 mM Tris, pH7.5. Eluted material was loaded on Concanavalin A agarose (Sigma), washed with 0.5 M NaCl, 20 mM Tris, pH7.5 and eluted with 0.5 M NaCl, 1mM MgCl₂, 100 mM methyl-α-D-manno-pyranoside, 20 mM Tris, pH7.5. Concentration of eluted fusion protein was determined from Coomassie stained SDS PAGE gels using BSA as standard.

N-streptag Sdc4ΔTMC protein was partially purified from conditioned medium by absorbing on Streptavidin Agarose (Thermo Scientific), washing with 0.5 M NaCl, 20 mM Tris, pH 7.5 protein and elution with 2 mM biotin in same buffer. Eluted protein was diluted to 150 mM NaCl and bound to DEAE Sephadex A-50. Beads were washed with TBS to remove biotin and eluted with 1 M NaCl, 20 mM Tris, pH 7.5. Eluted protein was dialyzed against TBS and kept at -20°C before use.

For the *in vitro* binding assay white, high binding ELISA 96 well plates (Greiner) were coated overnight at 4°C with 100 µL of 2 µg/mL Streptavidin in bicarbonate buffer (NaHCO₃, 50 mM, pH 9,6). Wells were washed 6 times with 230 µL TBST and incubated with 230 µL of blocking buffer (5% BSA, 1mM MgCl₂ on TBST) on a shaker for 1 h at RT. The wells were loaded with 100 µL N-streptag Sdc4ΔTMC protein in blocking buffer and allowed to bind overnight on a shaker at 4°C. Wells were washed 6 times with 230 µL TBST and incubated with 230 µL of blocking buffer for 1 h. For the binding experiments, 100 µL of serially diluted hRspo3ΔC-AP were placed in wells coated with blocking buffer or N-streptag Sdc4ΔTMC. After 2 h incubation at RT wells were washed 6 times with 230 µL TBST. Bound AP activity was measured using chemiluminescent SEAP Reporter Gene Assay (Roche). For each dilution, background binding value was subtracted from hRspo3ΔC-AP value. Binding data were analyzed by Scatchard plot using Excel.

### In vivo [³⁵S] Sulfate metabolic labeling

HEK293T cells transiently transfected with N-streptag Sdc4ΔTMC or GFP control were metabolically labeled with 0,2 mCi/ml [³⁵S] Sulfate according to (Tooze, 2001).

After 24 h labeling conditioned medium was harvested, supplemented with 1% NP40, 10 mM cold Na₂SO₄ and incubated overnight with streptavidin beads. Beads were washed with 0.5M NaCl, 1mM Na₂SO₄, 1% NP40, 20 mM Tris pH 7.5. Bound labeled N-streptag Sdc4ΔTMC was eluted with 2mM biotin in the same buffer and analyzed by Western blot and autoradiography.

### SNAP tag labeling

Conditioned medium containing hRspo3ΔC-SNAP was partially purified at 4°C on Heparin agarose as described for hRspo3ΔC-AP, with the modification that all buffers were supplemented with 1 mM mercaptoethanol. Eluted protein was concentrated on Amicon filter (Millipore) and labeled for 3h at RT with SNAP-surface 549 substrate according to the manufacturer (New England Biolabs). Labeled hRspo3ΔC-SNAP549 protein was separated from free substrate on a Sephadex G50 column (final concentration, A₅₅₆=0,93) and kept in aliquots at -20°C.

### Xenopus methods

*In vitro* fertilization, embryo culture, preparation of mRNA, microinjection, and culture of embryo explants were carried out as described (Gawantka et al. 1995). Whole-mount *in situ* hybridization was carried out according to (Bradley et al. 1996). The mRNA doses for injections were as indicated in the legends and otherwise were (pg per embryo): 250 Fz5, 100 XWnt11, 250 XFz7, 200 XDsh-GFP, 250 membrane-bound RFP or Venus (gift from N. Kinoshita, Kinoshita et al, 2003), 500 ΔDIX and ΔDEP of *Xenopus Dishevelled,* 500 dominant negative Rab5 mutant (gift of M Zerial, Bucci et al, 1992) and dominant negative caveolin-1 (YF mutant, gift of C. Mastik, Sanguinetti et al, 2003). The antisense Morpholino oligonucleotide doses for injections were (ng per embryo): 20 Sdc4Mo (Munoz et al. 2006), 2.5 LRP6Mo (Hassler et al. 2007), 10 Wnt5a (Schambony and Wedlich 2007) 10 or 40 Rspo3 (Kazanskaya et al. 2007), 25 Dsh (Sheldahl et al. 2003). Equal amount of total Mo were injected by adjustment with the standard control Mo (Gene Tools), where necessary. For protrusion assays dorsal or ventral mesoderm cells from stage 10.5 embryos injected as described in legend were dissected, dissociated in Ca²⁺-free MBS and cultured in 0.5x Barth including 1% BSA (BSA-Barth) for 1 hour on a fibronectin-coated plate (50 µg/mL). Alcian Blue staining of head cartilage was carried out as described (Berry et al., 1998). To prepare head cartilage, tadpoles were treated with Proteinase K (100 µg/mL in PBS) for 2 hours followed by 0.05% Trypsin (GIBCO) for 3 hours and head cartilage was manually dissected out under a stereo microscope.

### Immunostaining, internalization assays, and Rspo3 treatment of Xenopus embryonic cells

To monitor JNK phoshorylation, stage 10.5 dorsal marginal zones were dissected and fixed in Dent's fixative overnight at -20 °C. After rehydration explants were blocked in 20% horse serum, 1% blocking reagent (Roche Molecular Biochemicals) in PBST (0.1% Tween in PBS). Incubation with the primary anti-pJNK antibody (V7931, Promega, 1:1000) overnight at 4 °C was followed by anti-rabbit Alexa488 (1:1000) for 4 h at RT and Hoechst staining. To monitor Rspo3 internalization, stage 8 animal caps were dissected from animally injected embryos, dissociated in Ca²⁺-free MBS and cultured in BSA-Barth together with either hRspo3ΔC-SNAP549 protein (1:40) or Fluorescein-Dextran (1 µg/mL) (Molecular Probes) for 1 hour. Cells were washed twice with BSA-Barth, fixed with MEMFA for 15 minutes and Hoechststained. For endocytosis inhibitor treatments, dissociated animal cap cells were pre-treated with MDC (monodansylcadaverine, 1mM, Sigma), filipin III (300ng/mL, Sigma), nystatin (25µg/mL, Sigma), or 2% DMSO for 45 minutes and continued for 1 h in the presence of hRspo3ΔC-SNAP549 (1:40) in BSA-Barth. To induce and detect phospho-JNK in animal caps, microscopy cover-glasses were pre-treated with IgG (4.5µg/mL IgG (Sigma) in 50 mM NaHCO₃, pH 9.6) overnight at 4°C, washed with TBST, blocked with 5% BSA in TBST for 1 hour, and then loaded with hRspo3ΔC-streptag- PrA₂ conditioned medium or Protein A (1µg/mL, Amersham) proteins overnight at 4 °C. The glass was used after washing with BSA-Barth. Stage 8 animal caps were dissected from injected embryos and pre-treated 45 min. open face-up under a BSA treated cover glass with endocytosis inhibitors at the mentioned doses. The cover glass was then replaced by a hRspo3ΔC-streptag- PrA₂ or Protein A loaded cover glass and incubation in presence of inhibitors was continued for 1 hour. Animal caps were removed, fixed in Dent's fixative overnight at -20 °C, rehydrated, and immunostained with anti-pJNK antibody as described. Confocal laser scanning was done on a Nikon e-C1plus microscope.

### Interaction assay Syndecan4 with Rspondin3:

An ELISA based assay can be used to see interaction between Rspondin3 and Syndecan 4. For this Syndecan4 containing membranes are coated overnight at 4°C into 96 well or 384 well plates. Full length Syndecan4 coding sequence was fused with a 6His Tag for detection and cloned into pTT5 vector for transient transfection in 293-6E cells as described in Durocher et al. (see ref). The expression level was about 30 mg/L. For Syndecan4 membranes preparation cell pellets were homogenized, dounced followed by centrifugation at 40,000g for 30 minutes. The pellet is washed and resuspended and used for coating. Syndecan4 membranes are coated at various concentrations ranging from 1 µg/mL to 20 µg/mL. Coated plates are washed and blocked using PBS containing 2% BSA for 2 hours. After washing the blocked plates, different amounts of Rspondin3-alkaline phosphatase fusion proteins are loaded onto the coated wells (and non-coated wells as control) and incubated at room temperature for 1-2 hours. After washing the plates, bound Rspondin3-alkaline phosphatase fusion is detected by adding MUP, a substrate for alkaline phosphatase (fluorescent readout, excitation at 340 nm and emission at 450 nm).

### R-Spo3-ALPL fusion protein preparation

Different length of the coding sequences for human and rat RSpo3 (corresponding to AA1-132, AA1-207 and AA1-273) were fused with the coding sequence of a carboxy-terminal deleted ALPL protein (AAS 1-502). These constructs were then cloned into pTT5 vectors and transfected into 293-6E cells as described in Durocher et al. Conditioned media containing these proteins were recovered 120 hrs post-transfection (expression level > 25 mg/L) and centrifuged at 1000 x *g* for 10 min to discard cell debris before storage at - 20 °C until use. All the purification works were done at 4 °C using Akta chromatography machines (GE Healthcare). 10X buffer was first added to the CM for a final concentration of 50 mM Hepes pH 7.5, 300 mM NaCl, 10 mM Imidazole. These materials were then clarified by centrifugation 1 hr at 15,000 x *g* at 4 °C and loaded on HisTrap 5 mL column (GE Healthcare). The fusion proteins eluted at about 320 mM Imidazole in 50 mM Hepes pH 7.5, 350 mM NaCl, 0.1% CHAPS. Protein analysis using Coomassie-Blue stained SDS-PAGE showed that these proteins were about 95% pure. A unique band was observed for RSpo3(1-102)-ALP(1-502) fusion protein. A doublet was observed for RSpo3(1-207)-ALP(1-502) and RSpo3(1-272)-ALP(1-502) fusion proteins.

### Results and Discussion

### Syndecan 4 is an R-spondin 3 receptor

In search of an R-spondin receptor we noted that thrombospondin (TSP1) domains as they are present in R-spondins, are known to bind to HSPGs (Chen et al. 1996). Indeed, in cell surface binding assays (Figure 1A and 1D) both full-length Rspo3 or its TSP domain bound specifically to cells transfected with *glypican3* or *syndecan4 (Sdc4).* In contrast, no Rspo3 binding was detected with *LRP6, Kremen1* or *Frizzled5 (Fzd5)* transfected cells, which however bound recombinant Dkk1 and Wnt5a, respectively.

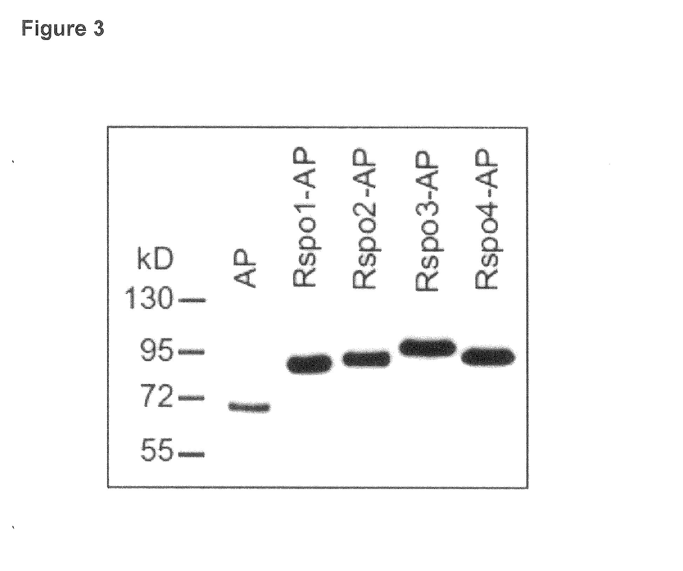

We also tested binding of AP-fusions of all R-spondins to all syndecans. We found that Rspo2 and Rspo3 bind to all syndecans, while Rspo1 and Rspo4 showed no detectable binding under these conditions (Figure 2A). None of the R-spondins bound to Fz7 transfected cells. All four AP-fusion proteins were secreted (Figure 3) and active in Wnt reporter assays indicating that they are biologically active. The absence of Rspo1 and Rspo-4 binding to syndecans correlates with their significantly lower Wnt signaling activity in reporter assays compared to Rspo2 and - 3 (Kim et al., 2008b).

Unlike Wnt5a alone, a Wnt8-Fzd5 fusion protein bound to *Sdc4* transfected cells, suggesting that Wnt bound to its receptor is preferentially recognized by this HSPG (Figure 1B and 1E). This fusion protein was used as it is a closely related protein to Wnt5A, and its behaviour may be assumed to model that of Wnt5a in these experiments.

Early data suggested that the Rspo3 interaction was mediated by the protein core of Sdc4, since cell surface binding was also observed in cells transfected with a GAG-less *Sdc4* mutant and the same was true for Wnt8-Fzd5 fusion protein binding (Figure 1B and 1E). However further investigation revealed that this mutant still incorporated ³⁵S-sulfate and therefore still contained GAGs..

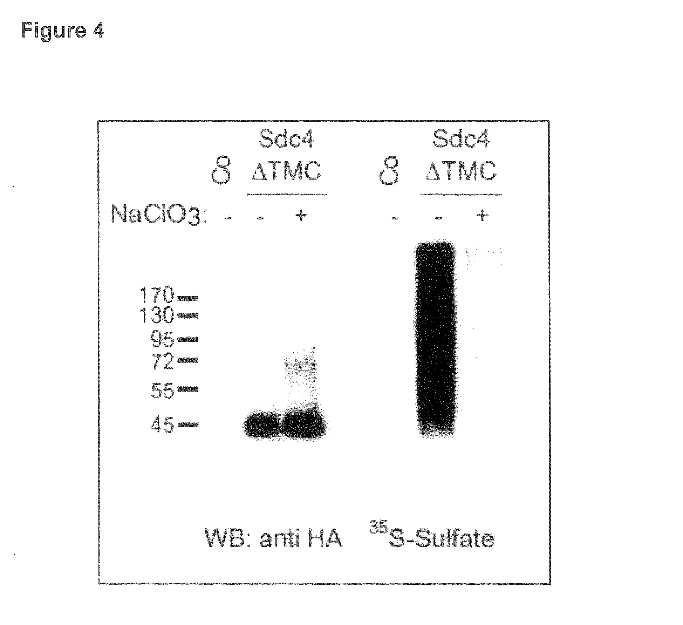

Therefore, as demonstrated herein, the Rspo3-Sdc4 interaction requires GAGs, Sdc4 produced from chlorate-treated cells (Keller et al., 1989), which inhibits sulfation (Figure 4), abolished Rspo3 binding (Figure 2B, 2C). Binding to glypican was also GAG dependent (Figure 1C and 1F).

Using purified extracellular domain of Sdc4 and purified hRspo3-AP we determined the apparent Kd as 0,88 nM (Figure 2D). We conclude that R-spondins bind to syndecans but not to Fz5 or -7, or LRP6, suggesting that these HSPGs may function as high affinity receptors or co-receptors for R-spondins.

Regardless of the mechanism by which Rspondins 2 or 3 interact with Syndecans 1, 2, 3 or 4, a person of skill in the art will appreciate the need to use in any assays a protein that is correctly folded and contains all the essential post-translational modifications required for activity. This is the case regardless of whether the protein is purified from its native environment or expressed in a heterologous manner. Means to ensure that the protein(s) contain all the required post-translational modifications are well within the abilities of a person of skill in the art and additionally are taught herein. In particular a person of skill in the art will appreciate that the use of a protein which produces a functional response in an assay (e.g. the wnt assays as described here) demonstrates that the protein contains all the required post-translational modifications.

### Rspo3 interacts with human Glypicans.

In cell surface binding assays we found that Rspo3 binds to human Glypican-1, Glypican-2, Glypican-3, Glypican-4, Glypican-5 and Glypican-6 (Figure 2E).

### Rspo3 functions in Wnt/PCP signaling during gastrulation

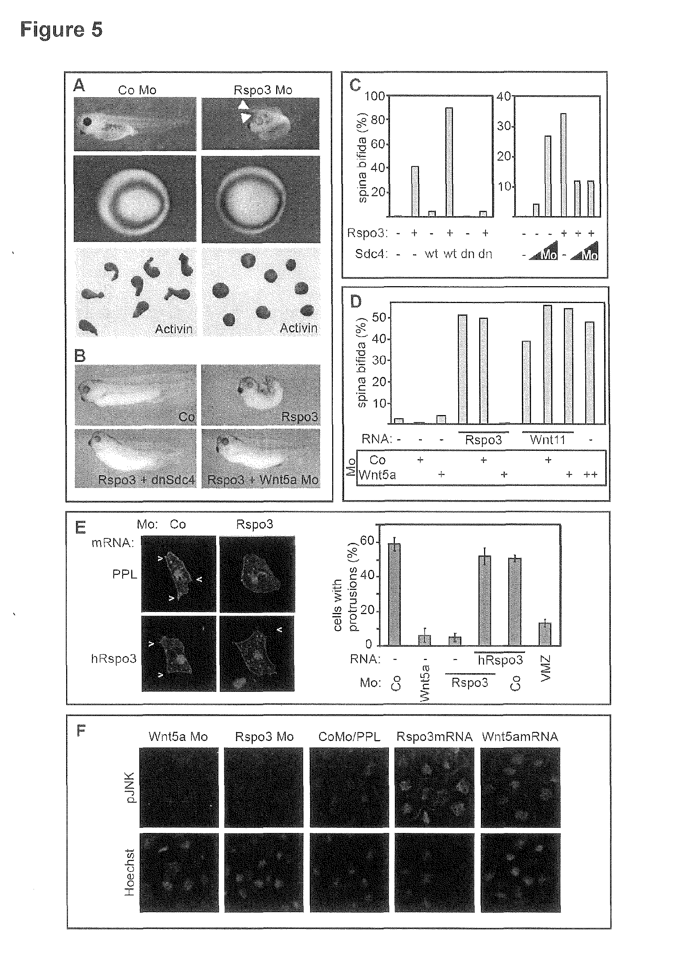

In *Xenopus,* Sdc4 is prominently involved in Wnt/PCP mediated morphogenesis (Munoz et al. 2006; Matthews et al. 2008) and notably promotes gastrulation by regulating mediolateral cell intercalation and convergent extension. This raised the question if Rspo3 signaling may not exclusively activate Wnt/b-catenin as is generally believed, but also Wnt/PCP signaling. Indeed, injection of an *Rspo3* antisense Morpholino, which was previously characterized (Kazanskaya et al. 2007), induced gastrulation defects (spina bifida) when targeting dorsal mesoderm (Figure 5A) and at 10-fold higher dose than used previously (Figure 5A). This Morpholino effect was specific and was almost fully rescued by human Rspo3 mRNA injection (61% spina bifida Rspo3 Mo, n= 66; 4% spina bifida Rspo3 Mo + hRspo3 mRNA, n=45).

Gastrulation is driven primarily by the dorsal mesoderm and *Rspo3* and sdc4 show prominent expression in this region (Figure 6A). Expression of the mesodermal marker *Xbra* in *Rspo3* Morphants was normal (Figure 5A) and the expression of other mesodermal markers including *Xbra, chordin, dkk1, myoD* and *ventx2 (Xvent2)* in activin-injected animal cap tissues were normal (Figure 6G), ruling out that the gastrulation defects were due to reduced mesoderm induction rather than by affecting morphogenesis proper. *Rspo3* Mo also blocked Activin induced animal cap elongation, confirming that *Rspo3* is required for convergent extension movements (Figure 5A).

As is characteristic for genes regulating gastrulation movements, mRNA overexpression of *Rspo3* mRNA also induced gastrulation defects and synergized with *Sdc4* (Figure. 5A, 5B and 5C). This *Rspo3* overexpression effect was almost completely abolished by dominant negative *Sdc4* (Munoz et al. 2006) (Figure. 5B) and by a *Wnt5a* Mo (Schambony and Wedlich 2007) (Figure. 5B and 5D). Wnt5a promotes embryonic Wnt/PCP signaling and gastrulation in *Xenopus* (Schambony and Wedlich 2007). In contrast, *Wnt11* mRNA-induced gastrulation defects were not rescued by *Wnt5a* Mo. This excludes that Rspo3 signaling simply induces expression of a PCP-Wnt gene such as *Wnt11*.

Convergent extension movements are driven by protrusive activity of mesodermal cells (Wallingford et al., 2000; Winklbauer et al., 1996). *Rspo3* Mo inhibited protrusive activity of dorsal mesodermal cells and this was rescued by coinjection of low doses of human *Rspo3* mRNA, confirming Morpholino specificity (Figure. 5E). Wnt/PCP signaling activates JNK phosphorylation to induce convergent extension movements (Yamanaka et al., 2002). Consistently, *Rspo3* and *Wnt5a* mRNA injection induced JNK phosphorylation, while the respective Morpholinos inhibited JNK phosphorylation in dorsal mesodermal cells (Fig. 5F).

Taken together, the results indicate that Rspo3 functions in Wnt/PCP signaling during gastrulation and requires Wnt5a and Sdc4.

### Rspo3 is required for head cartilage morphogenesis

In the course of our analysis we discovered a role of Rspo3 in another Wnt/PCP regulated process, head cartilage morphogenesis. During morphogenesis of the head cartilage, chondrocytes flatten and intercalate to form a column that gives rise to rod- and plate shaped cartilage elements. This intercalation involves chondrocyte elongation and stacking, very reminiscent of dorsal mesodermal cells during gastrulation (Clement et al., 2008; Piotrowski et al., 1996; Schilling et al., 1996). In zebrafish embryos it was shown that like gastrulation, head cartilage morphogenesis depends on Wnt/PCP signaling and HSPGs. Mutations in five PCP genes, including Wnt5a, knypek/glypican 4/6, a transporter of activated sulfate, and two glycosyltransferases required for HSPG synthesis, all interfere with head cartilage morphogenesis, leading to compacted cartilage and stunted head (Clement et al., 2008; Piotrowski et al., 1996; Topczewski et al., 2001).

Similarly, we found that in Xenopus embryos injection of Rspo3 Mo into animal blastomeres targeting the ectoderm (and thus future neural crest) instead of primary mesoderm, induces stunted heads and compacted cartilage (Figure 7A-B). All cartilage elements were present in the Morphants, but they were shorter and thicker (Figure 7C-D), indicating that the phenotype was not due to differentiation or neural crest migration defects. Consistent with this there was no effect of Rspo3 Mo on neural crest marker expression (Figure 8). Instead stacking and elongation of chondrocytes was impaired. The average length-to-width ratio of individual chondrocytes was reduced from 2.4 in wild type to 1.6 in Morphant tadpoles (Figure 7E-F). Compacted head cartilage with impaired stacking and elongation of chondrocytes was also obtained following injection of a previously characterized Wnt5a Mo (Figure 7G-J) (Schambony and Wedlich, 2007).

Moreover, low Rspo3 Mo doses synergized in inducing this phenotype either when co-injected with low doses of dominant-negative Sdc4 mRNA, or Sdc4 Mo, which by themselves elicited no phenotype (Figure 9A-C, E), or when combined with mild chlorate treatment, which inhibits HSPG sulfation (Figure 9D). Rspo3 and Sdc4 are coexpressed in head cartilage during head mesoderm development consistent with a direct role in this tissue (Figure 6F). These results indicate that Rspo3 is required for head cartilage morphogenesis, where it also functionally interacts with Sdc4.

### Cooperation of Rspo3 and Wnt5a is required for Sdc4 mediated PCP signaling

During Wnt/PCP signaling certain Wnt/Fzd combinations activate rho, rac and JNK through Dvl (Angers and Moon 2009; Tada and Kai 2009), leading to activation of the transcription factor ATF2 (Schambony and Wedlich 2007; Zhou et al. 2007). To molecularly corroborate Wnt/PCP activation, we tested various JNK responsive reporters to monitor Wnt/PCP stimulation in *Xenopus* embryos. We identified an JNK responsive-ATF2-luciferase reporter (van Dam et al., 1995), which faithfully monitored Wnt/PCP signaling in gastrulae.

This ATF2 reporter was activated by microinjected *Wnt5a* mRNA in combination with *Fzd7,* a receptor-ligand combination, which mediates Wnt/PCP signaling in *Xenopus* (Kim et al., 2008a). It was also activated by *Rspo3* mRNA in combination with *Fzd7* (Figure 10A). In unstimulated embryos ATF2 reporter activity resulting from endogenous signaling was reduced by Morpholinos targeting *Rspo3, Wnt5a* or *Sdc4,* but not *LRP6* (Figure 10B). Importantly, endogenous reporter activity was reduced by Rspo3 Mo to a similar extend as by Wnt5a and Sdc4 Mo, consistent with the effect of Rspo3 Mo on gastrulation, mesodermal cell protrusive activity and JNK phosphorylation (Figure 10B, left). This effect of Rspo3 Mo on endogenous ATF2 reporter activity was rescued by human Rspo3 mRNA injection, again confirming Mo specificity (Figure 11).

Likewise, reporter activity in *Wnt5a* mRNA exogenously stimulated embryos was blocked by Morpholinos targeting *Rspo3* and *Sdc4.* An *LRP6* Mo, which potently inhibits canonical Wnt signaling (Hassler et al. 2007) had no effect on *Wnt5a* mRNA signaling (Figure 10B), confirming that the reporter is PCP specific. This reveals that Wnt5a/ATF2 signaling in early *Xenopus* embryos requires *Rspo3* as well as *Sdc4.* Since the phenotypic data suggested that also the reverse is true, namely that Rspo3 signaling relies upon endogenous Wnt5a (Figure 5D), we confirmed that ATF2 reporter activity stimulated by *Fzd7*/*Rspo3* was indeed reduced by Morpholinos targeting *Wnt5a* and *Sdc4* but not *LRP6* (Figure 10C). Dvl plays a critical role in Wnt/PCP signaling. Notably, Dvl binding to Sdc4 is required for Fz7-PCP signaling in *Xenopus* (Munoz et al. 2006). Hence we tested whether Rspo3/ATF2 signaling was affected by Dvl knockdown. Injection of a *Dvl2* Mo reduced endogenous, *Wnt5a-* as well as *Fzd7*/*Rspo3* stimulated ATF2 reporter activity (Figure 10D).

From these gain and loss of function data we conclude that in early *Xenopus* embryos i) Rspo3 is required for Wnt/PCP signaling, ii) that its signaling requires Sdc4 and its downstream effector Dvl, consistent with a Rspo3 receptor function, and iii) that Wnt5a and Rspo3 mutually require each other during Wnt/PCP signaling.

Additional experiments were carried out for determining the binding of Rspondins 1-4 to Syndecans 1-4. From these experiments, it can be concluded that Rspo2 and Rspo3 are binding all four Syndecans, but Rspo1 and Rspo4 do not bind to any of the Syndecans under the test conditions (Figure 2A).

### Rspo3 induces clathrin-mediated endocytosis which is required for Wnt/PCP signaling

A hallmark of syndecans is their ability to induce endocytosis following ligand binding, which is essential e.g. for FGF signal transduction (Fuki et al., 2000; Li et al., 2006; Tkachenko et al., 2004; Wittrup et al., 2009). Moreover, there is a body of evidence that Wnt signaling proceeds via an endocytic compartment and that Wnt-receptor complex internalization is an essential step both in canonical and non-canonical Wnt signaling (reviewed in (Kikuchi and Yamamoto, 2007)). This raised the possibility that the molecular mechanism by which Rspo3 promotes Wnt/PCP signaling is by binding Sdc4 and inducing internalization of the Wnt-receptor complex. Internalization assays with recombinant SNAP549-labeled Rspo3 showed that the protein is indeed internalized within 1 h of application in Xenopus animal cap cells, where it was found in intracellular vesicles (Figure 12A). Endocytosis of Rspo3 was inhibited by Morpholinos against *Sdc4, Fz7* and but not *Wnt5a* or *Lrp6,* consistent with its internalization proceeding via an Sdc4/Fz7 complex. To rule out unspecific effects of the Morpholinos on endocytosis, we monitored fluorescent-Dextran uptake, which remained completely unaffected (Figure 13A, 13B).

Dvl plays a key role in endocytosis during Wnt/PCP signaling and upon Wnt signaling is recruited to endocytic vesicles (Chen et al., 2003; Kim et al., 2008a; Kishida et al., 2007; Yu et al., 2007). We therefore monitored the accumulation of Dvl-GFP in vesicles upon stimulation. In animal cap cells, Dvl-GFP shows a diffuse staining but upon coinjection of Fz7 and *Rspo3* mRNA it accumulated in punctate structures. This accumulation was blocked by Morpholinos against *Sdc4, Wnt5a* but not *Lrp6* (Figure 12B). These results support that Rspo3 induces endocytosis of the Wnt receptor complex and Dvl.

The findings raised the question whether Rspo3 mediated endocytosis is merely an epiphenomenon e.g. of receptor clearance or whether the internalization is required for Wnt/PCP signaling to proceed. To test this we treated animal cap cells with Rspo3 protein and after 1 h we monitored either Rspo3 internalization (in dissociated cells) or stained for phospho-JNK (in intact explants) as a read-out for Wnt/PCP activation. Following Rspo3 treatment the labeled protein was internalized and this was accompanied by nuclear phospho-JNK induction, consistent with Wnt/PCP activation (Figure 12C-D, top). Importantly, blocking clathrin mediated endocytosis using a Morpholino targeting the clathrin adaptor *AP2µ2* (Borner et al., 2007; Motley et al., 2003; Yu et al., 2007) led to Rspo3 clustering at the cell surface and impaired internalization and JNK phosphorylation (Figure 12C). This uncoupling of Rspo3 binding from endocytosis provides compelling evidence for the importance of the internalisation for signaling.

Dominant negative *Rab5* mRNA, which inhibits both clathrin and caveolin mediated endocytosis (Shin et al., 2005) also blocked internalisation as well as nuclear phospho-JNK induction. Likewise, the clathrin endocytosis inhibitor monodansylcadaverine (MDC) (Schlegel et al., 1982) blocked Rspo3 internalisation and phospho-JNK induction (Figure 12D). In contrast, inhibitors of caveolin-mediated endocytosis including dominant negative *caveolin* mRNA (Sanguinetti and Mastick, 2003), as well as filipin and nystatin treatment (Rothberg et al., 1992) had no effect on Rspo3 internalisation and phospho-JNK accumulation, while they significantly reduced Fluorescein-Dextran uptake (Figure 13C, 13D).

We conclude that Rspo3 induces clathrin mediated endocytosis and that this internalization is essential for Wnt/PCP signaling.

Since the discovery of Rspondins as novel Wnt effectors, they have attracted increasing attention and great progress has been made characterizing their biology and their implication in disease. In contrast, their mode of signaling and the identity of their receptor remained poorly understood and controversial. Our study sheds light on the mechanism of action of Rspondins, and its major findings are i) the discovery of Syndecans as receptors of Rspo2 and Rspo3; ii) the implication of Rspo2 and Rspo3 in Wnt/PCP signaling; iii) the essential role of Rspo2 and Rspo3 in *Xenopus* gastrulation and head cartilage morphogenesis; iv) its mechanism of action by inducing clathrin mediated endocytosis..

### Rspo3 binds Syndecan 4

We provide independent lines of evidence that Sdc4 functions as Rspo3 receptor or co-receptor, including cell surface binding, recombinant protein binding, functional cooperation, and requirement of Sdc4 for Rspo3/PCP signaling *in vivo.* The affinity of Rspo3 to Sdc4 is similar to the binding of bFGF to Sdc3 (Kd=0.5 nM) and this binding requires heparin chains, (Chernousov and Carey, 1993). Another example is pleiotrophin, which binds Sdc3 with 0.6 nM Kd (Raulo et al., 1994). On the other hand a number of ligands bind syndecans with 10-100 fold lower affinity, for example cathepsin G (56 nM), elastase (35 nM) (Kainulainen et al., 1998) or interleukin 8 (23 nM) (Halden et al., 2004). Thus, Rspo3 can be added to the list of high affinity ligands of syndecans.

Considering the numerous studies implicating syndecans in growth factor signaling and extracellular matrix interaction it is surprising that mice mutants lacking *Sdc1, 3,* or 4 develop without gross developmental abnormalities (Alexander et al., 2000; Ishiguro et al., 2000; Kaksonen et al., 2002). In contrast, R-spondin mutant mice show quite obvious abnormalities, including sex reversal (*Rspo1*), limb and craniofacial defects (*Rspo2*), embryonic lethality (*Rspo3*) and anonychia (*Rspo4*). One likely reason for the rather benign mutant phenotypes in syndecan mutants may be their ubiquitous and high expression, which may promote functional compensation. Yet, closer examination reveals a number of overlapping biological roles between R-spondins and syndecans in vertebrates beyond those described in our study. *Rspo3* and *Sdc2* are essential for angiogenesis (Chen et al., 2004; Kazanskaya et al., 2008) and *Rspo2, Sdc3* and Sdc4 regulate myogenic differentiation (Cornelison et al., 2004; Kazanskaya et al., 2004). *Sdc1* is required for keratinocyte activation during wound healing and *Rspo1* deficient patients show skin defects (Parma et al., 2006; Stepp et al., 2002). Finally, *Rspo2, Rspo3* and *Sdc1* all promote mouse mammary tumorigenesis (Alexander et al., 2000; Lowther et al., 2005; Theodorou et al., 2007).

### Rspo3 functions in Wnt/PCP signaling in Xenopus embryogenesis

In lower vertebrates, HSPGs play a prominent role in Wnt/PCP signaling and morphogenesis. Zebrafish, *glypican4*/*6* (*Knypek*) and Xenopus *Sdc4* regulate gastrulation movements, neural tube closure and neural crest migration (Munoz et al., 2006; Topczewski et al., 2001). In Xenopus gastrulation, Sdc4 functions together with Fz7 to mediate convergent extension and PCP signaling (Munoz et al., 2006). We show that Sdc4/PCP signaling in gastrulae requires Rspo3, revealing that R-spondin function is not limited to Wnt/β-catenin signaling, and consistent with the proposed Rspo3 receptor/co-receptor function of Sdc4. In line with the notion that R-spondins require Wnts to signal, we find that Rspo3 relies upon Wnt5a, which is known to control cell shape and movement during zebrafish and Xenopus gastrulation by engaging the PCP pathway to activate JNK and ATF2 (Cha et al., 2008; Kilian et al., 2003; Ma and Wang, 2007; Schambony and Wedlich, 2007).

Besides gastrulation we show that Rspo3 and Sdc4 function together in another process known to be regulated by Wnt/PCP signaling, namely head cartilage morphogenesis. In zebrafish mutant for various Wnt/PCP components, morphogenesis of chondrocytes is impaired, which fail to polarize and intercalate, and hence show reduced elongation of larval cartilage elements (Clement et al., 2008; LeClair et al., 2009; Topczewski et al., 2001). We show that the same phenotype is exhibited by Xenopus embryos with reduced Rspo3, Sdc4 or Wnt5a function, raising the possibility that these genes may be involved more generally in PCP signaling, possibly including adult skeleton formation. Since Rspo3 mutant mice die during early organogenesis of angiogenic defects (Aoki et al., 2006; Kazanskaya et al., 2008) such an analysis will require conditional mutagenesis.

### Rspo3 induces syndecan 4 endocytosis to activate Wnt/PCP signaling

It is well documented that Wnt binding to its receptor Fz triggers internalization of ligand and receptor (Chen et al., 2003; Dubois et al., 2001; Kurayoshi et al., 2007; Rives et al., 2006). There is now accumulating evidence that Wnt induced endocytosis is not only a mechanism for clearing receptor-ligand complexes or transporting Wnt proteins by transcytosis, but that internalization is an obligatory step in activating signal transduction. For the β-catenin pathway, Wnt-Fz internalization is required for proper signaling (Blitzer and Nusse, 2006; Seto and Bellen, 2006) and caveolae-mediated endocytosis of LRP6 plays a critical role in this process (Yamamoto et al., 2006). Internalization is also required for PCP signaling. The DEP domain of Dvl mediates PCP signaling and interacts with the clathrin adaptor complex 2 (AP-2), which is required for Frizzled-4 internalization and PCP signaling (Yu et al., 2007). One critical regulator in this context is β-arrestin, which regulates Fz endocytosis and PCP signaling in Xenopus (Chen et al., 2003; Kim and Han, 2007). Similarly, a hallmark of syndecans is that their binding to ligands induces endocytosis (Fuki et al., 1997; Fuki et al., 2000; Tkachenko et al., 2004) and this is intimately linked to signaling initiation (Li et al., 2006).

We provide evidence that Rspo3 promotes Wnt signaling via clathrin mediated endocytosis, an internalization route which has previously been implicated in Wnt signaling (Blitzer and Nusse, 2006; O'Connell et al. 2010, ; Yu et al., 2007).

Our data corroborate the importance of endocytosis in Wnt signal transduction and they suggest a model (Fig. 14) whereby syndecan acts as a Wnt-Frizzled coreceptor, whose internalization is regulated by R-spondin.

While the present study discloses its role in Wnt/PCP signaling, Rspo3 is also a potent activator of Wnt/β-catenin signaling. Our data clearly indicate that Rspo3 signals independent of Lrp6 in Wnt/PCP signaling. This is consistent with the notion that Lrp6 directs Wnt signaling towards the β-catenin pathway. The results indicate that R-spondins serve to amplify Wnt ligand signaling in β-catenin as well as PCP signaling

### List of References

Adams, J.C. 1992. Biotin amplification of biotin and horseradish peroxidase signals in histochemical stains. J Histochem Cytochem 40: 1457-63.
Angers S, Thorpe CJ, Biechele TL, Goldenberg SJ, Zheng N, MacCoss MJ, Moon RT. 2006. The KLHL 12-Cullin-3 ubiquitin ligase negatively regulates the Wnt-beta-catenin pathway by targeting Dishevelled for degradation. Nat Cell Biol.4:348-57.
Angers, S. and R.T. Moon. 2009. Proximal events in Wnt signal transduction. Nat Rev Mol Cell Biol 10: 468-77.
Aoki, M., M. Mieda, T. Ikeda, Y. Hamada, H. Nakamura, and H. Okamoto. 2006. R-spondin3 is required for mouse placental development. Dev Biol.
Bass, M.D., M.R. Morgan, and M.J. Humphries. 2009. Syndecans shed their reputation as inert molecules. Sci Signal 2: pe18.
Bellin, R., I. Capila, J. Lincecum, P.W. Park, O. Reizes, and M.R. Bernfield. 2002. Unlocking the secrets of syndecans: transgenic organisms as a potential key. Glycoconj J 19: 295-304.
Berry, D.L., C.S. Rose, B.F. Remo, and D.D. Brown. 1998. The expression pattern of thyroid hormone response genes in remodeling tadpole tissues defines distinct growth and resorption gene expression programs. Dev Biol 203: 24-35.
Binnerts, M.E., K.A. Kim, J.M. Bright, S.M. Patel, K. Tran, M. Zhou, J.M. Leung, Y. Liu, W.E. Lomas, 3rd, M. Dixon, S.A. Hazell, M. Wagle, W.S. Nie, N. Tomasevic, J. Williams, X. Zhan, M.D. Levy, W.D. Funk, and A. Abo. 2007. R-Spondin1 regulates Wnt signaling by inhibiting internalization of LRP6. Proc Natl Acad Sci U S A 104: 14700-5.
Blaydon, D.C., Y. Ishii, A. O'Toole E, H.C. Unsworth, M.T. Teh, F. Ruschendorf, C. Sinclair, V.K. Hopsu-Havu, N. Tidman, C. Moss, R. Watson, D. de Berker, M. Wajid, A.M. Christiano, and D.P. Kelsell. 2006. The gene encoding R-spondin 4 (RSPO4), a secreted protein implicated in Wnt signaling, is mutated in inherited anonychia. Nat Genet 38: 1245-7.
Blitzer, J.T. and R. Nusse. 2006. A critical role for endocytosis in Wnt signaling. BMC Cell Biol 7: 28.
Bradley, L., D. Wainstock, and H. Sive. 1996. Positive and negative signals modulate formation of the Xenopus cement gland. Development 122: 2739-2750.
Bucci C, Parton RG, Mather IH, Stunnenberg H, Simons K, Hoflack B, Zerial M., 1992, The small GTPase rab5 functions as a regulatory factor in the early endocytic pathway. Cell. 70(5):715-28.
Charnaux, N., S. Brule, M. Hamon, T. Chaigneau, L. Saffar, C. Prost, N. Lievre, and L. Gattegno. 2005. Syndecan-4 is a signaling molecule for stromal cell-derived factor-1 (SDF-1)/CXCL12. Febs J 272: 1937-51.
Chen, H., J. Sottile, D.K. Strickland, and D.F. Mosher. 1996. Binding and degradation of thrombospondin-1 mediated through heparan sulphate proteoglycans and low-density-lipoprotein receptor-related protein: localization of the functional activity to the trimeric N-terminal heparin-binding region of thrombospondin-1. Biochem J 318 (Pt 3): 959-63.
Chen, W., D. ten Berge, J. Brown, S. Ahn, L.A. Hu, W.E. Miller, M.G. Caron, L.S. Barak, R. Nusse, and R.J. Lefkowitz. 2003. Dishevelled 2 recruits beta-arrestin 2 to mediate Wnt5A-stimulated endocytosis of Frizzled 4. Science 301: 1391-4.
Clement, A., M. Wiweger, S. von der Hardt, M.A. Rusch, S.B. Selleck, C.B. Chien, and H.H. Roehl. 2008. Regulation of zebrafish skeletogenesis by ext2/dackel and papst1/pinscher. PLoS Genet 4: e1000136.
Durocher, Y., Perret, S. and Kamen, A.. High level and high-throughput recombinant protein production by transient transfection of suspension-growing human 293-EBNA1 cells. Nucleic Acids Research, 2002, vol.30, 2-9.
Ellwanger, K., H. Saito, P. Clement-Lacroix, N. Maltry, J. Niedermeyer, W.K. Lee, R. Baron, G. Rawadi, H. Westphal, and C. Niehrs. 2008. Targeted disruption of the Wnt regulator Kremen induces limb defects and high bone density. Mol Cell Biol 28: 4875-82.
Fuki, I.V., K.M. Kuhn, I.R. Lomazov, V.L. Rothman, G.P. Tuszynski, R.V. lozzo, T.L. Swenson, E.A. Fisher, and K.J. Williams. 1997. The syndecan family of proteoglycans. Novel receptors mediating internalization of atherogenic lipoproteins in vitro. J Clin Invest 100: 1611-22.
Fuki, I.V., M.E. Meyer, and K.J. Williams. 2000. Transmembrane and cytoplasmic domains of syndecan mediate a multi-step endocytic pathway involving detergent-insoluble membrane rafts. Biochem J 351 Pt 3: 607-12.
Gawantka, V., H. Delius, K. Hirschfeld, C. Blumenstock, and C. Niehrs. 1995. Antagonizing the Spemann organizer: role of the homeobox gene Xvent-1. EMBO J 14: 6268-79.
Grigoryan, T., P. Wend, A. Klaus, and W. Birchmeier. 2008. Deciphering the function of canonical Wnt signals in development and disease: conditional loss- and gain-of-function mutations of beta-catenin in mice. Genes Dev 22: 2308-41.
Hassler, C., C.M. Cruciat, Y.L. Huang, S. Kuriyama, R. Mayor, and C. Niehrs. 2007. Kremen is required for neural crest induction in Xenopus and promotes LRP6-mediated Wnt signaling. Development 134: 4255-63.
Holmen, S.L., A. Salic, C.R. Zylstra, M.W. Kirschner, and B.O. Williams. 2002. A novel set of Wnt-Frizzled fusion proteins identifies receptor components that activate beta -catenin-dependent signaling. J Biol Chem 277: 34727-35.
Iwabuchi, T. and P.F. Goetinck. 2006. Syndecan-4 dependent FGF stimulation of mouse vibrissae growth. Mech Dev 123: 831-41.
Kamata, T., K. Katsube, M. Michikawa, M. Yamada, S. Takada, and H. Mizusawa. 2004. R-spondin, a novel gene with thrombospondin type 1 domain, was expressed in the dorsal neural tube and affected in Wnts mutants. Biochim Biophys Acta 1676: 51-62.
Kazanskaya, O., A. Glinka, I. Del Barco Barrantes, P. Stannek, C. Niehrs, and W. Wu. 2004. R-Spondin2 Is a Secreted Activator of Wnt/beta-Catenin Signaling and Is Required for Xenopus Myogenesis. Dev Cell 7: 525-34.
Kazanskaya, O., B. Ohkawara, M. Heroult, N. Maltry, H.G. Augustin, and C. Niehrs. 2007. The Wnt signaling regulator R-spondin3 acts upstream of VEGF to control the switch between angioblastic and hematopoietic cell fate determination. Development.
Kim, G.H. and J.K. Han. 2007. Essential role for beta-arrestin 2 in the regulation of Xenopus convergent extension movements. Embo J 26: 2513-26.
Kim, G.H., J.H. Her, and J.K. Han. 2008a. Ryk cooperates with Frizzled 7 to promote Wnt11-mediated endocytosis and is essential for Xenopus laevis convergent extension movements. J Cell Biol 182: 1073-82.
Kim, K.A., M. Kakitani, J. Zhao, T. Oshima, T. Tang, M. Binnerts, Y. Liu, B. Boyle, E. Park, P. Emtage, W.D. Funk, and K. Tomizuka. 2005. Mitogenic influence of human R-spondin1 on the intestinal epithelium. Science 309: 1256-9.
Kim, K.A., M. Wagle, K. Tran, X. Zhan, M.A. Dixon, S. Liu, D. Gros, W. Korver, S. Yonkovich, N. Tomasevic, M. Binnerts, and A. Abo. 2008b. R-Spondin family members regulate the Wnt pathway by a common mechanism. Mol Biol Cell 19: 2588-96.
Kinoshita N, lioka H, Miyakoshi A, Ueno N. 2003. PKC delta is essential for Dishevelled function in a noncanonical Wnt pathway that regulates Xenopus convergent extension movements. Genes Dev. 17(13):1663-76
Kishigami, S., E. Mizutani, H. Ohta, T. Hikichi, N.V. Thuan, S. Wakayama, H.T. Bui, and T. Wakayama. 2006. Significant improvement of mouse cloning technique by treatment with trichostatin A after somatic nuclear transfer. Biochem Biophys Res Commun 340: 183-9.
Kramer, K.L. and H.J. Yost. 2002. Ectodermal syndecan-2 mediates left-right axis formation in migrating mesoderm as a cell-nonautonomous Vg1 cofactor. Dev Cell 2: 115-24.
Kuriyama, S. and R. Mayor. 2009. A role for Syndecan-4 in neural induction involving ERK- and PKC-dependent pathways. Development 136: 575-84.
Li, X., J. Herz, and D. Monard. 2006. Activation of ERK signaling upon alternative protease nexin-1 internalization mediated by syndecan-1. J Cell Biochem 99: 936-51.
MacDonald, B.T., K. Tamai, and X. He. 2009. Wnt/beta-catenin signaling: components, mechanisms, and diseases. Dev Cell 17: 9-26.
Mao, B., W. Wu, Y. Li, D. Hoppe, P. Stannek, A. Glinka, and C. Niehrs. 2001. LDL-receptor-related protein 6 is a receptor for Dickkopf proteins. Nature 411: 321-325.
Matthews, H.K., L. Marchant, C. Carmona-Fontaine, S. Kuriyama, J. Larrain, M.R. Holt, M. Parsons, and R. Mayor. 2008. Directional migration of neural crest cells in vivo is regulated by Syndecan-4/Rac1 and non-canonical Wnt signaling/RhoA. Development 135: 1771-80.
Munoz, R., M. Moreno, C. Oliva, C. Orbenes, and J. Larrain. 2006. Syndecan-4 regulates non-canonical Wnt signalling and is essential for convergent and extension movements in Xenopus embryos. Nat Cell Biol 8: 492-500.
Nam, J.S., T.J. Turcotte, P.F. Smith, S. Choi, and J.K. Yoon. 2006a. Mouse cristin/R-spondin family proteins are novel ligands for the Frizzled 8 and LRP6 receptors and activate beta-catenin-dependent gene expression. J Biol Chem 281: 13247-57.
Nam, J.S., T.J. Turcotte, and J.K. Yoon. 2006b. Dynamic expression of R-spondin family genes in mouse development. Gene Expr Patterns.
Nusse, R. 2005. Wnt signaling in disease and in development. Cell Res 15: 28-32.
Oh, E.S., A. Woods, and J.R. Couchman. 1997. Multimerization of the cytoplasmic domain of syndecan-4 is required for its ability to activate protein kinase C. J Biol Chem 272: 11805-11.
Olivares, G.H., H. Carrasco, F. Aroca, L. Carvallo, F. Segovia, and J. Larrain. 2009. Syndecan-1 regulates BMP signaling and dorso-ventral patterning of the ectoderm during early Xenopus development. Dev Biol 329: 338-49.
Parma, P., O. Radi, V. Vidal, M.C. Chaboissier, E. Dellambra, S. Valentini, L. Guerra, A. Schedl, and G. Camerino. 2006. R-spondin1 is essential in sex determination, skin differentiation and malignancy. Nat Genet 38: 1304-9.
Pelkmans, L., T. Burli, M. Zerial, and A. Helenius. 2004. Caveolin-stabilized membrane domains as multifunctional transport and sorting devices in endocytic membrane traffic. Cell 118: 767-80.
Piotrowski, T., T.F. Schilling, M. Brand, Y.J. Jiang, C.P. Heisenberg, D. Beuchle, H. Grandel, F.J. van Eeden, M. Furutani-Seiki, M. Granato, P. Haffter, M. Hammerschmidt, D.A. Kane, R.N. Kelsh, M.C. Mullins, J. Odenthal, R.M. Warga, and C. Nusslein-Volhard. 1996. Jaw and branchial arch mutants in zebrafish II: anterior arches and cartilage differentiation. Development 123: 345-56.
Reinke, R. and S.L. Zipursky. 1988. Cell-cell interaction in the Drosophila retina: the bride of sevenless gene is required in photoreceptor cell R8 for R7 cell development. Cell 55: 321-30.
Sanguinetti AR, Mastick CC. 2003. c-Abl is required for oxidative stress-induced phosphorylation of caveolin-1 on tyrosine 14. Cell Signal. 15(3):289-98.
Schambony, A. and D. Wedlich. 2007. Wnt-5A/Ror2 regulate expression of XPAPC through an alternative noncanonical signaling pathway. Dev Cell 12: 779-92.
Schilling, T.F., T. Piotrowski, H. Grandel, M. Brand, C.P. Heisenberg, Y.J. Jiang, D. Beuchle, M. Hammerschmidt, D.A. Kane, M.C. Mullins, F.J. van Eeden, R.N. Kelsh, M. Furutani-Seiki, M. Granato, P. Haffter, J. Odenthal, R.M. Warga, T. Trowe, and C. Nusslein-Volhard. 1996. Jaw and branchial arch mutants in zebrafish I: branchial arches. Development 123: 329-44.
Sheldahl, L.C., D.C. Slusarski, P. Pandur, J.R. Miller, M. Kuhl, and R.T. Moon. 2003. Dishevelled activates Ca2+ flux, PKC, and CamKII in vertebrate embryos. J Cell Biol 161: 769-77.
Speel, E.J., A.H. Hopman, and P. Komminoth. 1999. Amplification methods to increase the sensitivity of in situ hybridization: play card(s). J Histochem Cytochem 47: 281-8.
Tada, M. and M. Kai. 2009. Noncanonical Wnt/PCP signaling during vertebrate gastrulation. Zebrafish 6: 29-40.
Tkachenko, E., E. Lutgens, R.V. Stan, and M. Simons. 2004. Fibroblast growth factor 2 endocytosis in endothelial cells proceed via syndecan-4-dependent activation of Rac1 and a Cdc42-dependent macropinocytic pathway. J Cell Sci 117: 3189-99.
Tkachenko, E. and M. Simons. 2002. Clustering induces redistribution of syndecan-4 core protein into raft membrane domains. J Biol Chem 277: 19946-51.
Topczewski, J., D.S. Sepich, D.C. Myers, C. Walker, A. Amores, Z. Lele, M. Hammerschmidt, J. Postlethwait, and L. Solnica-Krezel. 2001. The zebrafish glypican knypek controls cell polarity during gastrulation movements of convergent extension. Dev Cell 1: 251-64.
van Dam, H., D. Wilhelm, I. Herr, A. Steffen, P. Herrlich, and P. Angel. 1995. ATF-2 is preferentially activated by stress-activated protein kinases to mediate c-jun induction in response to genotoxic agents. Embo J 14: 1798-811.
Wei, Q., C. Yokota, M.V. Semenov, B. Doble, J. Woodgett, and X. He. 2007. R-spondin1 is a high affinity ligand for LRP6 and induces LRP6 phosphorylation and beta-catenin signaling. J Biol Chem 282: 15903-11.
Yamanaka et al., 2002, EMBO Rep.3: 69-75.
Yamamoto, H., H. Komekado, and A. Kikuchi. 2006. Caveolin Is Necessary for Wnt-3a-Dependent Internalization of LRP6 and Accumulation of beta-Catenin. Dev Cell 11: 213-23.
Yu, A., J.F. Rual, K. Tamai, Y. Harada, M. Vidal, X. He, and T. Kirchhausen. 2007. Association of Dishevelled with the clathrin AP-2 adaptor is required for Frizzled endocytosis and planar cell polarity signaling. Dev Cell 12: 129-41.
Zhao, J., K.A. Kim, J. De Vera, S. Palencia, M. Wagle, and A. Abo. 2009. R-Spondin1 protects mice from chemotherapy or radiation-induced oral mucositis through the canonical Wnt/beta-catenin pathway. Proc Natl Acad Sci U S A 106: 2331-6.
Zhou, W., L. Lin, A. Majumdar, X. Li, X. Zhang, W. Liu, L. Etheridge, Y. Shi, J. Martin, W. Van de Ven, V. Kaartinen, A. Wynshaw-Boris, A.P. McMahon, M.G. Rosenfeld, and S.M. Evans. 2007. Modulation of morphogenesis by noncanonical Wnt signaling requires ATF/CREB family-mediated transcriptional activation of TGFbeta2. Nat Genet 39: 1225-34.

The present invention is also described by the following items:
1. A method of identifying a modulator of Rspondin-2 (Rspo2), Rspondin-3 (Rspo3) and/or Syndecan (Sdc), i.e. Syndecan-1 (Sdc1), Syndecan-2 (Sdc2), Syndecan-3 (Sdc3) and/or Syndecan-4 (Sdc4) activity,
   comprising evaluating and/or screening, if a test compound has the ability to modulate binding of an Rspo2 polypeptide or an Rspo3 polypeptide to an Sdc1, Sdc2, Sdc3 and/or Sdc4 polypeptide compared to a control.
2. The method of item 1, comprising evaluating and/or screening, if a test compound has the ability to stimulate binding.
3. The method of item 1, comprising evaluating and/or screening, if a test compound has the ability to inhibit binding.
4. The method of any one of items 1-3, wherein the binding is determined in a cell-free system.
5. The method of any one of items 1-3, wherein the binding is determined in a cellular system, e.g. a recombinant cell or non-human transgenic organism.
6. The method of item 5, wherein the binding is determined in a cell or organism which overexpresses Rspo2 or Rspo3 and/or an Sdc, selected from Sdc1, Sdc2, Sdc3, Sdc4 and combinations thereof.
7. The method of any one of items 1-6 for identifying and/or evaluating candidate agents for the treatment of an Rspo2, Rspo3 and/or Sdc associated disorder.
8. The method of any one of items 1-7 for identifying and/or evaluating candidate agents for the treatment of a proliferation associated disorder.
9. The method of item 7 or 8, wherein the disorder is selected from cancer, an inflammatory disorder, a bone associated disorder, or wound healing.
10. An antagonist of a Rspondin-2 (Rspo2) or Rspondin-3 (Rspo3) polypeptide for use as a medicament, wherein said antagonist inhibits or blocks the interaction of a Rspondin-2 (Rspo2) or Rspondin-3 (Rspo3) polypeptide with a Syndecan (Sdc) polypeptide.
11. The antagonist of item 10, wherein said Syndecan (Sdc) polypeptide is Syndecan-4 (Sdc4).
12. The antagonist of item 10 or 11, wherein said antagonist is an antibody, preferably a monoclonal antibody.
13. The antagonist of any one of items 10-12, wherein said antibody is an antibody against Rspondin-2 (Rspo2).
14. The antagonist of any one of items 10-12, wherein said antibody is an antibody against Rspondin-3 (Rspo3).
15. The antagonist of any one of items 10-14, wherein said antagonist is used in the treatment of cancer, an inflammatory disorder, a bone associated disorder, or wound healing
16. An antagonist of a Syndecan (Sdc) polypeptide for use as a medicament, wherein said antagonist inhibits or blocks the interaction of a Syndecan (Sdc) polypeptide with a Rspondin-2 (Rspo2) or a Rspondin-3 (Rspo3) polypeptide.
17. The antagonist of item 16, wherein said antagonist blocks the interaction of a Syndecan (Sdc) polypeptide with a Rspondin-3 (Rspo3) polypeptide.
18. The antagonist of item 16 or 17, wherein said antagonist is an antibody, preferably a monoclonal antibody.
19. The antagonist of any one of items 16-18, wherein said antagonist is used in the treatment of cancer, an inflammatory disorder, a bone associated disorder, or wound healing
20. An antagonist of a Rspondin-2 (Rspo2) or Rspondin-3 (Rspo3) polypeptide or a Rspondin-2 (Rspo) or Rspondin-3 (Rspo3) nucleic acid for use in the treatment of disorders caused by, associated with and/or accompanied by Syndecan (Sdc), i.e. Sdc1, Sdc2, Sdc3 and/or Sdc4 hyperactivity.
21. The antagonist for the use of item 20, which is (i) an anti-Rspo2 antibody, (ii) an anti-Rspo3 antibody, (iii) an Sdc, i.e. Sdc1, Sdc2, Sdc3 or Sdc4 fragment or (iv) a nucleic acid molecule capable of inhibiting Rspo2 and/or Rspo3 expression.
22. An antagonist for the use of item 20 or 21, wherein the treatment comprises determination of Sdc activity in a subject to be treated.
23. The antagonist for the use of any one of items 20-22 for the treatment of a proliferative disorder selected from cancer, an inflammatory disorder, a bone associated disorder, or wound healing, wherein the disorder is characterized by an increased amount and/or activity of an Sdc selected from Sdc1, Sdc2, Sdc3, Sdc4 and combinations thereof.
24. An antagonist of a Syndecan (Sdc) polypeptide or a Syndecan (Sdc) nucleic acid for for use in the treatment of disorders caused by, associated with and/or accompanied by Rspondin-2 (Rspo2) and/or Rspondin-3 (Rspo3) hyperactivity, wherein the Sdc is selected from Sdc1, Sdc2, Sdc3, Sdc4 and combinations thereof.
25. The antagonist for use in item 24, which is (i) an anti-Sdc antibody, i.e. an anti-Sdc1 antibody, an anti-Sdc2 antibody, an anti-Sdc3 antibody or an anti-Sdc4 antibody, (ii) an Rspo2 fragment, (iii) an Rspo3 fragment or (iv) a nucleic acid molecule capable of inhibiting Sdc expression, wherein the Sdc is selected from Sdc1, Sdc2, Sdc3, Sdc4 or combinations thereof.
26. The antagonist for use in item 24 or 25, wherein the treatment comprises determination of Rspo2 and/or a Rspo3 activity in a subject to be treated.
27. The antagonist for the use of any one of items 24-26 for the treatment of a proliferative disorder selected from cancer, an inflammatory disorder, a bone associated disorder, or wound healing, wherein the disorder is characterized by an increased amount and/or activiy of Rspo2 and/or Rspo3.
28. The antagonist of any one of items 10-27 for use in human or veterinary medicine.

## Claims

1. An antagonist of a Rspondin-2 (Rspo2) or Rspondin-3 (Rspo3) polypeptide for use as a medicament, wherein said antagonist is an antibody which inhibits or blocks the interaction of a Rspondin-2 (Rspo2) or Rspondin-3 (Rspo3) polypeptide with a Syndecan (Sdc) polypeptide, preferably Syndecan-4 (Sdc4).

2. The antagonist of claim 1, wherein said antagonist is a monoclonal antibody.

3. The antagonist of any one of claims 1 or 2, wherein said antagonist is used in the treatment of cancer, an inflammatory disorder, a bone associated disorder, or wound healing.

4. An antagonist of a Syndecan (Sdc) polypeptide for use as a medicament, wherein said antagonist is an antibody which inhibits or blocks the interaction of a Syndecan (Sdc) polypeptide with a Rspondin-2 (Rspo2) or a Rspondin-3 (Rspo3) polypeptide.

5. The antagonist of claim 4, wherein said antagonist is used in the treatment of cancer, an inflammatory disorder, a bone associated disorder, or wound healing.

6. An antagonist of a Rspondin-2 (Rspo2) or Rspondin-3 (Rspo3) polypeptide, which is an anti-Rspo2 antibody, an anti-Rspo3 antibody or a Syndecan fragment, for use in the treatment of disorders caused by, associated with and/or accompanied by Syndecan (Sdc), i.e. Sdc1, Sdc2, Sdc3 and/or Sdc4 hyperactivity.

7. An antagonist of a Rspondin-2 (Rspo2) or Rspondin-3 (Rspo3) nucleic acid, which is a nucleic acid molecule capable of inhibiting Rspo2 and/or Rspo3 expression, for use in the treatment of disorders caused by, associated with and/or accompanied by Syndecan (Sdc), i.e. Sdc1, Sdc2, Sdc3 and/or Sdc4 hyperactivity.

8. The antagonist for the use of any one of claims 1 to 7 for the treatment of a proliferative disorder selected from cancer, an inflammatory disorder, a bone associated disorder, or wound healing, wherein the disorder is **characterized by** an increased amount and/or activity of an Sdc selected from Sdc1, Sdc2, Sdc3, Sdc4 and combinations thereof.

9. An antagonist of (a) a Rspondin-2 (Rspo2) or Rspondin-3 (Rspo3) polypeptide, or (b) a Syndecan (Sdc) polypeptide, wherein said antagonist is an antibody which inhibits or blocks the interaction of a Rspondin-2 (Rspo2) or Rspondin-3 (Rspo3) polypeptide with a Syndecan (Sdc) polypeptide, preferably Syndecan-4 (Sdc4).


SEQUENCE LISTING

<110>  Deutsches Krebsforschungszentrum

<120>  Syndecans as Receptors of Rspo2 and Rspo3

<130>  46558P EP-WO-1

<160>  13

<170>  PatentIn version 3.3

<210>  1
<211>  272
<212>  PRT
<213>  Homo sapiens


<220>
<221>  misc_feature
<222>  (1)..(272)
<223>  human Rspondin-3

<400>  1

Met His Leu Arg Leu Ile Ser Trp Leu Phe Ile Ile Leu Asn Phe Met
1               5                   10                  15


Glu Tyr Ile Gly Ser Gln Asn Ala Ser Arg Gly Arg Arg Gln Arg Arg
            20                  25                  30


Met His Pro Asn Val Ser Gln Gly Cys Gln Gly Gly Cys Ala Thr Cys
        35                  40                  45


Ser Asp Tyr Asn Gly Cys Leu Ser Cys Lys Pro Arg Leu Phe Phe Ala
    50                  55                  60


Leu Glu Arg Ile Gly Met Lys Gln Ile Gly Val Cys Leu Ser Ser Cys
65                  70                  75                  80


Pro Ser Gly Tyr Tyr Gly Thr Arg Tyr Pro Asp Ile Asn Lys Cys Thr
                85                  90                  95


Lys Cys Lys Ala Asp Cys Asp Thr Cys Phe Asn Lys Asn Phe Cys Thr
            100                 105                 110


Lys Cys Lys Ser Gly Phe Tyr Leu His Leu Gly Lys Cys Leu Asp Asn
        115                 120                 125


Cys Pro Glu Gly Leu Glu Ala Asn Asn His Thr Met Glu Cys Val Ser
    130                 135                 140


Ile Val His Cys Glu Val Ser Glu Trp Asn Pro Trp Ser Pro Cys Thr
145                 150                 155                 160

Lys Lys Gly Lys Thr Cys Gly Phe Lys Arg Gly Thr Glu Thr Arg Val
                165                 170                 175

Arg Glu Ile Ile Gln His Pro Ser Ala Lys Gly Asn Leu Cys Pro Pro
            180                 185                 190

Thr Asn Glu Thr Arg Lys Cys Thr Val Gln Arg Lys Lys Cys Gln Lys
        195                 200                 205

Gly Glu Arg Gly Lys Lys Gly Arg Glu Arg Lys Arg Lys Lys Pro Asn
    210                 215                 220

Lys Gly Glu Ser Lys Glu Ala Ile Pro Asp Ser Lys Ser Leu Glu Ser
225                 230                 235                 240

Ser Lys Glu Ile Pro Glu Gln Arg Glu Asn Lys Gln Gln Gln Lys Lys
                245                 250                 255

Arg Lys Val Gln Asp Lys Gln Lys Ser Val Ser Val Ser Thr Val His
            260                 265                 270

<210>  2
<211>  2089
<212>  DNA
<213>  Homo sapiens

<220>
<221>  misc_feature
<222>  (1)..(2089)
<223>  human Rspondin-3

<400>  2
ggagcgtctc ctgctcgcgc acgccagaag cagctcgggg tctctccgcc gccgccgccg      60

ctcgcccgcc cggatcccgc ctgcggcagt tgccgcacaa catgctacct gcggccgccc     120

cggcggctcc tggaaccccg gttcgcggcg atgccagcca ccccagcgaa gccgccgcag     180

ttcagtgctt ggataatttg aaagtacaat agttggtttc cctgtccacc cgccccactt     240

cgcttgccat cacagcacgc ctatcggatg tgagaggaga agtcccgctg ctcgggcact     300

gtctatatac gcctaacacc tacatatatt ttaaaaacat taaatataat taacaatcaa     360

aagaaagagg agaaaggaag ggaagcatta ctgggttact atgcacttgc gactgatttc     420

ttggcttttt atcattttga actttatgga atacatcggc agccaaaacg cctcccgggg     480

aaggcgccag cgaagaatgc atcctaacgt tagtcaaggc tgccaaggag gctgtgcaac     540

atgctcagat tacaatggat gtttgtcatg taagcccaga ctattttttg ctctggaaag      600

aattggcatg aagcagattg gagtatgtct ctcttcatgt ccaagtggat attatggaac     660

tcgatatcca gatataaata agtgtacaaa atgcaaagct gactgtgata cctgtttcaa 720

caaaaatttc tgcacaaaat gtaaaagtgg attttactta caccttggaa agtgccttga 780

caattgccca gaagggttgg aagccaacaa ccatactatg gagtgtgtca gtattgtgca 840

ctgtgaggtc agtgaatgga atccttggag tccatgcacg aagaagggaa aaacatgtgg 900

cttcaaaaga gggactgaaa cacgggtccg agaaataata cagcatcctt cagcaaaggg 960

taacctgtgt cccccaacaa atgagacaag aaagtgtaca gtgcaaagga agaagtgtca 1020

gaagggagaa cgaggaaaaa aaggaaggga gaggaaaaga aaaaaaccta ataaaggaga 1080

aagtaaagaa gcaatacctg acagcaaaag tctggaatcc agcaaagaaa tcccagagca 1140

acgagaaaac aaacagcagc agaagaagcg aaaagtccaa gataaacaga aatcggtatc 1200

agtcagcact gtacactaga gggttccatg agattattgt agactcatga tgctgctatc 1260

tcaaccagat gcccaggaca ggtgctctag ccattaggac cacaaatgga catgtcagtt 1320

attgctctgt ctaaacaaca ttcccagtag ttgctatatt cttcatacaa gcatagttaa 1380

caacaaagag ccaaaagatc aaagaaggga tactttcaga tggttgtctt gtgtgcttct 1440

ctgcattttt aaaagacaag acattcttgt acatattatc aataggctat aagatgtaac 1500

aacgaaatga tgacatctgg agaagaaaca tcttttcctt ataaaaatgt gttttcaagc 1560

tgttgtttta agaagcaaaa gatagttctg caaattcaaa gatacagtat cccttcaaaa 1620

caaataggag ttcagggaag agaaacatcc ttcaaaggac agtgttgttt tgaccgggag 1680

atctagagag tgctcagaat tagggcctgg catttggaat cacaggattt atcatcacag 1740

aaacaactgt tttaagatta gttccatcac tctcatcctg tatttttata agaaacacaa 1800

gagtgcatac cagaattgaa tataccatat gggattggag aaagacaaat gtggaagaaa 1860

tcatagagct ggagactact tttgtgcttt acaaaactgt gaaggattgt ggtcacctgg 1920

aacaggtctc caatctatgt tagcactatg tggctcagcc tctgttaccc cttggattat 1980

atatcaacct gtaaacatgt gcctgtaact tacttccaaa aacaaaatca tacttattag 2040

aagaaaattc tgattttata aaaaaaaaaa aaaaaaaaaa aaaaaaaaa 2089

<210> 3
<211> 243
<212> PRT
<213> Homo sapiens

<220>
<221> misc_feature
<222> (1)..(243)
<223> human Rspondin-2

<400> 3

Met Gln Phe Arg Leu Phe Ser Phe Ala Leu Ile Ile Leu Asn Cys Met
1               5                   10                  15

Asp Tyr Ser His Cys Gln Gly Asn Arg Trp Arg Arg Ser Lys Arg Ala
            20                  25                  30

Ser Tyr Val Ser Asn Pro Ile Cys Lys Gly Cys Leu Ser Cys Ser Lys
        35                  40                  45

Asp Asn Gly Cys Ser Arg Cys Gln Gln Lys Leu Phe Phe Phe Leu Arg
    50                  55                  60

Arg Glu Gly Met Arg Gln Tyr Gly Glu Cys Leu His Ser Cys Pro Ser
65                  70                  75                  80

Gly Tyr Tyr Gly His Arg Ala Pro Asp Met Asn Arg Cys Ala Arg Cys
                85                  90                  95

Arg Ile Glu Asn Cys Asp Ser Cys Phe Ser Lys Asp Phe Cys Thr Lys
            100                 105                 110

Cys Lys Val Gly Phe Tyr Leu His Arg Gly Arg Cys Phe Asp Glu Cys
        115                 120                 125

Pro Asp Gly Phe Ala Pro Leu Glu Glu Thr Met Glu Cys Val Glu Gly
    130                 135                 140

Cys Glu Val Gly His Trp Ser Glu Trp Gly Thr Cys Ser Arg Asn Asn
145                 150                 155                 160

Arg Thr Cys Gly Phe Lys Trp Gly Leu Glu Thr Arg Thr Arg Gln Ile
                165                 170                 175

Val Lys Lys Pro Val Lys Asp Thr Ile Leu Cys Pro Thr Ile Ala Glu
            180                 185                 190

Ser Arg Arg Cys Lys Met Thr Met Arg His Cys Pro Gly Gly Lys Arg
        195                 200                 205

Thr Pro Lys Ala Lys Glu Lys Arg Asn Lys Lys Lys Lys Arg Lys Leu
    210                 215                 220

Ile Glu Arg Ala Gln Glu Gln His Ser Val Phe Leu Ala Thr Asp Arg
225                 230                 235                 240

Ala Asn Gln

<210> 4
<211> 3149
<212> DNA
<213> Homo sapiens

<220>
<221> misc_feature
<222> (1)..(3149)
<223> human Rspondin-2

<400> 4
   agcctagact tagatgcctt ggaccacagc accacctact tatagaagca tcccaagcct 60
   cagccggtct gcatctccat cggaaagtgc gcttgccaca tcccttcgga tcacttcgtc 120
   ctcccgagag cgttctgcct tctacagctc ggaaagaaag aaatcttagc tgtgaagtga 180
   ccgtggagaa agcgcaggaa gcgacacaat tggttaggga ggcagagagt gtgagcgggc 240
   gcaccccttg cctggggacc gcgctcgcgg gcggggacgg agcatcccag tggctgcacc 300
   cgccgctccg cgctcctgcc tggcgtcgcc aaccccgcgg cggccgctgg aattccagag 360
   ctgccaggcg ctcccagccg gtctcggcaa acttttcccc agcccacgtg ctaaccaagc 420
   ggctcgcttc ccgagcccgg gatggagcac cgcgcctagg gaggccgcgc cgcccgagac 480
   gtgcgcacgg ttcgtggcgg agagatgctg atcgcgctga actgaccggt gcggcccggg 540
   ggtgagtggc gagtctccct ctgagtcctc cccagcagcg cggccggcgc cggctctttg 600
   ggcgaaccct ccagttccta gactttgaga ggcgtctctc ccccgcccga ccgcccagat 660
   gcagtttcgc cttttctcct ttgccctcat cattctgaac tgcatggatt acagccactg 720
   ccaaggcaac cgatggagac gcagtaagcg agctagttat gtatcaaatc ccatttgcaa 780
   gggttgtttg tcttgttcaa aggacaatgg gtgtagccga tgtcaacaga agttgttctt 840
   cttccttcga agagaaggga tgcgccagta tggagagtgc ctgcattcct gcccatccgg 900
   gtactatgga caccgagccc cagatatgaa cagatgtgca agatgcagaa tagaaaactg 960
   tgattcttgc tttagcaaag acttttgtac caagtgcaaa gtaggctttt atttgcatag 1020
   aggccgttgc tttgatgaat gtccagatgg ttttgcacca ttagaagaaa ccatggaatg 1080
   tgtggaagga tgtgaagttg gtcattggag cgaatgggga acttgtagca gaaataatcg 1140
   cacatgtgga tttaaatggg gtctggaaac cagaacacgg caaattgtta aaaagccagt 1200
   gaaagacaca atactgtgtc caaccattgc tgaatccagg agatgcaaga tgacaatgag 1260
   gcattgtcca ggagggaaga gaacaccaaa ggcgaaggag aagaggaaca agaaaaagaa 1320
   aaggaagctg atagaaaggg cccaggagca acacagcgtc ttcctagcta cagacagagc 1380
   taaccaataa aacaagagat ccggtagatt tttaggggtt tttgtttttg caaatgtgca 1440
   caaagctact ctccactcct gcacactggt gtgcagcctt tgtgctgctc tgcccagtat 1500
   ctgttcccag taacatggtg aaaggaagca ccaccagcat ggcccctgtg ttatttatgc 1560
   tttgatttga atctggagac tgtgaaggca ggagtaagtg cacagcccgt gacttggctc 1620
   agtgtgtgct gagagaatcc gtccccggca ccatggacat gctagaggtg tgaggctgca 1680
   gaacaccgct ggaggacgga cttgtgccta tttatgtgaa agaagatgct tggcaggcaa 1740
   tgcgctactc actcgtgacc tttatttctc acattgtgca ttttcaagga tatgtttgtg 1800
   tggatatctg cttagtgtta ccacatggta ttctcagcat gttaccttca cactgttgtg 1860
   cgatgaaact gcttttagct gaggatatgc tctggaaatt cctgctcagt ttcactgcag 1920
   ccctaatatg tacatatact gcaggagcta catataaagc tcttatttac tgtatattta 1980
   tgctttcttg tgggtaacaa gtcatacctg attaatatga tgccactttg tttctagtgg 2040
   ttcctaaccc attgtctgat aaatgacttt tctagtttgg ggaattgaca cttgttttgt 2100
   tgcctcttga aacttttttt ttttcccctc attgtgggct tatttctcat tgtaagggta 2160
   ggataaacta gtttttgtat atagagtcaa atgaccagtg tcaaagagtt tgcatattgg 2220
   gtagaccttc tccactccac atgtcccaca catatagata aagcagcagg cggcatctgg 2280
   caatcagaag cccaaactgc ctttgagtct aagatgtgat gactttgatg aaacacaact 2340
   gaaaacatga gggactatat ccagtcactt gtagccagtt tcacaggcca gctacagaat 2400
   tgtccaaaca aacattattt ctgactgcaa tttttttccc ccaaatttaa agcaatccct 2460
   ggctttaaat gacaaggcac ctaccaatgt tcttgggtca ctgaagaagc tactaccatg 2520
   agcctgggca tagaatttta ggagataaaa ggatgaattt ctgtgactgc cagtcagatc 2580
   ttaacaggtt tctgttgagc cagaatctgt ttcagatcca agatggagag gaacactatg 2640
   gaaacttccc aggtgacttt cagagcagtt gtttcaaaca catcattgtc cttttagggg 2700
   aaccagtttt tagaaggttg tgaattggct ttttcacaaa gcatgattat cttcctggct 2760
   gatccaggag aaaattagaa cagaaaaata atggttgtgg attttgaaac aaagcaaggt 2820
   aaagcctttt ttttttcacc ttgcattggc aaaactacct cttcagtgtt tttaactttt 2880
   gattcaaaag catcttacca ataaggataa atatcatata catcgttatg aaaatattgc 2940
   tatgagataa taagccacat atgaatgttg tatacaactt tagggtttac atttaatcct 3000
   gaagtgttac ctcctttcat gtctatttac actattttcc catttactaa gtggggaggg 3060
   ggtctcctta tatagtgctt catcgttaat aagtcaatac ctgttgttcc tgggatgttc 3120
   ttttttgtgc attaaaaact tcaaaatta 3149

<210> 5
<211> 310
<212> PRT
<213> Homo sapiens

<220>
<221>  misc_feature
<222>  (1)..(310)
<223>  human Syndecan-1

<400>  5

Met Arg Arg Ala Ala Leu Trp Leu Trp Leu Cys Ala Leu Ala Leu Ser
1               5                   10                  15

Leu Gln Pro Ala Leu Pro Gln Ile Val Ala Thr Asn Leu Pro Pro Glu
            20                  25                  30

Asp Gln Asp Gly Ser Gly Asp Asp Ser Asp Asn Phe Ser Gly Ser Gly
        35                  40                  45

Ala Gly Ala Leu Gln Asp Ile Thr Leu Ser Gln Gln Thr Pro Ser Thr
    50                  55                  60

Trp Lys Asp Thr Gln Leu Leu Thr Ala Ile Pro Thr Ser Pro Glu Pro
65                  70                  75                  80

Thr Gly Leu Glu Ala Thr Ala Ala Ser Thr Ser Thr Leu Pro Ala Gly
                85                  90                  95

Glu Gly Pro Lys Glu Gly Glu Ala Val Val Leu Pro Glu Val Glu Pro
            100                 105                 110

Gly Leu Thr Ala Arg Glu Gln Glu Ala Thr Pro Arg Pro Arg Glu Thr
        115                 120                 125

Thr Gln Leu Pro Thr Thr His Gln Ala Ser Thr Thr Thr Ala Thr Thr
    130                 135                 140

Ala Gln Glu Pro Ala Thr Ser His Pro His Arg Asp Met Gln Pro Gly
145                 150                 155                 160

His His Glu Thr Ser Thr Pro Ala Gly Pro Ser Gln Ala Asp Leu His
                165                 170                 175

Thr Pro His Thr Glu Asp Gly Gly Pro Ser Ala Thr Glu Arg Ala Ala
            180                 185                 190

Glu Asp Gly Ala Ser Ser Gln Leu Pro Ala Ala Glu Gly Ser Gly Glu
        195                 200                 205

Gln Asp Phe Thr Phe Glu Thr Ser Gly Glu Asn Thr Ala Val Val Ala
    210                 215                 220

Val Glu Pro Asp Arg Arg Asn Gln Ser Pro Val Asp Gln Gly Ala Thr
225                 230                 235                 240

Gly Ala Ser Gln Gly Leu Leu Asp Arg Lys Glu Val Leu Gly Gly Val
                245                 250                 255

Ile Ala Gly Gly Leu Val Gly Leu Ile Phe Ala Val Cys Leu Val Gly
            260                 265                 270

Phe Met Leu Tyr Arg Met Lys Lys Lys Asp Glu Gly Ser Tyr Ser Leu
        275                 280                 285

Glu Glu Pro Lys Gln Ala Asn Gly Gly Ala Tyr Gln Lys Pro Thr Lys
    290                 295                 300

Gln Glu Glu Phe Tyr Ala
305                 310

<210>  6
<211>  3217
<212>  DNA
<213>  Homo sapiens

<220>
<221>  misc_feature
<222>  (1)..(3217)
<223>  human Syndecan-1

<400>  6
ggccgggaga cctggcggag ctgggggtgg ggggccagtt tttgcaacgg ctaaggaagg      60

gcctgtgggt ttattataag gcggagctcg gcgggagagg tgcgggccga atccgagccg     120

agcggagagg aatccggcag tagagagcgg actccagccg gcggaccctg cagccctcgc     180

ctgggacagc ggcgcgctgg gcaggcgccc aagagagcat cgagcagcgg aacccgcgaa     240

gccggcccgc agccgcgacc cgcgcagcct gccgctctcc cgccgccggt ccgggcagca     300

tgaggcgcgc ggcgctctgg ctctggctgt gcgcgctggc gctgagcctg cagccggccc     360

tgccgcaaat tgtggctact aatttgcccc ctgaagatca agatggctct ggggatgact     420

ctgacaactt ctccggctca ggtgcaggtg ctttgcaaga tatcaccttg tcacagcaga     480

ccccctccac ttggaaggac acgcagctcc tgacggctat tcccacgtct ccagaaccca     540

ccggcctgga ggctacagct gcctccacct ccaccctgcc ggctggagag gggcccaagg     600

agggagaggc tgtagtcctg ccagaagtgg agcctggcct caccgcccgg gagcaggagg     660

ccaccccccg acccagggag accacacagc tcccgaccac tcatcaggcc tcaacgacca     720

cagccaccac ggcccaggag cccgccacct cccaccccca cagggacatg cagcctggcc     780

accatgagac ctcaacccct gcaggaccca gccaagctga ccttcacact ccccacacag     840

aggatggagg tccttctgcc accgagaggg ctgctgagga tggagcctcc agtcagctcc 900
cagcagcaga gggctctggg gagcaggact tcacctttga aacctcgggg gagaatacgg 960
ctgtagtggc cgtggagcct gaccgccgga accagtcccc agtggatcag ggggccacgg 1020
gggcctcaca gggcctcctg gacaggaaag aggtgctggg aggggtcatt gccggaggcc 1080
tcgtggggct catctttgct gtgtgcctgg tgggtttcat gctgtaccgc atgaagaaga 1140
aggacgaagg cagctactcc ttggaggagc cgaaacaagc caacggcggg gcctaccaga 1200
agcccaccaa acaggaggaa ttctatgcct gacgcgggag ccatgcgccc cctccgccct 1260
gccactcact aggcccccac ttgcctcttc cttgaagaac tgcaggccct ggcctcccct 1320
gccaccaggc cacctcccca gcattccagc ccctctggtc gctcctgccc acggagtcgt 1380
ggggtgtgct gggagctcca ctctgcttct ctgacttctg cctggagact tagggcacca 1440
ggggtttctc gcataggacc tttccaccac agccagcacc tggcatcgca ccattctgac 1500
tcggtttctc caaactgaag cagcctctcc ccaggtccag ctctggaggg gagggggatc 1560
cgactgcttt ggacctaaat ggcctcatgt ggctggaaga tcctgcgggt ggggcttggg 1620
gctcacacac ctgtagcact tactggtagg accaagcatc ttgggggggt ggccgctgag 1680
tggcagggga caggagtcca ctttgtttcg tggggaggtc taatctagat atcgacttgt 1740
ttttgcacat gtttcctcta gttctttgtt catagcccag tagaccttgt tacttctgag 1800
gtaagttaag taagttgatt cggtatcccc ccatcttgct tccctaatct atggtcggga 1860
gacagcatca gggttaagaa gacttttttt tttttttttt aaactaggag aaccaaatct 1920
ggaagccaaa atgtaggctt agtttgtgtg ttgtctcttg agtttgtcgc tcatgtgtgc 1980
aacagggtat ggactatctg tctggtggcc ccgtttctgg tggtctgttg gcaggctggc 2040
cagtccaggc tgccgtgggg ccgccgcctc tttcaagcag tcgtgcctgt gtccatgcgc 2100
tcagggccat gctgaggcct gggccgctgc cacgttggag aagcccgtgt gagaagtgaa 2160
tgctgggact cagccttcag acagagagga ctgtagggag ggcggcaggg gcctggagat 2220
cctcctgcag accacgcccg tcctgcctgt ggcgccgtct ccaggggctg cttcctcctg 2280
gaaattgacg aggggtgtct tgggcagagc tggctctgag cgcctccatc caaggccagg 2340
ttctccgtta gctcctgtgg ccccaccctg ggccctgggc tggaatcagg aatattttcc 2400
aaagagtgat agtcttttgc ttttggcaaa actctactta atccaatggg tttttccctg 2460
tacagtagat tttccaaatg taataaactt taatataaag tagtcctgtg aatgccactg 2520
ccttcgcttc ttgcctctgt gctgtgtgtg acgtgaccgg acttttctgc aaacaccaac 2580
atgttgggaa acttggctcg aatctctgtg ccttcgtctt tcccatgggg agggattctg 2640
gttccagggt ccctctgtgt atttgctttt ttgttttggc tgaaattctc ctggaggtcg 2700

gtaggttcag ccaaggtttt ataaggctga tgtcaatttc tgtgttgcca agctccaagc 2760

cccatcttct aaatggcaaa ggaaggtgga tggccccagc acagcttgac ctgaggctgt 2820

ggtcacagcg gaggtgtgga gccgaggcct accccgcaga caccttggac atcctcctcc 2880

cacccggctg cagaggccag aggcccccag cccagggctc ctgcacttac ttgcttattt 2940

gacaacgttt cagcgactcc gttggccact ccgagaggtg ggccagtctg tggatcagag 3000

atgcaccacc aagccaaggg aacctgtgtc cggtattcga tactgcgact ttctgcctgg 3060

agtgtatgac tgcacatgac tcgggggtgg ggaaaggggt cggctgacca tgctcatctg 3120

ctggtccgtg ggacggtgcc caagccagag gctgggttca tttgtgtaac gacaataaac 3180

ggtacttgtc atttcgggca aaaaaaaaaa aaaaaaa 3217

<210> 7
<211> 201
<212> PRT
<213> Homo sapiens

<220>
<221> misc_feature
<222> (1)..(201)
<223> human Syndecan-2

<400> 7

Met Arg Arg Ala Trp Ile Leu Leu Thr Leu Gly Leu Val Ala Cys Val
1               5                   10                  15

Ser Ala Glu Ser Arg Ala Glu Leu Thr Ser Asp Lys Asp Met Tyr Leu
            20                  25                  30

Asp Asn Ser Ser Ile Glu Glu Ala Ser Gly Val Tyr Pro Ile Asp Asp
        35                  40                  45

Asp Asp Tyr Ala Ser Ala Ser Gly Ser Gly Ala Asp Glu Asp Val Glu
    50                  55                  60

Ser Pro Glu Leu Thr Thr Ser Arg Pro Leu Pro Lys Ile Leu Leu Thr
65                  70                  75                  80

Ser Ala Ala Pro Lys Val Glu Thr Thr Thr Leu Asn Ile Gln Asn Lys
                85                  90                  95

Ile Pro Ala Gln Thr Lys Ser Pro Glu Glu Thr Asp Lys Glu Lys Val
            100                 105                 110

His Leu Ser Asp Ser Glu Arg Lys Met Asp Pro Ala Glu Glu Asp Thr
        115                 120                 125

Asn Val Tyr Thr Glu Lys His Ser Asp Ser Leu Phe Lys Arg Thr Glu
    130                 135                 140

Val Leu Ala Ala Val Ile Ala Gly Gly Val Ile Gly Phe Leu Phe Ala
145                 150                 155                 160

Ile Phe Leu Ile Leu Leu Leu Val Tyr Arg Met Arg Lys Lys Asp Glu
                165                 170                 175

Gly Ser Tyr Asp Leu Gly Glu Arg Lys Pro Ser Ser Ala Ala Tyr Gln
            180                 185                 190

Lys Ala Pro Thr Lys Glu Phe Tyr Ala
        195                 200

<210>  8
<211>  3485
<212>  DNA
<213>  Homo sapiens

<220>
<221>  misc_feature
<222>  (1)..(3485)
<223>  human Syndecan-2

<400>  8
agtcgcccag gggagcccgg agaagcaggc tcaggaggga gggagccaga ggaaaagaag      60

aggaggagaa ggaggaggac ccggggaggg aggcgcggcg cgggaggagg aggggcgcag     120

ccgcggagcc agtggccccg cttggacgcg ctgctctcca gatacccccg gagctccagc     180

cgcgcggatc gcgcgctccc gccgctctgc ccctaaactt ctgccgtagc tccctttcaa     240

gccagcgaat ttattcctta aaaccagaaa ctgaacctcg gcacgggaaa ggagtccgcg     300

gaggagcaaa accacagcag agcaagaaga gcttcagaga gcagccttcc cggagcacca     360

actccgtgtc gggagtgcag aaaccaacaa gtgagagggc gccgcgttcc cggggcgcag     420

ctgcgggcgg cgggagcagg cgcaggagga ggaagcgagc gcccccgagc cccgagcccg     480

agtccccgag cctgagccgc aatcgctgcg gtactctgct ccggattcgt gtgcgcgggc     540

tgcgccgagc gctgggcagg aggcttcgtt ttgccctggt tgcaagcagc ggctgggagc     600

agccggtccc tggggaatat gcggcgcgcg tggatcctgc tcaccttggg cttggtggcc     660

tgcgtgtcgg cggagtcgag agcagagctg acatctgata aagacatgta ccttgacaac     720

agctccattg aagaagcttc aggagtgtat cctattgatg acgatgacta cgcttctgcg     780

tctggctcgg gagctgatga ggatgtagag agtccagagc tgacaacatc tcgaccactt     840

ccaaagatac tgttgactag tgctgctcca aaagtggaaa ccacgacgct gaatatacag     900

aacaagatac ctgctcagac aaagtcacct gaagaaactg ataaagagaa agttcacctc 960
tctgactcag aaaggaaaat ggacccagcc gaagaggata caaatgtgta tactgagaaa 1020
cactcagaca gtctgtttaa acggacagaa gtcctagcag ctgtcattgc tggtggagtt 1080
attggctttc tctttgcaat ttttcttatc ctgctgttgg tgtatcgcat gagaaagaag 1140
gatgaaggaa gctatgacct tggagaacgc aaaccatcca gtgctgctta tcagaaggca 1200
cctactaagg agttttatgc gtaaaactcc aacttagtgt ctctatttat gagatcactg 1260
aacttttcaa aataaagctt ttgcatagaa taatgaagat ctttgttttt tgttttcatt 1320
aaagagccat tctggcactt taatgataaa atcccattgt atttaaaaca tttcatgtat 1380
ttctttagaa caacataaaa ttaaaattta acatctgcag tgttctgtga atagcagtgg 1440
caaaatatta tgttatgaaa accctcgatg ttcatggaat tggtttaaac ttttatgcgc 1500
aaatacaaaa tgattgtctt tttcctatga ctcaaagatg aaagctgttt catttgtgtc 1560
agcatgtctc agattgacct taccaagttg gtcttacttt gttaatttat ctgttgtccc 1620
cttcctctcc tctgccctcc cttcttgtgc ccttaaaacc aaaccctatg ccttttgtag 1680
ctgtcatggt gcaatttgtc tttggaaaat tcagataatg gtaatttagt gtatatgtga 1740
ttttcaaata tgtaaacttt aacttccact ttgtataaat ttttaagtgt cagactatcc 1800
attttacact tgctttattt ttcattacct gtagctttgg gcagatttgc aacagcaaat 1860
taatgtgtaa aattggatta ttactacaaa accgtttagt catatctatc taatcagatc 1920
ttcttttggg aggatttgat gtaagttact gacaagcctc agcaaaccca aagatgttaa 1980
cagtatttta agaagttgct gcagattcct ttggccactg tatttgttaa tttcttgcaa 2040
tttgaaggta cgagtagagg tttaaagaaa aatcagtttt tgttcttaaa aatgcattta 2100
agttgtaaac gtctttttaa gcctttgaag tgcctctgat tctatgtaac ttgttgcaga 2160
ctggtgttaa tgagtatatg taacagttta aaaaaaaagt tggtatttta taagcacaga 2220
caattctaat ggtaactttt gtagtcttat gaatagacat aaattgtaat ttgggaacat 2280
aaaaactact gaataaatca tgtggcctaa tattgaaaat gtcactgtta taaattttgt 2340
acatttttga tcaaatgtac atctcccctt tgctaacggc cgtctgctct caaggatgac 2400
gtgggtttga tttctaagtg tttcacagtg tctgtaaatc aagaccaaag agcctgtcga 2460
tgagactgtt tattaccaga ttcacttctg aattggccag aggaaatctg aatgtattat 2520
cctgtgtgtg tctaggtaga gatattggaa ggctgccagg ggatttcgaa gtttgcaacc 2580
tttataggat aactgatggc aatattaaga cagacgcctg cttttgcaaa taacttacaa 2640
gactgtaaat tccaaagatc tgaatggggc tttcctgatg ttggtatcta aggcttaggc 2700
ctatagattg atttaccttt ggaattgtgc tccaaatgtc tactgaagct taaccgaaga 2760
actaataaat ggactacagt agctcacgtt acagggaagg agggtaggca gggaggctct 2820

gtgtgttaaa atgagggtct cactgcttta ggattgaagt ggctggaaag agtgatgcct 2880

ggggaaggag atggagttat gagggtactg tggctggtac tttctgtact aaacatttcc 2940

tttttctatt ttaccactaa ttttgtttta aactgtgagc cgtccaagtc agaagaagac 3000

agcaaaaaaa gcaacttttc caacatacaa tttactttta ataaagtatg aatatttcat 3060

tttgagaaca ttccctggaa ttgccacata attcattaaa aacatttttt taagcaacac 3120

ttggaacagt gtttacttta aatccttaat ggccttaatt aattctcaga ttcctgcccc 3180

atcacttaca gaaccaattc actttagagt gactaaaagg aaacgatagc ctagctttct 3240

aaagccacgc tgtgtccctc aattacagag ggtaggaatg ggtatacctc taactgtgca 3300

aagcagagtg aaattcaatt catagaataa caactgctgg gaatatccgt gccaggaaaa 3360

gaaaaatttc tggcaaatat tttgtcactg ctgtaaagca aaatatttgt gaaagtgcca 3420

aaataaagtc tgtcatgcca aaagtaaatc attgtataga ctgacatcca gttttcttca 3480

actgt 3485

<210> 9
<211> 442
<212> PRT
<213> Homo sapiens

<220>
<221> misc_feature
<222> (1)..(442)
<223> human Syndecan-3

<400> 9

Met Lys Pro Gly Pro Pro His Arg Ala Gly Ala Ala His Gly Ala Gly
1               5                   10                  15

Ala Gly Ala Gly Ala Ala Ala Gly Pro Gly Ala Arg Gly Leu Leu Leu
            20                  25                  30

Pro Pro Leu Leu Leu Leu Leu Leu Ala Gly Arg Ala Ala Gly Ala Gln
        35                  40                  45

Arg Trp Arg Ser Glu Asn Phe Glu Arg Pro Val Asp Leu Glu Gly Ser
    50                  55                  60

Gly Asp Asp Asp Ser Phe Pro Asp Asp Glu Leu Asp Asp Leu Tyr Ser
65                  70                  75                  80

Gly Ser Gly Ser Gly Tyr Phe Glu Gln Glu Ser Gly Ile Glu Thr Ala
                85                  90                  95

Met Arg Phe Ser Pro Asp Val Ala Leu Ala Val Ser Thr Thr Pro Ala
            100                 105                 110

Val Leu Pro Thr Thr Asn Ile Gln Pro Val Gly Thr Pro Phe Glu Glu
        115                 120                 125

Leu Pro Ser Glu Arg Pro Thr Leu Glu Pro Ala Thr Ser Pro Leu Val
    130                 135                 140

Val Thr Glu Val Pro Glu Glu Pro Ser Gln Arg Ala Thr Thr Val Ser
145                 150                 155                 160

Thr Thr Met Ala Thr Thr Ala Ala Thr Ser Thr Gly Asp Pro Thr Val
                165                 170                 175

Ala Thr Val Pro Ala Thr Val Ala Thr Ala Thr Pro Ser Thr Pro Ala
            180                 185                 190

Ala Pro Pro Phe Thr Ala Thr Thr Ala Val Ile Arg Thr Thr Gly Val
        195                 200                 205

Arg Arg Leu Leu Pro Leu Pro Leu Thr Thr Val Ala Thr Ala Arg Ala
    210                 215                 220

Thr Thr Pro Glu Ala Pro Ser Pro Pro Thr Thr Ala Ala Val Leu Asp
225                 230                 235                 240

Thr Glu Ala Pro Thr Pro Arg Leu Val Ser Thr Ala Thr Ser Arg Pro
                245                 250                 255

Arg Ala Leu Pro Arg Pro Ala Thr Thr Gln Glu Pro Asp Ile Pro Glu
            260                 265                 270

Arg Ser Thr Leu Pro Leu Gly Thr Thr Ala Pro Gly Pro Thr Glu Val
        275                 280                 285

Ala Gln Thr Pro Thr Pro Glu Thr Phe Leu Thr Thr Ile Arg Asp Glu
    290                 295                 300

Pro Glu Val Pro Val Ser Gly Gly Pro Ser Gly Asp Phe Glu Leu Pro
305                 310                 315                 320

Glu Glu Glu Thr Thr Gln Pro Asp Thr Ala Asn Glu Val Val Ala Val
                325                 330                 335

Gly Gly Ala Ala Ala Lys Ala Ser Ser Pro Pro Gly Thr Leu Pro Lys
            340                 345                 350

Gly Ala Arg Pro Gly Pro Gly Leu Leu Asp Asn Ala Ile Asp Ser Gly
        355                 360                 365

Ser Ser Ala Ala Gln Leu Pro Gln Lys Ser Ile Leu Glu Arg Lys Glu
    370                 375                 380

Val Leu Val Ala Val Ile Val Gly Gly Val Val Gly Ala Leu Phe Ala
385                 390                 395                 400

Ala Phe Leu Val Thr Leu Leu Ile Tyr Arg Met Lys Lys Lys Asp Glu
                405                 410                 415

Gly Ser Tyr Thr Leu Glu Glu Pro Lys Gln Ala Ser Val Thr Tyr Gln
            420                 425                 430

Lys Pro Asp Lys Gln Glu Glu Phe Tyr Ala
        435                 440

<210>  10
<211>  5121
<212>  DNA
<213>  Homo sapiens

<220>
<221>  misc_feature
<222>  (1)..(5121)
<223>  human Syndecan-3

<400>  10
acaaaggcgc ccgcccgccg cccgccgccc gcgcccgcgc cgccgccatg aagccggggc      60

cgccgcaccg tgccggggcc gcccacgggg ccggcgccgg ggccggggcc gcggccgggc     120

ccggggcccg cgggctgctc ctgccaccgc tgctgctgct gctgctggcg gggcgcgccg     180

cgggggccca gcgctggcgc agtgagaact tcgagagacc cgtggacctg gagggctctg     240

gggatgatga ctcctttccc gatgatgaac tggatgacct ctactcgggg tcgggctcgg     300

gctacttcga gcaggagtcg ggcattgaga cagccatgcg cttcagccca gatgtagccc     360

tggcggtgtc caccacacct gcggtgctgc ccaccacgaa catccagcct gtgggcacac     420

catttgaaga gctcccctct gagcgcccca ccctggagcc agccaccagc cccctggtgg     480

tgacagaagt cccggaagag cccagccaga gagccaccac cgtctccact accatggcta     540

ccactgctgc cacaagcaca ggggacccga ctgtggccac agtgcctgcc acagtggcca     600

ccgccacccc cagcacccct gcagcacccc cttttacggc caccactgct gttataagga     660

ccactggcgt acggaggctt ctgcctctcc cactgaccac agtggctacg gcacgggcca     720

ctacccccga ggcgccctcc ccgcccacca cggcggctgt cttggacacc gaggccccaa     780

cacccaggct ggtcagcaca gctacctccc ggccaagagc ccttcccagg ccggccacca 840
cccaggagcc tgacatccct gagaggagca ccctgcccct ggggaccact gcccctggac 900
ccacagaggt ggctcagacc ccaactccag agaccttcct gaccacaatc cgggatgagc 960
cagaggttcc ggtgagtggg gggcccagtg gagacttcga gctgccagaa gaagagacca 1020
cacaaccaga cacagccaat gaggtggtag ctgtgggagg ggctgcggcc aaggcatcat 1080
ctccacctgg gacactgccc aagggtgccc gcccgggccc tggcctcctg gacaatgcca 1140
tcgactcggg cagctcagct gctcagctgc ctcagaagag tatcctggag cggaaggagg 1200
tgctcgtagc tgtgattgtg ggcggggtgg tgggcgccct ctttgctgcc ttcttggtca 1260
cactgctcat ctatcgtatg aagaaaaagg atgagggcag ctacacgctg gaggaaccca 1320
agcaggcgag cgtcacatac cagaagcctg acaagcagga ggagttctat gcctagtgga 1380
gccacagtgc ctccctgcag cctcaacacc accctgctgt ccagtcccca gcctggcccc 1440
accagcccaa gcctgggact gggcctggaa cctggcccca gttcttctct gccctctctc 1500
ccaaggtctg cccaggctgc cagcctcaca cagatcttcc ccgaggaaga ggggctgctg 1560
ccatctgccc cagactgtgc ccttacgagc tcatctcttg ttcccctcat ccctgccacc 1620
agtctggggc ttcaggacct catgtcagat ggatgggagg aagaaagctc ctgattggct 1680
ggtggtggaa gaaagggtgg ggcttgagat gagcctgagc cctgacttgg cacccacagt 1740
gctcactgag atctcctttt tggggcagag aggcactcag gctggtttcc aggacaaaca 1800
tttggtaaac acagcccttg aaatcatcta gacactgcaa cctcttgctc gtatcccagg 1860
gcctctctct agctgggtga gagggtgtcc cttgtcacca gcctgttttg tcctggtctc 1920
tctggggttg ttgaatctct cctcttgcct gccaagtaca catgtaccca gacttcattt 1980
ctttctgcat cttcccccaa gaaacagctt cctgagggtg ctggggcagc cactggtgag 2040
gaggggctgc tctgatgtcc ctcctatgag gggactctgc acagacacca ttgcccacac 2100
tatcaccata ttttcactca gtcacacaca agacaaaagc atgcaatgac aaaaccatac 2160
gcaatcctga ccgcccagcc aatcaagaca tatcacagaa cacacgcgtc cttccaagaa 2220
tgtttatcct catgcatcac ttacacaccc ccagacacgt actgcaatgc aagtcactag 2280
tcatggtcac atgacagtga cagtgtggcc tcctcctacc ccaaatacac ccacactctg 2340
gcaccacaca cattgtctcc agctttcagg cttactgggg agggtggaat cgagccagaa 2400
caatcagccc atattgggtc cccctaagtt gccccgtcat actcagtccc atgccatggt 2460
gcccacacca accatgcagc cgccaacccc agccagtgtc agacacaatc ccatgtggat 2520
gcacagtctc actccacatg acctgctctc aatgctggag ggaaacaggc aggcccttgc 2580
ctttctcgga aaaagtgtgg ggccacagcc cttttagggc attgcatgca ggtgggcctg 2640
gcttcacccc tacctgcttc ctccccaccg cagctggcag agggggaggt ttggggccag 2700

acccccacta gctgggagcc tgggggctcc tctaaggctg aggaaggaaa tttggcccca 2760
ggttgttggg gggtcttggg tctcccagga cggaaggccc agggcaggga gggggcatgt 2820
ggttgggctc ctttatctcc ctgtgtcccc ttcctgcttt gagctagggg gctgactctg 2880
cctcccagga cacaagtctc caaagtgcct gtgagggcgg gccctccgca ccctctgccc 2940
tctgcctggc aggccaacct cagcccacct gcccagaggc cctccctgtg gacaccccct 3000
cacctatttg gccaaacaat tctggctgca gcttcagggg ccatggctgg aagcagcccc 3060
tgcagatccc tcaggccccg aggtcagggt ttgagggatg agaccaggtg atagtggggg 3120
aggggttact tcctttgtta cctagcaagt agggctattt ccatcggtat tttaaatgtg 3180
gggtcacaga tcttttgggg agggtgtgct tggcaggggg cctcttggag ccaaagggat 3240
gtggtgtgag ttgcgattgg ctggcactca cacccccacc cctcacccac atcccagatt 3300
caagtcagga aggcaggttt tatttcaggg cccttttcaa gatgccctgg cagcagattt 3360
ctgcaggatg aggggtagcg gtgtgtaggc agtgaggggg aggttccagg ggctgtccca 3420
cagcctgtct tttccaggct gggctccatc ttccagtccc aaaaccctcc ttcacagggc 3480
ccagaggctt gtgaggaagc caggtggacc cagccttaga agagtgggca tgggggcccc 3540
ctgatatctg gagggggcgg gttggcctca gtcatctttg gagcagaagg gctgggtcct 3600
ggggccacag accacaaggc tcagcctccc taccctgctc cctggggtgc tgctgtcttg 3660
gagagcacag ctctggtgag acggcctggg caggccgagg ctgagaaacc agggaggata 3720
gaggagaaaa gggcttgggc ccccagcccc agaagatgct ggaccccagg tgggagaccc 3780
aacagtgggt gcagtttctc agtagggctg gagccaatgg tgggggtggc cccggcaggc 3840
ctggctcctc acatcccagg ggttggcttc tgatttgggg cttgggctcc aggcactggc 3900
ttctcttctc tgtgtcctta gcatttgaga gaagaggcca ggggccttgt tcatggatcc 3960
ctggacccaa ggcagatgtc caggctttat cctcctgagg atgaggagtc tgaccagccc 4020
aaatctgccc tggccggcct gaccggggca aggcagtcca agagagttca gtgaggacca 4080
gctaggctct cccaggtgca atgtgggtgc agggccctca tgtcccccta cccctgcctg 4140
tgatggagtg ttctgagggg ctttggcatt tgctggaagc acagggagtt ccaaatgaga 4200
gggagcttct gtggcttgag agcctctggg gccttggctg ccagagcacg aggcaggcca 4260
ggacctggag agcccagacc ctgtctccag gaggccagga ggccagatgg gggccttgcc 4320
tgaaagactg gtccccttga tcgctggagg catgtgggtg gcaaccaggg ctgggcaggg 4380
cttagggtgt gtgggccaaa cccccctggg gttggcaaag ccgcctgtca ggcctcctgg 4440
tgggggcccc tggacacagg gagcagaccc tctgcctcat ggggtaggag tggctgcctc 4500
ctgtgttctc tggatttctt ctcccaacaa ctacaaccct ggacttgcct ccccaggcct 4560

cttgcctgta aatagaagcc cgcaaactgt acagatttac agaggcatcg agactgggcc 4620

ctgggagttg ccatctgaga gccgatggcc ccagcatccc ccaggtgcct gcctggcacc 4680

acagtgaccc tggcctcagc gtggcaaatg catgtaaata tttttcgtag gcagcgtggc 4740

tccagagagc cccctgaaga cagtgtccct ccctcctgtg agtcctttct cctgtacaga 4800

acctgcctgg ggtgggtggg ggtctgccat tccctccccc aggccttccc tgccccttct 4860

ctcccctgta acctgtttat taaccatacc tgtcctgagt tcatggccaa aaccttaaat 4920

aagaaaaaca aaagaaaaag acagtggaaa aaagagacca aggcgcctgc cccactgcgg 4980

gtactctcct gttccagcct tgtgaaggaa ctggttttgt tttgtttttt tttttttttt 5040

tttttttttt gtttgtttgt ttttttaaca cttcctgtgc tgtgcccatt tataagagga 5100

aataaaatta agctgaaatg a 5121

<210> 11
<211> 198
<212> PRT
<213> Homo sapiens

<220>
<221> misc_feature
<222> (1)..(198)
<223> human Syndecan-4

<400> 11

Met Ala Pro Ala Arg Leu Phe Ala Leu Leu Leu Phe Phe Val Gly Gly
1               5                   10                  15

Val Ala Glu Ser Ile Arg Glu Thr Glu Val Ile Asp Pro Gln Asp Leu
            20                  25                  30

Leu Glu Gly Arg Tyr Phe Ser Gly Ala Leu Pro Asp Asp Glu Asp Val
        35                  40                  45

Val Gly Pro Gly Gln Glu Ser Asp Asp Phe Glu Leu Ser Gly Ser Gly
    50                  55                  60

Asp Leu Asp Asp Leu Glu Asp Ser Met Ile Gly Pro Glu Val Val His
65                  70                  75                  80

Pro Leu Val Pro Leu Asp Asn His Ile Pro Glu Arg Ala Gly Ser Gly
                85                  90                  95

Ser Gln Val Pro Thr Glu Pro Lys Lys Leu Glu Glu Asn Glu Val Ile
            100                 105                 110

Pro Lys Arg Ile Ser Pro Val Glu Glu Ser Glu Asp Val Ser Asn Lys

115                 120                 125

Val Ser Met Ser Ser Thr Val Gln Gly Ser Asn Ile Phe Glu Arg Thr
    130                 135                 140

Glu Val Leu Ala Ala Leu Ile Val Gly Gly Ile Val Gly Ile Leu Phe
145                 150                 155                 160

Ala Val Phe Leu Ile Leu Leu Leu Met Tyr Arg Met Lys Lys Lys Asp
                165                 170                 175

Glu Gly Ser Tyr Asp Leu Gly Lys Lys Pro Ile Tyr Lys Lys Ala Pro
            180                 185                 190

Thr Asn Glu Phe Tyr Ala
        195

<210>  12
<211>  2640
<212>  DNA
<213>  Homo sapiens

<220>
<221>  misc_feature
<222>  (1)..(2995)
<223>  human Syndecan-4

<400>  12
actcgccgca gcctgcgcgc cttctccagt ccgcggtgcc atggcccccg cccgtctgtt      60

cgcgctgctg ctgttcttcg taggcggagt cgccgagtcg atccgagaga ctgaggtcat     120

cgacccccag gacctcctag aaggccgata cttctccgga gccctaccag acgatgagga     180

tgtagtgggg cccgggcagg aatctgatga ctttgagctg tctggctctg gagatctgga     240

tgacttggaa gactccatga tcggccctga agttgtccat cccttggtgc ctctagataa     300

ccatatccct gagagggcag ggtctgggag ccaagtcccc accgaaccca agaaactaga     360

ggagaatgag gttatcccca agagaatctc acccgttgaa gagagtgagg atgtgtccaa     420

caaggtgtca atgtccagca ctgtgcaggg cagcaacatc tttgagagaa cggaggtcct     480

ggcagctctg attgtgggtg gcatcgtggg catcctcttt gccgtcttcc tgatcctact     540

gctcatgtac cgtatgaaga agaaggatga aggcagctat gacctgggca agaaacccat     600

ctacaagaaa gcccccacca atgagttcta cgcgtgaagc ttgcttgtgg gcactggctt     660

ggactttagc ggggagggaa gccaggggat tttgaagggt ggacattagg gtagggtgag     720

gtcaacctaa tactgacttg tcagtatctc cagctctgat tacctttgaa gtgttcagaa     780

gagacattgt cttctactgt tctgccaggt tcttcttgag ctttgggcct cagttgccct     840

ggcagaaaaa tggattcaac ttggcctttc tgaaggcaag actgggattg gatcacttct 900
taaacttcca gttaagaatc taggtccgcc ctcaagccca tactgaccat gcctcatcca 960
gagctcctct gaagccaggg ggctaacgga tgttgtgtgg agtcctggct ggaggtcctc 1020
ccccagtggc cttcctccct tcctttcaca gccggtctct ctgccaggaa atgggggaag 1080
gaactagaac cacctgcacc ttgagatgtt tctgtaaatg ggtacttgtg atcacactac 1140
gggaatctct gtggtatata cctggggcca ttctaggctc tttcaagtga cttttggaaa 1200
tcaacctttt ttatttgggg gggaggatgg ggaaaagagc tgagagttta tgctgaaatg 1260
gatttataga atatttgtaa atctattttt agtgtttgtt cgttttttta actgttcatt 1320
cctttgtgca gagtgtatat ctctgcctgg gcaagagtgt ggaggtgccg aggtgtcttc 1380
attctctcgc acatttccac agcacctgct aagtttgtat ttaatggttt ttgtttttgt 1440
ttttgtttgt ttcttgaaaa tgagagaaga gccggagaga tgatttttat taattttttt 1500
tttttttttt tttttttact atttatagct ttagataggg cctcccttcc cctcttcttt 1560
ctttgttctc tttcattaaa ccccttcccc agtttttttt ttatacttta aaccccgctc 1620
ctcatggcct tggccctttc tgaagctgct tcctcttata aaatagcttt tgccgaaaca 1680
tagttttttt ttagcagatc ccaaaatata atgaagggga tggtgggata tttgtgtctg 1740
tgttcttata atatattatt attcttcctt ggttctagaa aaatagataa atatattttt 1800
ttcaggaaat agtgtggtgt ttccagtttg atgttgctgg gtggttgagt gagtgaattt 1860
tcatgtggct gggtgggttt ttgccttttt ctcttgccct gttcctggtg ccttctgatg 1920
gggctggaat agttgaggtg gatggttcta ccctttctgc cttctgtttg ggacccagct 1980
ggtgttcttt ggtttgcttt cttcaggctc tagggctgtg ctatccaata cagtaaccac 2040
atgcggctgt ttaaagttaa gccaattaaa atcacataag attaaaaatt ccttcctcag 2100
ttgcactaac cacgtttcta gaggcgtcac tgtatgtagt tcatggctac tgtactgaca 2160
gcgagagcat gtccatctgt tggacagcac tattctagag aactaaactg gcttaacgag 2220
tcacagcctc agctgtgctg ggacgaccct tgtctccctg ggtagggggg ggggaatggg 2280
ggagggctga tgaggcccca gctggggcct gttgtctggg accctccctc tcctgagagg 2340
ggaggcctgg tggcttagcc tgggcaggtc gtgtctcctc ctgaccccag tggctgcggt 2400
gaggggaacc accctccctt gctgcaccag tggccattag ctcccgtcac cactgcaacc 2460
cagggtccca gctggctggg tcctcttctg cccccagtgc ccttcccctt gggctgtgtt 2520
ggagtgagca cctcctctgt aggcacctct cacactgttg tctgttactg attttttttg 2580
ataaaaagat aataaaacct ggtactttct aaaaaaaaaa aaaaaaaaaa aaaaaaaaaa 2640

<210> 13
<211> 25

<212>  DNA
<213>  Artificial

<220>
<223>  Morpholino R3 Mo2

<400>  13
gcagtcgcaa ttgcatagta acctt                                              25